# EUROPEAN PATENT APPLICATION

(11) **EP 1 970 373 A1**
(43) Date of publication of application: **17.09.2008**
(21) Application number: 06833711.2
(22) Date of filing: 30.11.2006
(51) Int. Cl.: C07D 237/24

(54) **ALICYCLIC HETEROCYCLIC COMPOUND**

(30) Priority: 02.12.2005 JP 2005348597; 14.12.2005 US 750038 P
(71) Applicant: Mitsubishi Tanabe Pharma Corporation, Chuo-ku Osaka-shi Osaka 541-8505 (JP)
(72) Inventor: FURUKUBO, Shigeru, Osaka 541-8505 (JP); MIYAZAKI, Hiroshi, Osaka 541-8505 (JP)
(74) Representative: Srinivasan, Ravi Chandran
(86) International application number: PCT/JP2006/323908
(87) International publication number: WO 2007/063934

(57) **Abstract**

An alicyclic heterocyclic compound represented by the following formula or a pharmaceutically acceptable salt thereof: 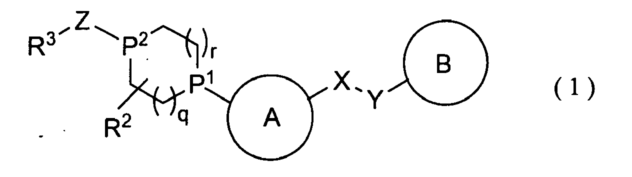 wherein ring A is a heterocyclic ring, ring B is a carbocyclic ring, a heterocyclic ring etc., P¹ and P² are CH or N, q and r are 0 to 2, X is -NH-, -O-, -CH₂-, etc., Y is -CH₂-, -CO-, -SO₂-, etc., Z is -CO-, -SO₂-, etc., and R³ is carbocyclic group, heterocyclic group, hydroxyl, alkoxy or amino,
is useful as a controlling agent of the function of CCR4 useful for the prevention or treatment for bronchial asthma, atopic dermatitis, etc.

## Description

### TECHNICAL FIELD

The present invention relates to the compounds having an activity for controlling the function of CCR4, or TARC and/or MDC useful for the prophylaxis or treatment for allergic diseases such as bronchial asthma or atopic dermatitis, inflammatory diseases and autoimmune diseases.

### BACKGROUND ART

Allergic diseases such as bronchial asthma and atopic dermatitis are chronic inflammatory diseases associated with infiltration or activation of inflammatory cells (non patent documents 1 and 2). Bronchial asthma is a disease associated with reversible airway obstruction with airway inflammation and increased airway hypersensitivity. As a symptom thereof, stridor, shortness of breath, cough, etc. are observed. Chronic inflammation such as infiltration of eosinophils, lymphocytes and mast cells to airway, edema under mucosa, deposit of eosinophil-derived tissue damaged granular protein, or damage of airway epithelium are histologically observed. Atopic dermatitis is cutaneous chronic inflammatory disease with strong pruritus consisting of eczema which repeats exacerbation and remission as a main symptom. It is said that the pathema participates in both impairment of the epidermal barrier function consisting mainly of dermal dryness and the abnormal production of cytokines by immune cells. Therefore, to control such chronic inflammation is considered as one of approaches in the therapy of allergic diseases.

Recently, it has been revealed that helper T (Th) cells and cytokines produced by Th cells play very important roles in the process of pathogenesis of allergic inflammation (non patent documents 1 and 3). Th cells are classified to two sub-classes according to cytokine producing pattern, namely to Th 1 cells producing interferon γ (IFN-γ) or interleukin 2 (IL-2), and to Th 2 cells producing interleukin 4 (IL-4) or interleukin 5 (IL-5) (non patent document 4). IFN-γ and IL-2 control cellular immunity such as defence to infection and so on by activating macrophages or natural killer (NK) cells. On the other hand, since IL-4 and IL-5 participate in production of immunoglobulin(Ig) E and activation of eosinophils, respectively, Th 2 cells are considered to play large roles in the development of allergic inflammation (non patent documents 1, 5, 6 and 7).

Chemokines are classified to an endogenic leucocyte chemotactic factor and play an important role to tissue accumulation of leukocytes. The majority of chemokines is produced at inflammatory regions by the inflammatry stimulation, etc., and act on leukocytes to induce the chemotactic response. Up to now more than 40 chemokines have been identified, and they are classified to sub-classes, namely CXC, CC, C and CX3C according to structural features thereof. On the other hand, chemokine receptors are seven-transmembrane receptors which are conjugated with G protein, and consist of CXC chemokine receptor, CC chemokine receptor, CX3C chemokine receptor and C chemokine receptor. It is known that the majority of chemokine receptors is combined with plural chemokines, and the majority of chemokines are combined with plural chemokine receptors.

The gene coding for CC chemokine receptor 4 (CCR4) was cloned from human basophil-like cell line KU-812 in 1995 (non patent document 8). Thereafter, TARC (thymus and activation-regulated chemokine)/CCL17 as a CC chemokine which specifically migrates T cells and then MDC (macrophage-derived chemokine)/CCL22 as CC chemokine which shows chemotactic activity to monocytes, dendritic cells and NK cells were cloned, respectively (non patent documents 9 and 10), and it was revealed that these chemokines are ligands of CCR4 (non patent documents 11 and 12). CCR4 is much expressed in thymus and peripheral blood lymphocytes (non patent document 8) and it is comparatively localized and expressed in Th cells in lymphocytes (non patent document 11). CCR4 is selectively expressed in Th 2 cells, and as it is revealed that the migration of Th 2 cells is induced by the stimulation of TARC/CCL17 or MDC/CCL22 (non patent documents 11∼15), the role of CCR4 in the process of pathogenesis of allergic diseases has been paid attention.

In regard to the relation of allergic diseases and CCR4, and the relation of its ligands, namely TARC/CCL17 and MDC/CCL22, it is reported that (1) T cells expressed mRNA of CCR4 are detected at bronchial mucosa of a patient suffering from chronic bronchial asthma and the number of CCR4 expressed T cells increases after antigen exposure (non patent document 16), (2) the expression of mRNA of CCR4 is promoted in peripheral blood T cells of a patient suffering from atopic dermatitis, and the expression level of CCR4 relates to the number of the blood eosinophils , the level of serum IgE and the severity of dermatitis (non patent documents 17 and 18), (3) the serum concentration of TARC/CCL17 and MDC/CCL22 in patients suffering from atopic dermatitis is higher than that of in healthy persons (non patent documents 19 and 20), (4) in experimental asthma model, by treating with anti TARC antibody or anti MDC antibody, increased airway reactivity or and infiltration of inflammatory cells to airway or pulmonary interstitium are inhibited (non patent documents 21 and 22) and so on.

Furthermore, as evidences showing the relation of CCR4 and/or its ligands with allergic diseases, inflammatory diseases and autoimmune diseases, there are following reports:
Dermatitis (atopic dermatitis, contact dermatitis): non patent documents 23-25
Asthma: non patent documents 16 and 26
Rhinitis: non patent document 27
Conjunctivitis: non patent document 28
Psoriasis: non patent document 29
Rheumatoid arthritis: non patent document 30
Systemic lupus erythematosus: non patent documents 31-33
Insulin dependant diabetes mellitus (IDDM): non patent document 34
Rejection on organ transplantation: non patent document 35
Inflammatory bowel disease (ulcerative colitis, Crohn's disease): non patent document 36
Glomerulonephritis: non patent document 37
Sepsis: non patent document 38
Pain: non patent document 39
Adult T cell leukemia (ATL): non patent document 40
Fibroid lung: non patent documents 41 and 42
Eosinophilic pneumonia: non patent document 43
Pneumoeosinophil granuloma: non patent document 44
Dermal T cell lymphoma: non patent documents 20 and 45
Ankylosing spondylitis: non patent document 46
Coronary disease: non patent document 47
Pemphigoid: non patent document 48
Hodgkin's disease: non patent document 49

These reports do not only suggest that abnormal expression of CCR4 and its ligands, namely TARC/CCL17 and MDC/CCL22 great participates in pathogenesis of many kinds of pathological condition such as allergic diseases, but a possibility to treat or improve these pathological condition by controlling the function of CCR4 and its ligands are also suggested.

Now β2 stimulants, xanthine, steroids and antiallergic agents (especially leukotriene antagonist) are used in clinical field as a therapeutic agent for bronchial asthma. Among them, inhaled steroids are positioned as the first-line drug and it is widely used for therapy of asthma. However, when the steroids are administered for a long term, the side effects are anxious and therefore, it can not maintain drug compliance.

In the therapy of atopic dermatitis, tacrolimus having immunosuppressive activity is used as an external preparation in order to suppress inflammatory as well as the steroids. External steroids are anxious for side effects such as hairiness or atrophia cutis in skin diseases. On the other hand, external tacrolimus does not show such side effects as the steroids, but the relation of tacrolimus with occurrence of feeling of dermal irritation and pathogenesis of carcinoma cutaneum are indicated.

Therefore, there is desired therapeutic and prophylactic agents for allergic diseases, inflammatory diseases and autoimmune diseases, which have the same strong therapeutic activity as steroids based on new mechanism with few side effects. Furthermore, since compounds having CCR4 antagonistic activity or CCR4 function-controlling activity can selectively control the infiltration and the activation of Th 2 cells to inflammatory regions, it is expected that these compounds will become an orally-available drug with few side effects, unlike steroids or immunosuppressant.

As the compounds having CCR4 antagonism or CCR4 function-controlling activity, there are known following compounds: a 5-cyanopyrimidine derivative (patent document 1), a bicyclic pyrimidine derivative (patent document 2), a 5-arylpyrimidine derivative (patent document 3), a bicyclic compound (patent document 4), a tricyclic compound (patent documents 5 and 6), a fused bicyclic pyrimidine derivative (patent document 7), a substituted pyrimidine derivative (patent document 8), a sulfonamide compound (patent documents 9 to 15) and so on.
Non Patent document 1: Immunol. Today, 13, 501 (1992)
Non Patent document 2: Allergy Clin. Immunol., 94, 1310 (1994)
Non Patent document 3: Am. Rev. Respir. Dis., 147, 540 (1993)
Non Patent document 4: J. Immunol., 1986, 136, 2348-57
Non Patent document 5: Immunol. Today, 12, 256 (1991)
Non Patent document 6: N. Eng. J. Med., 326, 298 (1992)
Non Patent document 7: Trends. Pharmacol. Sci., 15, 324 (1994)
Non Patent document 8: J. Biol. Chem., 270, 19495 (1995)
Non Patent document 9: J. Biol. Chem., 271, 21514 (1996)
Non Patent document 10: J. Exp. Med., 185, 1595 (1997)
Non Patent document 11: J. Biol. Chem., 272, 15036 (1997)
Non Patent document 12: J. Biol. Chem., 273, 1764 (1998)
Non Patent document 13: J. Exp. Med., 187, 129 (1998)
Non Patent document 14: J. Exp. Med., 187, 875 (1998)
Non Patent document 15: Int. Immunol., 11, 81 (1999)
Non Patent document 16: J. Clin. Invest., 107, 1357 (2001)
Non Patent document 17: J. Allergy Clin. Immunol., 107, 353 (2001)
Non Patent document 18: J. Invest. Dermatol., 117, 188 (2001)
Non Patent document 19: J. Allergy Clin. Immunol., 107, 535 (2001)
Non Patent document 20: Eur. J. Immunol., 30, 204 (2000)
Non Patent document 21: J. Immunol., 163, 403 (1999)
Non Patent document 22: J. Immunol., 166, 2055 (2001)
Non Patent document 23: The Journal of Clinical Investigation, 107, 535, 2001
Non Patent document 24: Journal of Investigative Dermatology, 115, 640, 2000
Non Patent document 25: Journal of Investigative Dermatology, 186, 1052, 2002
Non Patent document 26: Allergy, 57, 2, 173, 2002
Non Patent document 27: European Journal of Immunology, 32, 7, 1933, 2002
Non Patent document 28: Br J Ophthalmol, 86, 10, 1175, 2002
Non Patent document 29: Laboratory Investigation, 81, 335, 2001
Non Patent document 30: Arthritis & Rheumatism, 44, 2750, 2001
Non Patent document 31: Journal of Investigative Dermatology, 124, 1241, 2005
Non Patent document 32: Clinical Experimental Immunology, 138, 342, 2004
Non Patent document 33: Arthritis & Rheumatism, 46, 735, 2002
Non Patent document 34: The Journal of Clinical Investigation, 110, 1675, 2002
Non Patent document 35: European Journal of Immunology, 35, 128, 2005
Non Patent document 36: Clinical & Experimental Immunology, 132, 332, 2003
Non Patent document 37: American Journal of Pathology, 162, 1061, 2003
Non Patent document 38: Journal of Experimental Medicine, 191, 1755, 2000
Non Patent document 39: The Journal of Neuroscience, 21, 5027, 2001
Non Patent document 40: Cancer Res. 2005 Mar 15; 11(6): 2427-35
Non Patent document 41: American Journal of Respiratory and Critical Care Medicine, Vol. 173, pp. 310-317 (2006)
Non Patent document 42: The Journal of Immunology, 173, 4692, 2004
Non Patent document 43: Clinical Immunology, 116, 83, 2005
Non Patent document 44: American Journal of Pathology, 165, 1211, 2004
Non Patent document 45: British Journal of Dermatology, 152, 746, 2005
Non Patent document 46: Clinical Experimental Immunology, 138, 342, 2004
Non Patent document 47: Journal of the American College of Cardiology, 41, 1460,2003
Non Patent document 48: British Journal of Dermatology, 148, 203, 2003
Non Patent document 49: International Journal of Cancer, 98, 567, 2002
Patent document 1: WO03/082855
Patent document 2: WO03/104230
Patent document 3: WO2004/074260
Patent document 4: WO2004/020584
Patent document 5: WO2004/007472
Patent document 6: WO2005/023771
Patent document 7: WO2005/082865
Patent document 8: WO2005/085212
Patent document 9: WO2005/021513
Patent document 10: WO2004/108692
Patent document 11: WO2004/108717
Patent document 12: WO2004/108690
Patent document 13: WO03/059893
Patent document 14: WO03/051870
Patent document 15: WO02/30358

### DISCLOSURE OF INVENTION

### PROBLEM TO BE SOLVED BY INVENTION

The present invention provides to the compounds having an excellent activity for controlling the function of CCR4, or TARC/CCL17 and/or MDC/CCL22 with few side effects, useful as the prophylactic or therapeutic agent for allergic diseases, inflammatory diseases, autoimmune diseases and so on.

### MEANS FOR SOLVING PROBLEM

To solve the above-mentioned problem, the present inventors have earnestly studied, and found that the compounds represented by the following formula have an excellent activity for controlling the function of CCR4 or TARC/CCL17 and/or MDC/CCL22. Thus the present invention was accomplished.

Namely, the present invention is as follows.
1. A compound of the formula (1): 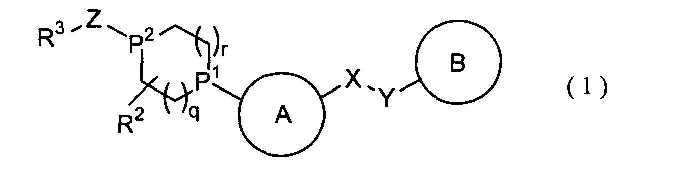 wherein the ring A is a group selected from the group consisting of the following formulas: 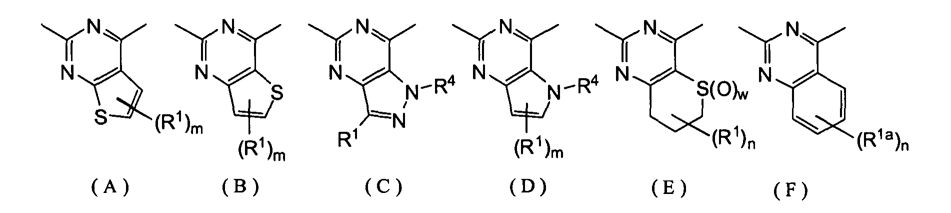 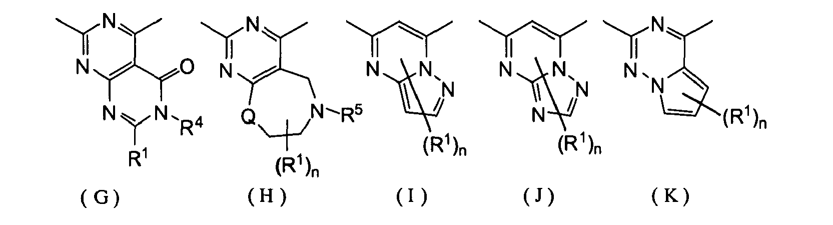 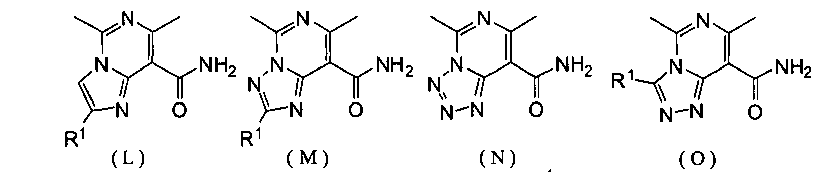 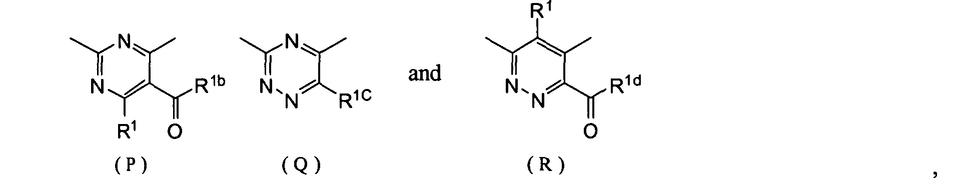 the ring B is an optionally substituted aromatic carbocyclic ring or an optionally substituted heterocyclic ring;
   P¹ and P² are the same or different and each is CH or N, provided that neither P¹ nor P² is CH at the same time;
   q and r are 0, 1 or 2 respectively;
   m is 1 or 2, and n is an integer of 1 to 3;
   w is 0, 1 or 2;
   Q is an oxygen atom, a sulfur atom or -N(R⁶)-
   X is -N(R⁷)-, -O- or -C(R⁸)(R⁹)-;
   Y is -C(R¹⁰)(R¹¹)-, -CO- or -SO₂-;
   Z is alkylene optionally substituted with oxo, -CON(R¹²)-, - SO₂ N(R¹²)-, -N(R¹²)- or -SO₂ -, provided that P² is CH when Z is -CON(R¹²)-, - SO₂ N(R¹²)- or -N(R¹²)-;
   R¹ is hydrogen, alkyl, alkoxy, halogen, carboxy, alkoxycarbonyl, optionally substituted carbamoyl, optionally substituted amino, nitro or optionally substituted ureido;
   R^{1a} is carboxy, alkoxycarbonyl, optionally substituted carbamoyl, optionally substituted amino, nitro, phenyl or optionally substituted ureido;
   R^{1b} is amino optionally mono- or di-substituted with alkyl(s), or optionally substituted phenyl;
   R^{1c} is carboxy, alkoxycarbonyl, cyano, carbamoyl optionally mono-or di-substituted with alkyl(s), or optionally substituted benzoyl;
   R^{1d} is hydroxy, alkoxy, amino optionally mono- or di-substituted with alkyl(s), or optionally substituted phenyl;
   R² is hydrogen, alkyl, alkoxycarbonyl, carboxy or oxo;
   R³ is an optionally substituted carbocyclic group, an optionally substituted heterocyclic group, hydroxy, alkoxy or optionally substituted amino;
   R⁴ is hydrogen or alkyl;
   R⁵ is hydrogen, alkyl or optionally substituted alkanoyl;
   R⁶ is hydrogen, alkyl or optionally substituted alkanoyl;
   R⁷ is hydrogen or alkyl;
   R⁸ and R⁹, and R¹⁰ and R¹¹ are the same or different, and each is hydrogen or alkyl;
   R¹² is hydrogen or alkyl;
   or a pharmaceutically acceptable salt thereof,
2. The compound or a pharmaceutically acceptable salt thereof mentioned in above 1, wherein the ring A is a group selected from the group consisting of the following formulas: 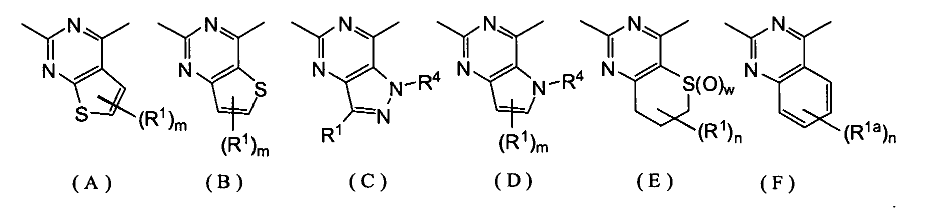 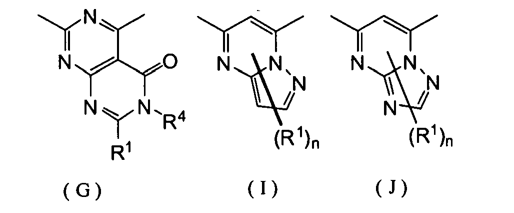 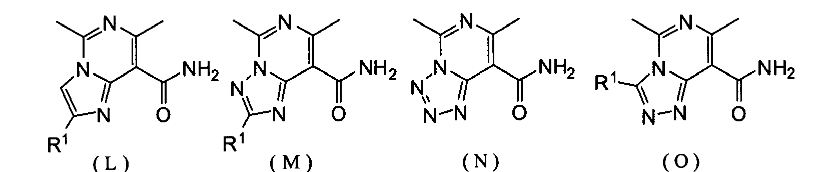 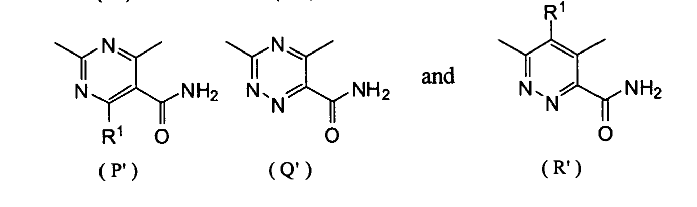 wherein each symbol is the same as described above,
3. The compound or a pharmaceutically acceptable salt thereof mentioned in above 1 or 2, wherein Z is alkylene substituted with oxo,
4. The compound or a pharmaceutically acceptable salt thereof mentioned in any one of above 1 to 3, wherein R³ is (1) pyrrolidinyl optionally substituted with oxo or cyano, (2) piperidinyl optionally substituted with a group selected from alkyl, alkanoyl and oxo, (3) piperazinyl optionally substituted with alkyl, (4) morpholinyl optionally substituted with alkyl, (5) imidazolyl optionally substituted with alkyl, (6) pyridyl, (7) thiomorpholinyl optionally substituted with one or two oxo(s), (8) tetrahydropyranyl or (9) tetrahydropyrimidinyl optionally substituted with one or two oxo(s),
5. The compound or a pharmaceutically acceptable salt thereof mentioned in any one of above 1 to 4, wherein X is -NH-, Y is -CH₂-, -CH(CH₃)- or - C(CH₃)₂- and the ring B is benzene substituted with one or two group(s) selected from halogen, alkyl and haloalkyl,
   In the compound of the formula (1) of the present invention, another preferred embodiments also include the following compounds.
6. A compound of the formula (1) 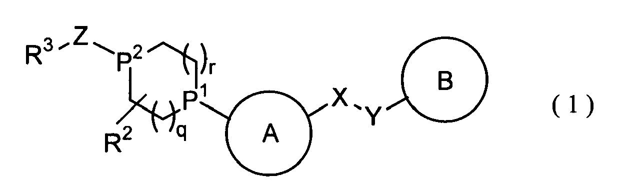 wherein the ring A is a group selected from the group consisting of the following formulas: 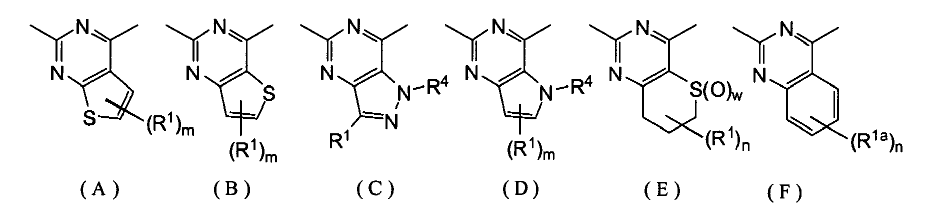 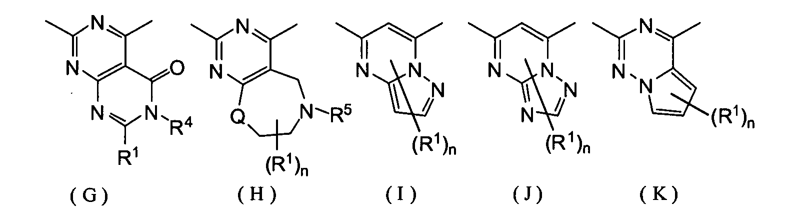 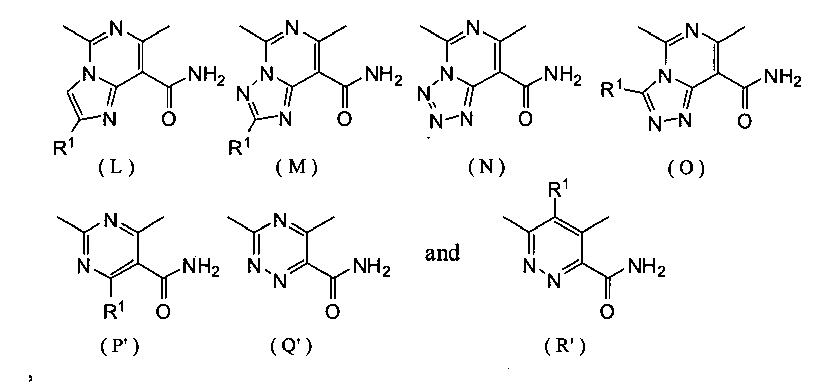 the ring B is an aromatic carbocyclic ring optionally substituted with the same or different one to three groups selected from halogen and cyano;
   P¹ and P² are the same or different and each is CH or N, provided that neither P¹ nor P² is CH at the same time;
   q and r are 0, 1 or 2 respectively;
   m is 1 or 2, and n is an integer of 1 to 3;
   w is 0, 1 or 2;
   Q is an oxygen atom, a sulfur atom or -N(R⁶)-
   X-Y is -NH(CH₂)- or -NHCH(CH₃) -;
   Z is alkylene optionally substituted with oxo, -CON(R¹²)-, - SO₂ N(R¹²)-, -N(R¹²)- or -SO₂ -, provided that P² is CH when Z is -CON(R¹²)-, - SO₂ N(R¹²)- or -N(R¹²)-;
   R¹ is hydrogen, alkyl, alkoxy, halogen, carboxy, alkoxycarbonyl, optionally substituted carbamoyl, optionally substituted amino, nitro or optionally substituted ureido;
   R^{1a} is carboxy, alkoxycarbonyl, optionally substituted carbamoyl, optionally substituted amino, nitro, phenyl or optionally substituted ureido;
   R² is hydrogen, alkyl, alkoxycarbonyl, carboxy or oxo;
   R³ is an optionally substituted heterocyclic group or optionally substituted amino;
   R⁴ is hydrogen or alkyl;
   R⁵ is hydrogen, alkyl or optionally substituted alkanoyl;
   R⁶ is hydrogen, alkyl or optionally substituted alkanoyl; and
   R¹² is hydrogen or alkyl;
   or a pharmaceutically acceptable salt thereof,
7. The compound or a pharmaceutically acceptable salt thereof mentioned in above 6, wherein R¹ is hydrogen or alkyl,
8. The compound or a pharmaceutically acceptable salt thereof mentioned in above 6 or 7, wherein R^{1a} is optionally substituted carbamoyl or optionally substituted ureido,
9. The compound or a pharmaceutically acceptable salt thereof mentioned in any one of above 6 to 8, wherein R² is hydrogen or carbamoyl,
10. The compound or a pharmaceutically acceptable salt thereof mentioned in any one of above 6 to 9, wherein R³ is a heterocyclic group optionally substituted with the same or different one to three groups selected from alkyl, oxo and halogen, or amino optionally mono- or di-substituted with alkyl(s),
11. The compound or a pharmaceutically acceptable salt thereof mentioned in any one of above 6 to 10, wherein R³ is (1) pyrrolidine optionally substituted with the same or different one to three groups selected from alkyl, oxo and halogen, (2) morpholine optionally substituted with alkyl, (3) piperidine optionally substituted with alkyl,
12. The compound or a pharmaceutically acceptable salt thereof mentioned in any one of above 6 to 11, wherein r is 1 and q is 0 or 1,
13. The compound or a pharmaceutically acceptable salt thereof mentioned in any one of above 6 to 12, wherein Z is -(CH₂)₂-, -CH₂ CO- or -CO-, or a pharmaceutically acceptable salt thereof,
14. The compound or a pharmaceutically acceptable salt thereof mentioned in any one of above 6 to 13, wherein the ring B is benzene optionally substituted with the same or different one to three groups selected from halogen and cyano, or a pharmaceutically acceptable salt thereof.
15. The compound or a pharmaceutically acceptable salt thereof mentioned in any one of above 6 to 14, wherein the ring A is a group selected from 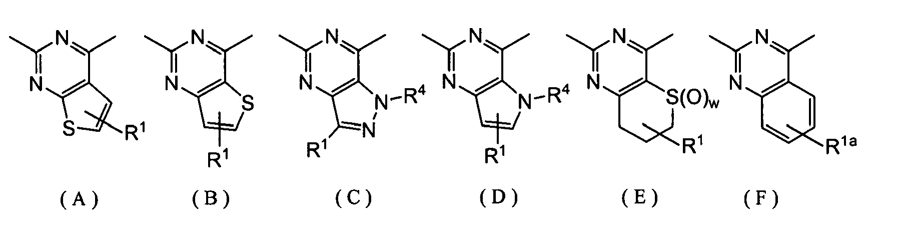 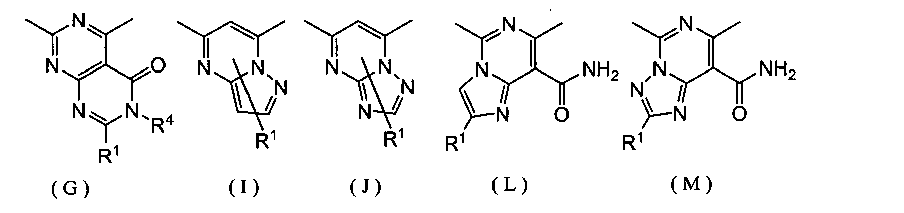 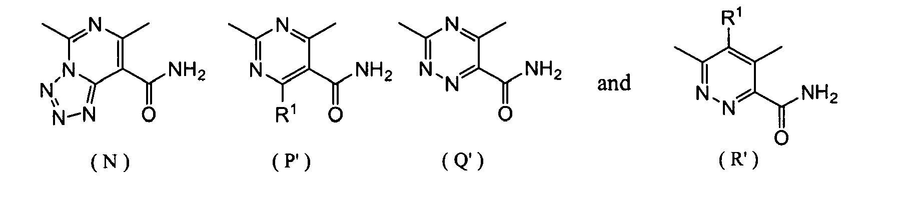 wherein each symbol is the same as described above,
16. A medicine comprising the compound or a pharmaceutically acceptable salt thereof mentioned in any one of above 1 to 15,
17. An agent for controlling the functions of CCR4 or TARC/CCL17 and/or MDC/CCL22 comprising the compound or a pharmaceutically acceptable salt thereof mentioned in any one of above 1 to 15, or a method for controlling the said function comprising by administrating said compound to a patient.
18. An prophylactic or therapeutic agent for an allergic diseases, inflammatory diseases or autoimmune diseases or cancer comprising the compound or a pharmaceutically acceptable salt thereof mentioned in any one of above 1 to 15 as an active ingredient, or a method for treating the said diseases by administrating said compound to a patient.
19. An prophylactic or therapeutic agent for asthma or dermatitis comprising the compound or a pharmaceutically acceptable salt thereof mentioned in any one of above 1 to 15 as an active ingredient, or a method for treating the said diseases by administrating said compound to a patient.

A group represented by each symbol in the present specification is set forth below. Abbreviations used in this specification mean as follows respectively.
- THF:: tetrahydrofuran
- DMF:: N,N-dimethylformamide
- DMSO:: dimethyl sulfoxide
- DMA:: dimethylacetamide
- DME:: 1,2-dimethoxyethane
- LDA:: lithium diisopropylamide
- DBU:: 1,8-diazabicyclo[5.4.0]-7-undecene
- DBN:: 1,5-diazabicyclo[4.3.0]nona-5-ene
- Me:: methyl
- Et:: ethyl
- Pr:: n-propyl
- iPr:: isopropyl
- t-Bu:: tert-butyl
- Boc:: tert-butoxycarbonyl
- Bn:: benzyl
- Ph:: phenyl

Examples of "aromatic carbocyclic ring" include a 6 to 14-membered monocyclic, bicyclic or tricyclic unsaturated carbocyclic ring, such as benzene, naphthalene, phenanthrene, anthracene and the like.

Examples of "heterocyclic ring" include a 3 to 15-membered monocyclic or bicyclic unsaturated, saturated or partially saturated heterocyclic rings containing one to four heteroatom(s) selected from a nitrogen atom, an oxygen atom and a sulfur atom. Specific examples of the unsaturated, saturated or partially saturated heterocyclic rings include pyrrole, imidazole, triazole, tetrazole, pyrazole, pyridine, pyrazine, pyrimidine, pyridazine, azepine, diazepine, furan, pyran, oxepin, thiophene, thiopyran, thiepin, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, benzofuran, benzothiophene, indazole, quinoline, isoquinoline, quinoxaline, quinazoline, benzoxazole, benzothiazole, benzimidazole, pyrroline, pyrrolidine, imidazoline, imidazolidine, pyrazoline, pyrazolidine, dihydropyridine, tetrahydropyridine, dihydropyrazine, tetrahydropyrazine, dihydropyrimidine, tetrahydropyrimidine, dihydroazepin, tetrahydroazepin, dihydrodiazepin, tetrahydrodiazepin, dihydrofuran, tetrahydrofuran, dihydropyran, tetrahydropran, dihydrothiophene, tetrahydrothiophene, dihydrothiopyran, tetrahydrothiopyran, piperidine, piperazine, morpholine, thiomorpholine, homopiperidine, and the like.

Examples of "alicyclic heterocyclic ring" include a 5 to 7-membered monocyclic saturated heterocyclic ring containing one or two heteroatom(s)selected from a nitrogen atom, a oxygen atom and a sulfur atom, such as piperidine, piperazine, morpholine, thiomorpholine, homopiperidine, tetrahydrooxazine and the like.

Examples of "alkylene" include a straight or branched C1-C10 alkylene, such as methylene, ethylene, trimethylene, propylene, tetramethylene, pentamethylene, hexamethylene, and the like.

Examples of "alkyl" include a straight or branched C1-C6 alkyl, such as methyl, ethyl, propyl, isopropyl, butyl, isobutyl, tert-butyl, pentyl, hexyl and the like.

Examples of "alkoxy" include a straight or branched C1-C6 alkoxy, such as methoxy, ethoxy, propoxy, isopropoxy, butoxy, isobutoxy, tert-butoxy, pentyloxy, hexyloxy and the like.

Examples of "halogen" include fluorine, chlorine, bromine and iodine.

Examples of "haloalkyl" include a straight or branched C1-C6 alkyl substituted with one to six halogen atom(s), such as fluoromethyl, chloromethyl, trifluoromethyl, 2,2,2-trifluoroethyl and the like.

Examples of "alkoxycarbonyl" include a straight or branched C2-C7 alkoxycarbonyl, such as methoxycarbonyl, ethoxycarbonyl, propoxycarbonyl, isopropoxycarbonyl, butoxycarbonyl isobutoxycarbonyl, tert-butoxycarbonyl and the like.

Examples of "alkanoyl" include a straight or branched C1-C6 alkanoyl, such as formyl, acetyl, propionyl, butyryl, pentanoyl, hexanoyl and the like.

Examples of "aralkyl" include a straight or branched C1-C6 alkyl substituted with an aromatic carbocyclic ring (preferably benzene), such as benzyl, 2-phenylethyl, 1-phenylethyl, 3-phenylpropyl and the like.

Examples of "cycloalkyl" include C3-C6 cycloalkyl, such as cyclopropyl, cyclobutyl, cyclopentyl ,cyclohexyl and the like.

Examples of "alkylsulfonyl" include a straight or branched C 1-C6 alkylsulfonyl, such as methylsulfonyl, ethylsulfonyl, propylsulfonyl, isopropylsulfonyl,butylsulfonyl and the like.

Examples of "alkylenedioxy" include a straight or branched C1-C4 alkylenedioxy, such as methylenedioxy, ethylenedioxy, trimethylenedoxy, propylenedioxy and the like.

Examples of "carbocyclic group" include a 3 to 15-membered monocyclic, bicyclic or tricyclic unsaturated, saturated or partially saturated carbocyclic group. Specific examples of the carbocyclic group include phenyl, naphthyl, phenanthryl, anthryl, cyclopropyl, cyclobutyl, cyclopentyl, cyclohexyl ,cycloheptyl, cyclooctyl, cyclopentenyl, cycohexenyl, cycloheptenyl, cyclooctenyl, cyclopentadienyl, cyclohexadienyl, cycloheptadienyl, cyclooctadienyl, indenyl, indanyl, dihydronaphthyl, tetrahydronaphthyl and the like.

Examples of "heterocyclic group" include a 3 to 15-membered monocyclic or bicyclic unsaturated, saturated or partially saturated heterocyclic group containing one to four heteroatom(s) selected from a nitrogen atom, an oxygen atom and a sulfur atom. Specific examples of the heterocyclic group include pyrrolyl, imidazolyl, triazolyl, tetrazolyl, pyrazolyl, pyridyl, pyrazinyl, pyrimidinyl, pyridazinyl, azepinyl, diazepinyl, furyl, pyranyl, oxepinyl, thienyl, thiopyranyl, thiepinyl, oxazolyl, isoxazolyl, thiazolyl, isothiazolyl, furazanyl, oxadiazolyl, oxazinyl, oxadiazinyl, oxazepinyl, oxadiazepinyl, thiadiazolyl, thiazinyl, thiadiazinyl, thiazepinyl, thiadiazepinyl, indolyl, isoindolyl, benzofuranyl, benzothiophenyl, indazolyl, quinolinyl, isoquinolinyl, quinoxalinyl, quinazolinyl, benzoxazolyl, benzothiazolyl, benzimidazolyl, pyrrolinyl, pyrrolidinyl, imidazolinyl, imidazolidinyl, pyrazolinyl, pyrazolidinyl, dihydropyridyl, tetrahydropyridyl, dihydropyrazinyl, tetrahydropyrazinyl, dihydropyrimidinyl, tetrahydropyrimidinyl, dihydroazepinyl, tetrahydroazepinyl, dihydrodiazepinyl, tetrahydrodiazepinyl, dihydrofuryl, tetrahydrofuryl, dihydropyranyl, tetrahydropranyl, dihydrothienyl, tetrahydrothienyl, dihydrothiopyranyl, tetrahydrothiopyranyl, piperidyl, piperazinyl, morpholinyl, thiomorpholinyl, homopiperidinyl, and the like.

Examples of "aromatic heterocyclic group" include a 3 to 15-membered monocyclic or bicyclic unsaturated heterocyclic group containing one to four heteroatom(s) selected from a nitrogen atom, an oxygen atom and a sulfur atom, such as pyrrolyl, imidazolyl, triazolyl, tetrazolyl, pyrazolyl, pyridyl, pyrazinyl, pyrimidinyl, pyridazinyl, furyl, thienyl, oxazolyl, isoxazolyl, thiazolyl, isothiazolyl, furazanyl, oxadiazolyl, thiadiazolyl, indolyl, isoindolyl, benzofuranyl, benzothiophenyl, indazolyl, quinolinyl, isoquinolinyl, quinoxalinyl, quinazolinyl, benzoxazolyl, benzothiazolyl, benzimidazolyl, and the like.

Examples of "alicyclic heterocyclic group" include a 5 to 7-membered monocyclic saturated heterocyclic group containing one or two heteroatom(s) selected from a nitrogen atom, an oxygen atom and a sulfur atom, such as piperidyl, piperazinyl, morpholinyl, thiomorpholinyl, homopiperidyl, tetrahydrooxazinyl and the like.

Examples of the substituents in "optionally substituted aromatic carbocyclic ring" and "optionally substituted heterocyclic ring" of the ring B include halogen, alkyl, haloalkyl, hydroxyl, alkoxy, cyano, carboxy, alkoxycarbonyl, nitro and the like and the said rings may be substituted with one to three of these substituent(s). Examples of the preferred substituents include halogen, alkyl and haloalkyl, and halogen is especially preferred.

Examples of "alkylene substituted with oxo" include carbonyl, 1-oxoethylene, 2-oxoethylene, 1-oxotrimethylene and the like.

Examples of the substituents in "optionally substituted carbamoyl" of R¹ and R^{1a} include alkyl and aralkyl and the carbamoyl group may be substituted with one or two of these substituent(s) which are the same or different.

Examples of the substituents in "optionally substituted amino" of R¹ and R^{1a} include alkyl, optionally substituted alkanoyl, alkylsulfonyl, optionally substituted alkoxycarbonyl, cycloalkylcarbonyl, hydroxyl and the like, and the amino group may be substituted with one or two of these substituent(s) which are the same or different. Examples of the substituents in the optionally substituted alkanoyl group and the optionally substituted alkoxycarbonyl group include alkoxy, hydroxyl and the like.

Examples of the substituents in "optionally substituted ureido" of R¹ and R^{1a} include alkyl and the like, and the amino group may be substituted with one or two alkyl group(s) which are the same or different. In addition, the two substituents may form a 5 to 7-membered alicyclic heterocyclic ring together with the adjacent nitrogen atom.

Examples of the substituents in "optionally substituted phenyl" of R^{1b} and "optionally substituted benzoyl" of R^{1c} include halogen, alkyl, alkoxy, hydroxy and the like, and the said group may be substituted with one to three of these substituents which are the same or different.

Examples of the substituents in "optionally substituted carbocyclic group" of R³ include an alicyclic heterocyclic group optionally substituted with oxo, optionally substituted alkyl, cyano, optionally substituted amino, alkylenedioxy and the like. Examples of the substituents in the optionally substituted alkyl include cyano and the like. Examples of the substituents in the optionally substituted amino include alkylsulfonyl and the like.

Examples of the substituents in "optionally substituted heterocyclic group" of R³ include oxo, carboxy, alkoxycarbonyl, amino optionally mono- or di-substituted with alkyl, a heterocyclic group, alkyl optionally substituted with phenyl, carbamoyl optionally mono- or di-substituted with alkyl, alkylsulfonyl, alkanoyl, phenyl optionally substituted with alkoxy, halogen, cyano and the like.

Examples of the substituents in "optionally substituted amino" of R³ include alkyl, alkanoyl, alkoxycarbonyl, optionally substituted phenyl and the like, and the amino group may be substituted with one or two of these substituents which are the same or different. Examples of the optionally substituted phenyl include halogen, alkyl, alkoxy, hydroxyl and the like.

Examples of the substituents in "optionally substituted alkanoyl" of R⁵ and R⁶ include cycloalkyl and the like.

Preferred examples of the groups represented by the following formula: 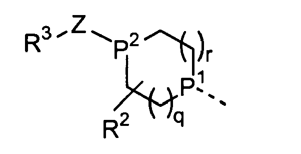 include the following groups;

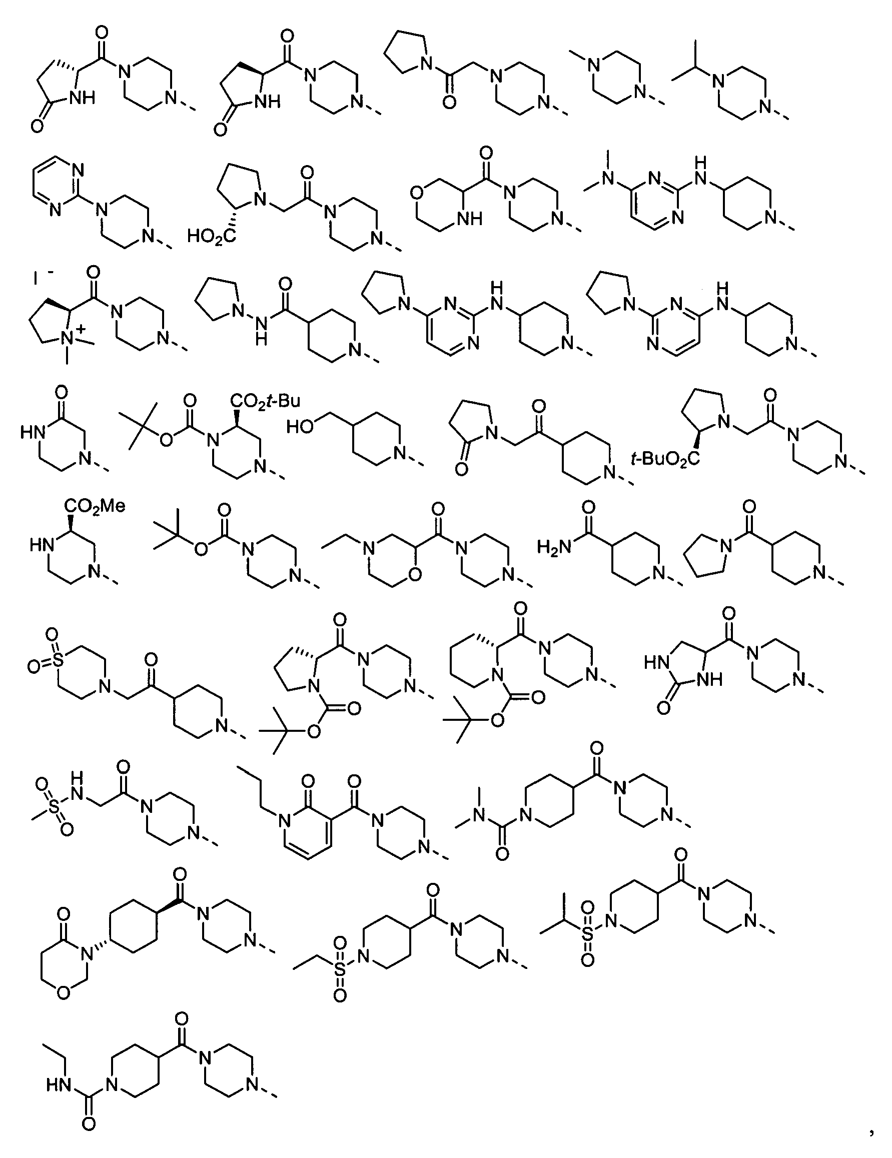

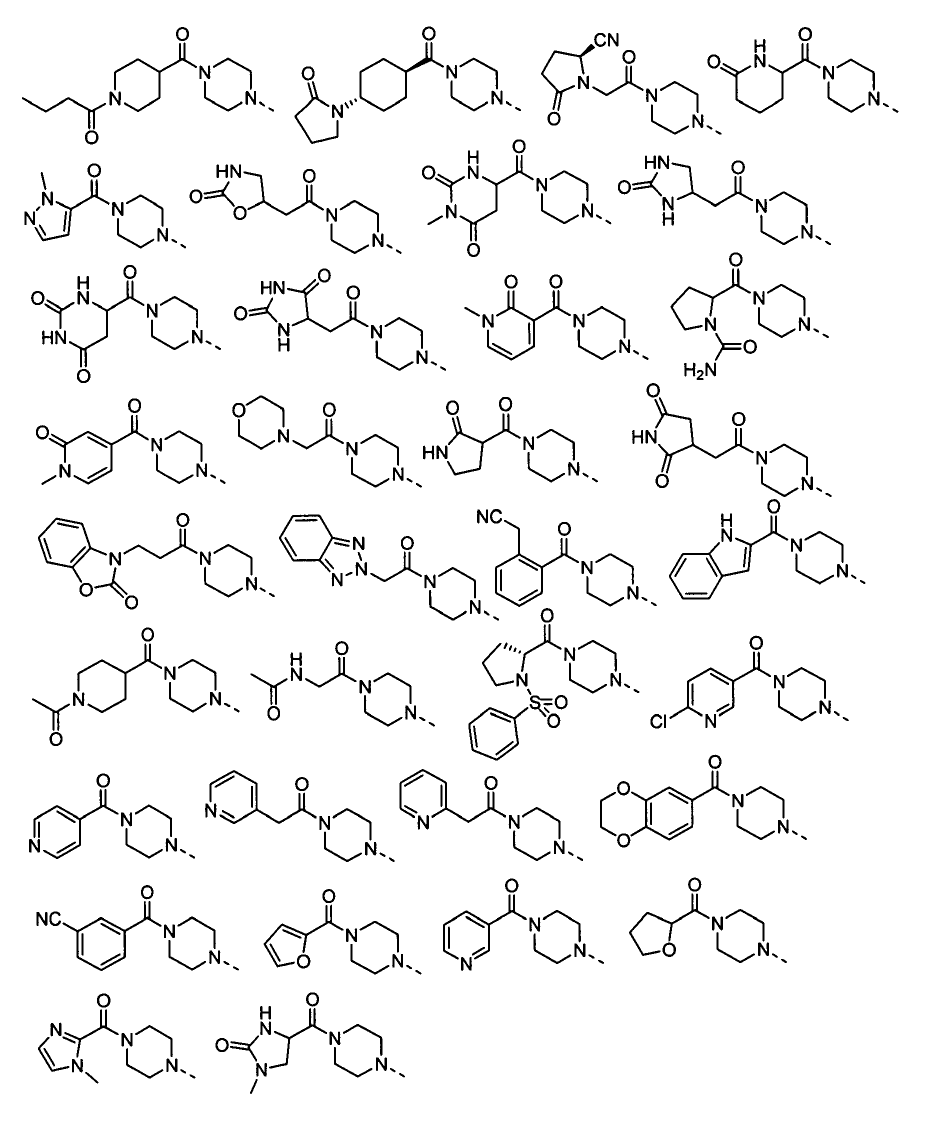

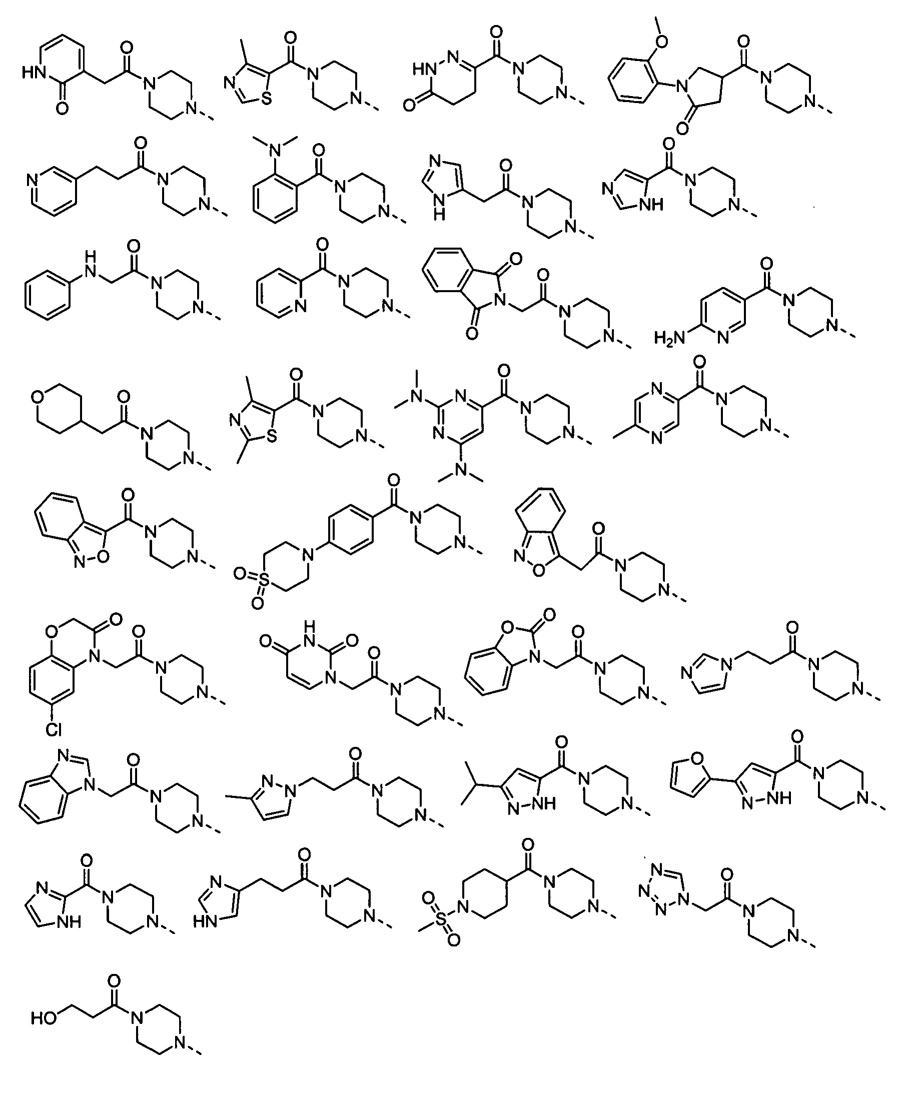

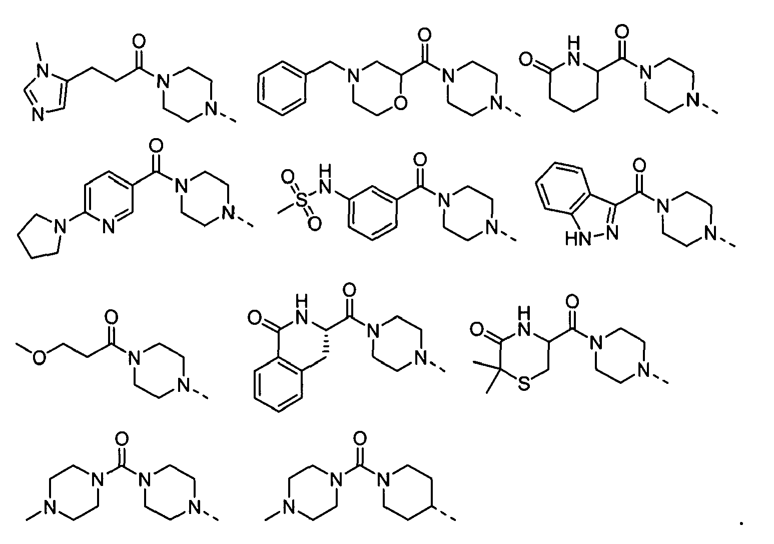

Examples of the pharmaceutically acceptable salt of the compound of the present invention include for example an inorganic acid salt such as hydrochloride, sulfate, phosphate, hydrobromide; and an organic acid salt such as acetate, fumarate, oxalate, citrate, methanesulfonate, benzenesulfonate, tosylate, malate, and the like. Furthermore, when the compound has an acidic group such as carboxy group and so on, the salt with a base such as an alkaline metal salt e.g. sodium salt, potassium salt etc., an alkaline earth metal salt e.g. calcium salt etc., an organic base salt e.g. triethylamine salt etc., and an amino acid salt e.g. lysine salt etc., are also include therein.

The compound of the present invention and the pharmaceutically acceptable salt thereof include an inner salt thereof and a solvate thereof such as a hydrate.

The compound (1) of the present invention exists in optically active isomers based on its asymmetric carbon, and includes any of forms of its isomers and a mixture thereof. Furthermore, when the compound (1) has a double bond or cycloalkandiyl group, the compound exists in trans or cis configuration, or tautomer based on an unsaturated bond such as carbonyl, and includes any isomer and a mixture thereof. Additionally, N-oxide is also included in the compound of the present invention.

A compound of the present invention can be prepared by the following methods;

Method 1: Compound (1) wherein P¹ is N can be prepared by the following method; 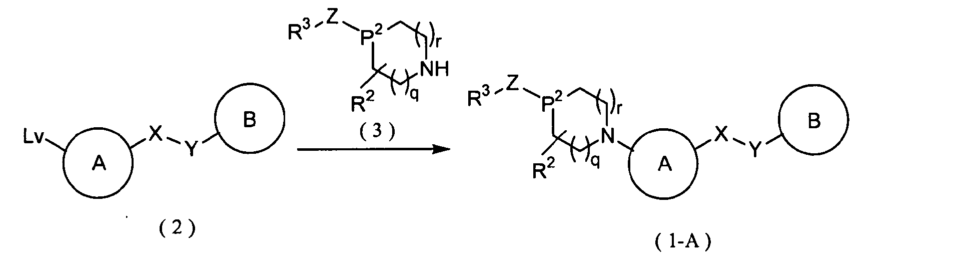 wherein Lv is halogen or CH₃S(O)ₚ (p is 0, 1 or 2) and other symbols are the same described above.

Compound(1-A) is obtained by reacting Compound(2) with Compound(3) in a solvent(THF, dioxane, diethyl ether, DMF, DMSO, methanol, ethanol, ethyleneglycol etc.) in the presence of a base(triethylamine, diisopropylethylamine, pyridine, sodium hydroxide, potassium hydroxide, sodium carbonate, potassium carbonate etc.) at 0-150°C for 1-24 hours.

Method 2: Compound(1) wherein P² is N and Z is -CO- or -SO₂- can be prepared by the following method;

R³-Z¹-Hal (5-a)

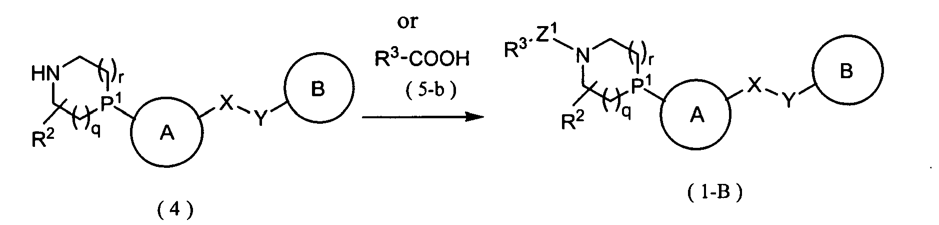

wherein Z¹ is -CO- or -SO₂-, Hal is halogen and other symbols are the same described above.
(1) Compound(1-B) is obtained by reacting Compound(4) with Compound(5-a) in the presence of a base(sodium hydrogen carbonate, potassium carbonate, triethylamine, pyridine etc.) at -20°C to room temperature for 30 minutes to 24 hours.
(2) Compound(1-B) is obtained by condensing Compound(4) with Compound(5-b) in the presence of a condensing agent(1,3-dicyclohexylcarbodiimide, 1-ethyl-3-(3-dimethylaminopropyl)carbodiimide, carbonyldiimidazole, diethyl cyanophosphate, etc.) in a solvent(DMF, THF, dioxane etc.), if necessary. The present reaction is usually carried out at 0-100°C for 30 minutes to 24 hours. Additionally the reaction using the condensing agent can be carried out in the presence of 1-hydroxybenzotriazole, N-hydroxysuccinimide and the like.
(3) Compound(1-B) is obtained by converting Compound(4) to the corresponding mixed acid anhydride(e.g., a carbonate ester with methyl chlorocarbonate, ethyl chlorocarbonate etc.), and reacting the said mixed acid anhydride with Compound(5-b) in a suitable solvent(THF, toluene, nitrobenzene, a mixed solvent thereof etc.) in the presence of a base(triethylamine, pyridine etc.) at room temperature to refluxing temperature of the solvent for 1-24 hours.

Method 3: Compound(1) wherein the ring A is a group (A) to (F) or (I) to (K), X is -N(R⁷)- or -O-, Y is -C(R¹⁰)(R¹¹)- and P¹ is N can be prepared by the following method; 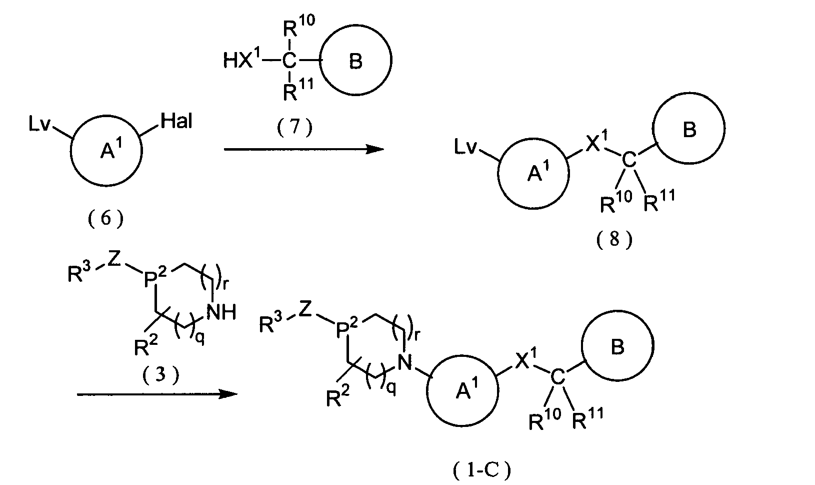 wherein the ring A¹ is a group (A) to (F) or (I) to (K), X is -N(R⁷)- or -O- and other symbols are the same as described above.

The reaction of Compound(6) and Compound(7), and the reaction of Compound(8) and Compound(3) can be carried out in the same manner as Method 1.

Method 4: Compound(1) wherein the ring A is a group (G), X is -N(R⁷)-or -O-, and P¹ is N can be prepared by the following method; 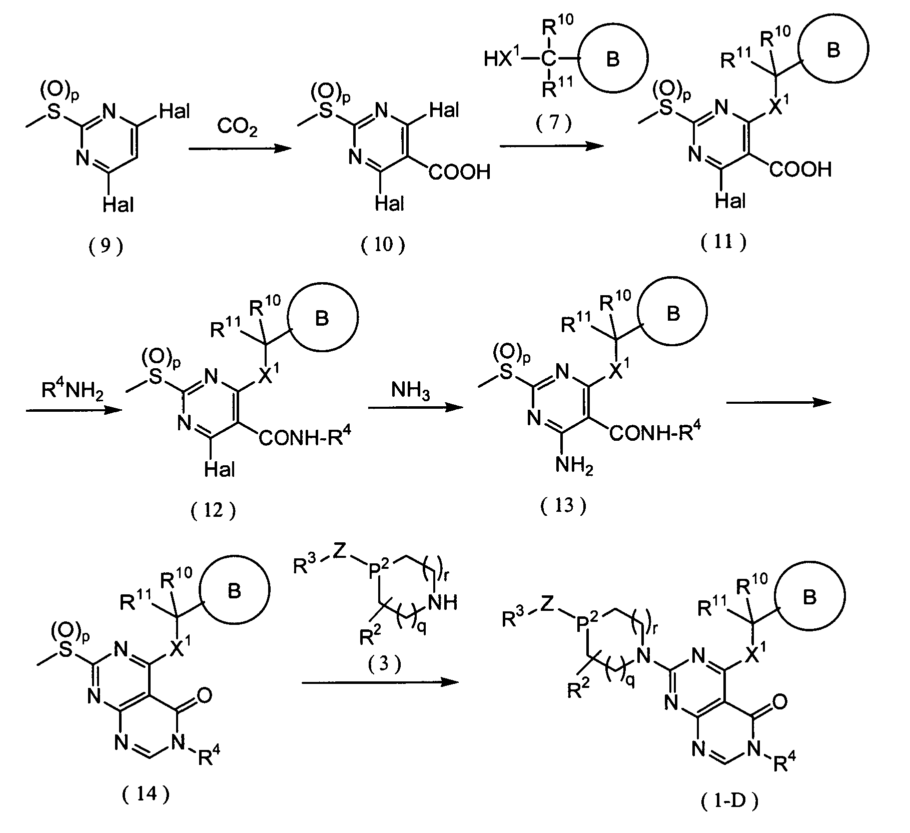 wherein p is 0, 1 or 2 and other symbols are the same as described above.

Compound(10) is obtained by treating Compound(9) with a base(n-butyl lithium, LDA etc.) in a solvent(THF, diethyl ether, dioxane etc.) at -78°C to ice cooling and reacting it with carbon dioxide at the same temperature for 1-12 hours.

Compound(11) is obtained by reacting Compound(10) with Compound(7) in the same manner as Method 1.

The reaction of Compound(11) and R⁴NH₂ can be carried out in the same manner as Method 2. In addition, acid halide of Compound(11) can be prepared by treating with a halogenating agent(e.g., thionyl chloride etc.) in the usual manner.

Compound(13) is obtained by reacting Compound(12) with ammonia in a solvent(THF, diethyl ether, dioxane etc.) at 0°C to reflux temperature of the solvent for an hour to 10 days.

Compound(14) is obtained by reacting Compound(13) with trialkyl orthoformate(e.g. trimethyl orthoformate, triethyl orthoformate, tripropyl orthoformate, tributyl orthoformate etc.) in the presence of an acid(e.g. acetic acid, acetic anhydride, hydrochloric acid, sulfuric acid etc.) at room temperature to 150°C for 1-12 hours.

According to a method described in WO 01/83460, Compound(1-D) is obtained by reacting Compound(14) with Compound(3) in a solvent(DMF, DMSO, chloroform, methylene chloride, THF etc.) in the presence of a base(triethylamine, diisopropylethylamine, pyridine etc.) at 0°C to 100°C for an hour to 2 days. Compound(14) in which p is 0 can be used in the present reaction after being converted to Compound(14) in which p is 1 or 2, by treating with an oxidizing agent(m-chloro perbenzoic acid, hydrogen peroxide etc.) in the usual manner.

Method 5: Compound(1) wherein the ring A is a group (H), X is -N(R⁷)-or -O-, Y is -C(R¹⁰)(R¹¹)- and P¹ is N can be prepared by the following method; 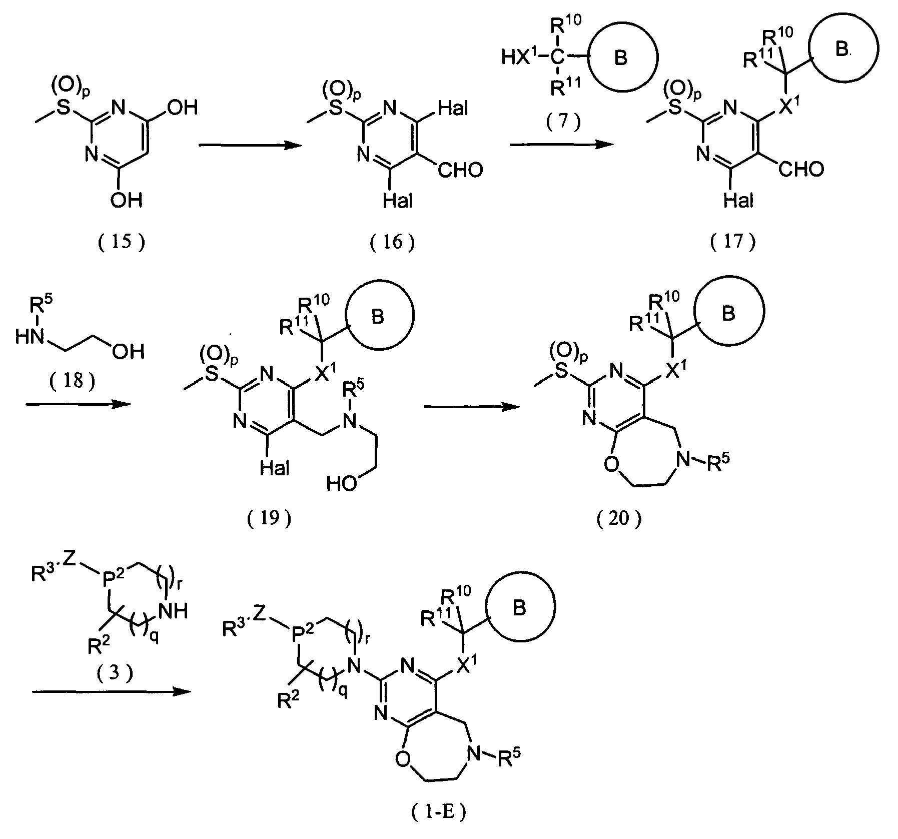 wherein each symbol is the same as described above.

Compound(16) is obtained by reacting Compound(15) with phosphorous oxychloride or phosphorous oxybromide at room temperature to reflux temperature of the solvent for 1-12 hours.

The reaction of Compound(16) and Compound(7) can be carried out in the same manner as Method 1.

Compound(19) is obtained by reacting Compound(17) and Compound(18) with a reducing agent(sodium borohydride, sodium cyanoborohydride, sodium triacetoxyborohydride etc.)in a solvent(methanol, ethanol, isopropyl alcohol, chloroform, methylene chloride, DMF, DMSO, THF, dioxane etc.), in the presence of an acid(hydrochloric acid, sulfuric acid, acetic acid, trifluoroacetic acid etc.) if necessary. The present reaction can be carried out at room temperature to reflux temperature of the solvent for 30 minutes to 2 days.

Compound(20) is obtained by treating Compound(19) in a solvent(THF, dioxane, diethyl ether, DMF, DMSO etc.) in the presence of a base(triethylamine, pyridine, sodium hydride, potassium t-butoxide, sodium t-butoxide etc.) at room temperature to reflux temperature of the solvent for 1-24 hours.

Compound(1-E) is obtained by reacting Compound(20) with Compound(3) in the same manner as Method 1.

Method 6: Compound(6) wherein the ring A is a group (I) or (J), can be prepared by the following method; 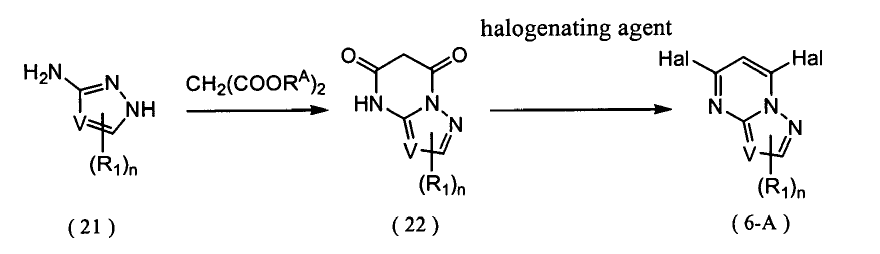 wherein V is CH or N, R^{A} is alkyl and the other symbols are the same as described above.

Compound(22) is obtained by reacting Compound(21) with dialkyl malonate in a solvent(methanol, ethanol, isopropyl alcohol, etc.) in the presence of a base(sodium methoxide, sodium ethoxide, potassium methoxide etc.) at room temperature to reflux temperature of the solvent for 1-48 hours.

Compound(6-A) is obtained by reacting Compound(22) in the same manner as the halogenating reaction of Method 3.

Method 7: Compound(6) wherein the ring A is a group (A) or (B), can be prepared by the following method; 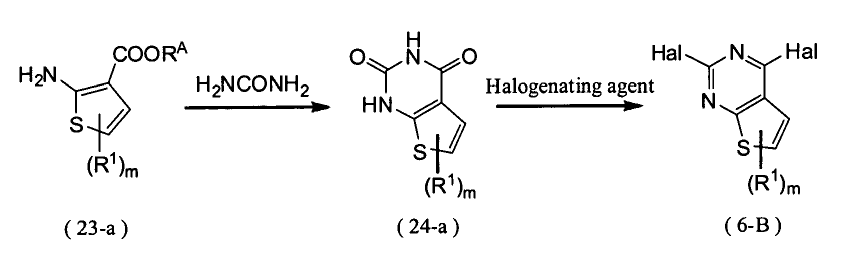 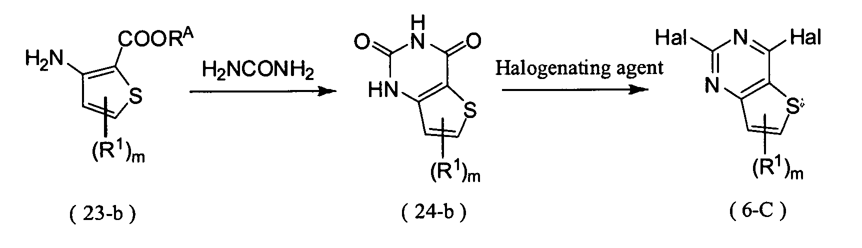 wherein each symbol is the same as described above.

Compound(24-a) or Compound(24-b) is obtained by reacting Compound(23-a) or Compound(23-b) with urea at 100 to 250°C for 1-12 hours.

Compound(6-B) or Compound(6-C) is obtained by reacting Compound(24-a) or Compound(24-b) in the same manner as the halogenating reaction of Method 3.

Method 8: Compound(6) wherein the ring A is a group (F), can be prepared by the following method; 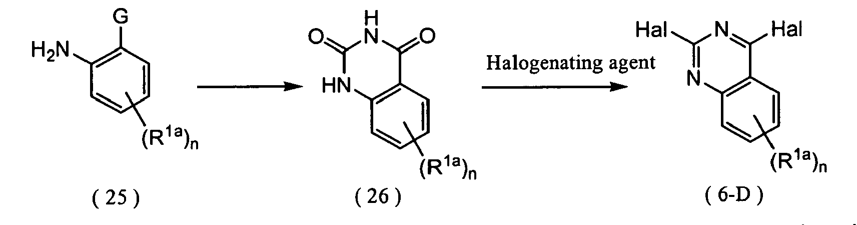 wherein G is carboxy or cyano and the other symbols are the same as described above.

Compound(26) is prepared according to a method described in Tetrahedron, 58, (2002) 3155-3158 or WO 95/32205. That is, Compound(26) is obtained (1) by reacting Compound(25) wherein G is cyano, with carbon dioxide in a solvent(DMF, DMSO, THF etc.) in the presence of excesive amount of a base(DBU, DBN etc.) at room temperature to 100°C for 1-48 hours, or (2) by reacting Compound(25) wherein G is carboxy, with urea at 100 to 250°C for 1-12 hours.

Compound(6-D) is obtained by reacting Compound(26) in the same manner as the halogenating reaction of Method 3.

Method 9: Compound(6) wherein the ring A is a group (E), can be prepared by the following method; 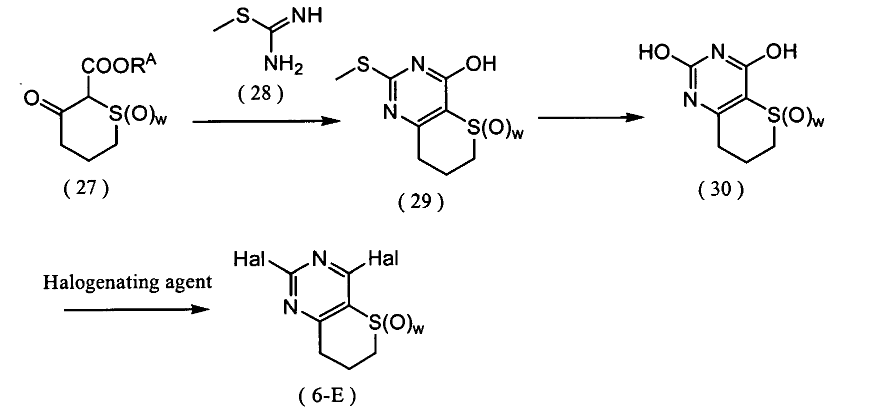 wherein each symbol is the same as described above.

The present reaction is carried out by a method described in Japanese Provisional Patent Publication No. 58-146586. That is, Compound(29) is obtained by reacting Compound(27) with Compound(28) or the salt thereof(hydrochloride, sulfate etc.) in a solvent(methanol, ethanol, isopropyl alcohol, DMF, DMSO etc.) in the presence of a base(sodium hydroxide, potassium hydroxide, sodium carbonate, potassium carbonate, sodium hydride, sodium methoxide, sodium ethoxide etc.) at room temperature to 100°C for 1-12 hours.

Compound(30) is obtained by reacting Compound(29) with an acid(acetic acid, hydrochloric acid, sulfuric acid etc.) or an alkali(sodium hydroxide, potassium hydroxide etc.) at room temperature to reflux temperature of the solvent for an hour to 3 days.

Compound(6-E) is obtained by reacting Compound(30) in the same manner as the halogenating reaction of Method 3.

Method 10: Compound(1) wherein the ring A is a group (E)and w is 1 or 2, can be prepared by the following method; 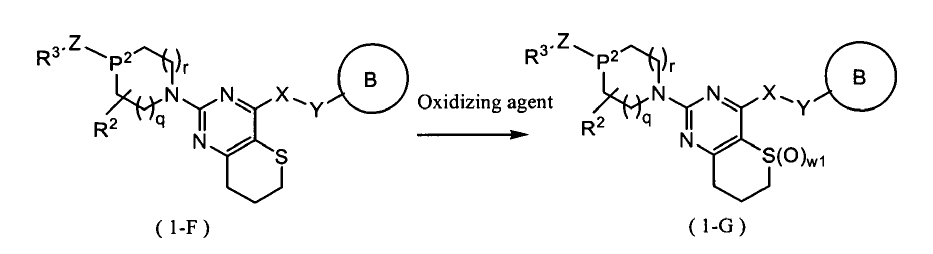 wherein w1 is 1 or 2 and the other symbols are the same as described above.

Compound(1-G) is obtained by reacting Compound(1-F) with an oxidizing agent(m-chloroperbenzoic acid, hydrogen peroxide etc.) in a solvent(acetic acid, dioxane, chloroform, methylene chloride etc.) at 0-100°C for 30 minutes to 24 hours.

Method 11: Compound(4) wherein P¹ is CH, X is -N(R⁷)- or -O- and Y is -C(R¹⁰)(R¹¹)- can be prepared by the following method; 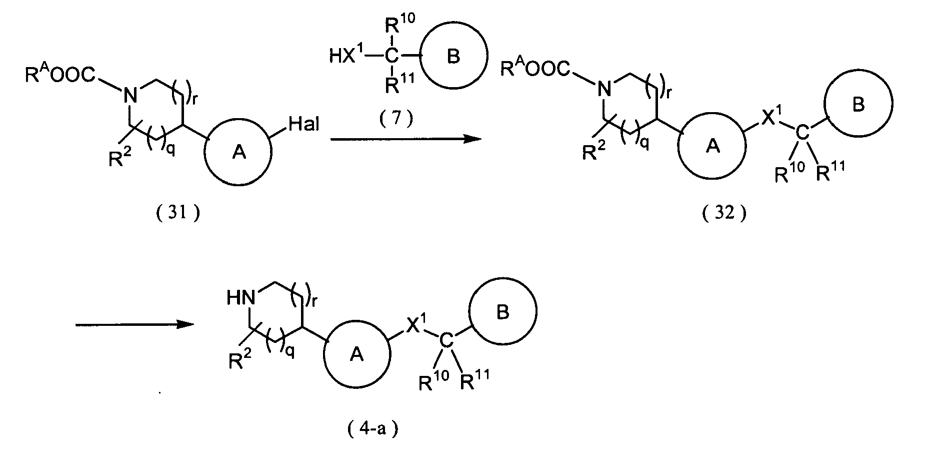 wherein each symbol is the same as described above.

The reaction of Compound(31) and Compound(7) can be carried out in the same manner as Method 1.

Comound(4-a) is obtained by treating Compound(32) with a base(sodium hydroxide, potassium hydroxide, sodium carbonate, potassium carbonate etc.) in a solvent(methanol, ethanol, isopropyl alcohol etc.) at room temperature to reflux temperature of the solvent for 1-24 hours.

Method 12: Compound(31) wherein the ring A is a group (I) or (J) can be prepared by the following method; 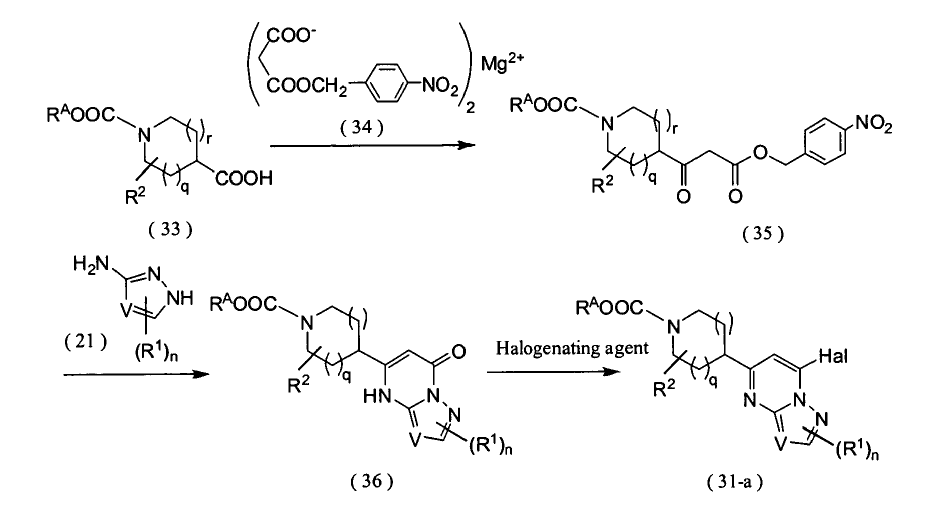 wherein each symbol is the same as described above.

Compound(35) is obtained by treating Compound(33) with carbonyldiimidazole, and then reacting it with Compound(34) in a solvent(THF, dioxane etc.). The present reaction can be carried out at room temperature to 100°C for 1-12 hours.

Compound(36) is obtained by reacting Compound(35) with Compound(21) in a solvent(acetic acid etc.) or without a solvent at 50-150°C for 1-48 hours.

Compound(31-a) is obtained by reacting Compound(36) in the same manner as the halogenating reaction of Method 3.

Method 13: Compound(1) wherein the ring A is a group (A), X is-N(R⁷)- or -O-, Y is -C(R¹⁰)(R¹¹)-, m is 1 and R¹ is alkoxycarbonyl can be prepared by the following method; 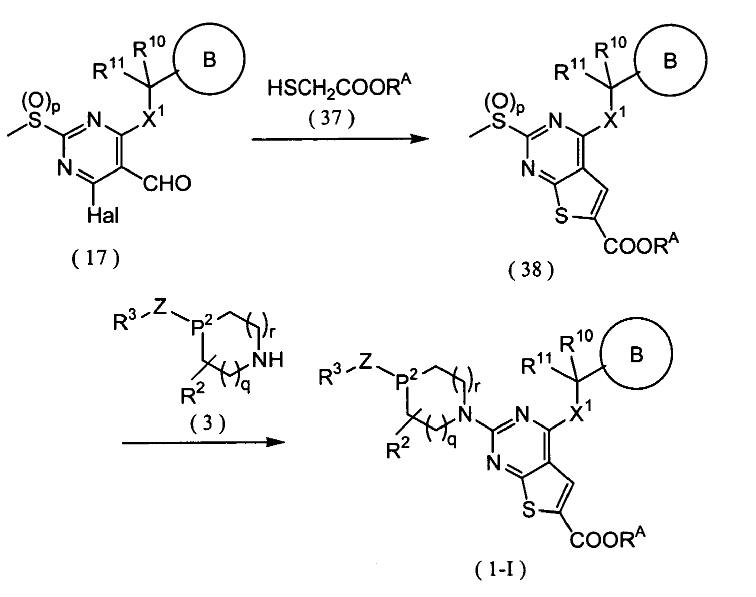 wherein each symbol is the same as described above.

Compound(38) is obtained by reacting Compound(17) with Compound(37) in a solvent(methanol, ethanol, isopropyl alcohol etc.) in the presence of a base(triethylamine, diisopropylethylamine, pyridine etc.) at 0-100°C for 1-12 hours.

Compound(1-I) is obtained by reacting Compound(38) with Compound(3) in the same manner as Method 1.

Method 14: Compound(25) wherein G is cyano and R^{1a} is alkoxycarbonyl can be prepared by the following method; 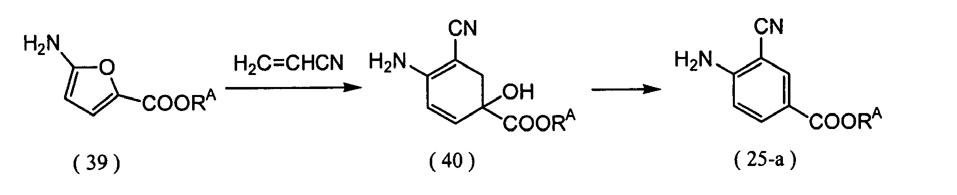 wherein each symbol is the same as described above.

Compound(40) is obtained by reacting Compound(39) with acrylonitrile in a solvent(benzene, toluene, xylene, chloroform, methylene chloride etc.) at room temperature to reflux temperature of the solvent for 1-24 hours.

Compound(25-a) is obtained by reacting Compound(40) with boron trifluoride diethyl ether complex preferably at reflux temperature of the solvent for 1-12 hours.

Method 15: Compound(3) wherein P¹ and P² are N and Z is -CO- can be prepared by the following method; 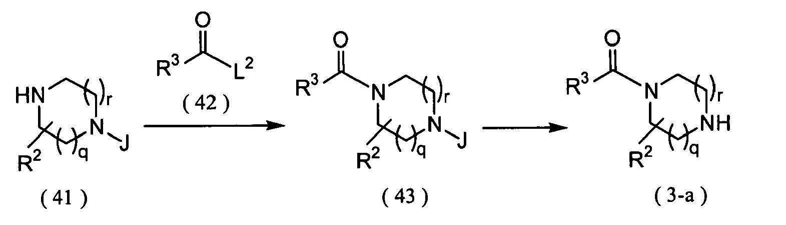 wherein J is an amino-protecting group such as benzyloxycarbonyl or Boc, L² is a leaving group such as halogen or alkoxy and the other symbols are the same as described above.

Compound(43) is obtained by reacting Compound(41) with Compound(42) in the same manner as Method 1.

Compound(3-a) is obtained by deprotection of Compound(43) according to a conventional method such as catalytic reduction using palladium-carbon or treatment with an acid(trifluoroacetic acid, hydrochloric acid etc.).

Method 16: a compound(1) wherein the ring A is a group (L), P¹ is N, X is -N(R⁷)- or -O- and Y is -C(R¹⁰)(R¹¹)- can be prepared by the following method; 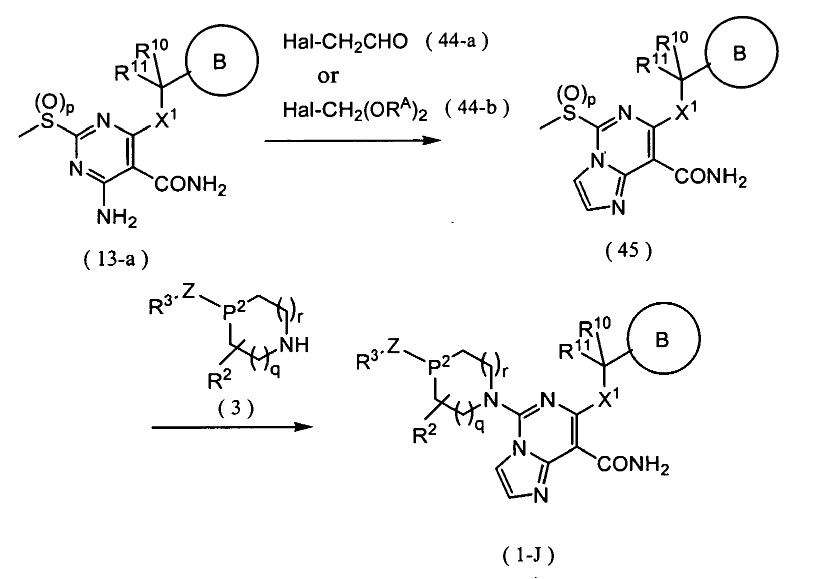 wherein each symbol is the same as described above.

Compound(45) is obtained by reacting Compound(13-a) with Compound(44-a) or Compound(44-b) in a solvent(THF, dioxane, DMF, DMSO, methanol, ethanol, isopropyl alcohol etc.) in the presence of a base(triethylamine, diisopropylethylamine, pyridine etc.) if necessary, at room temperature to reflux temperature of the solvent for 1-24 hours.

Compound(1-J) is obtained by reacting Compound(45) with Compound(3) in the same manner as Method 1.

Method 17: Compound(1) wherein the ring A is a group (N), P¹ is N, X is -N(R⁷)- or -O- and Y is -C(R¹⁰)(R¹¹)- can be prepared by the following method; 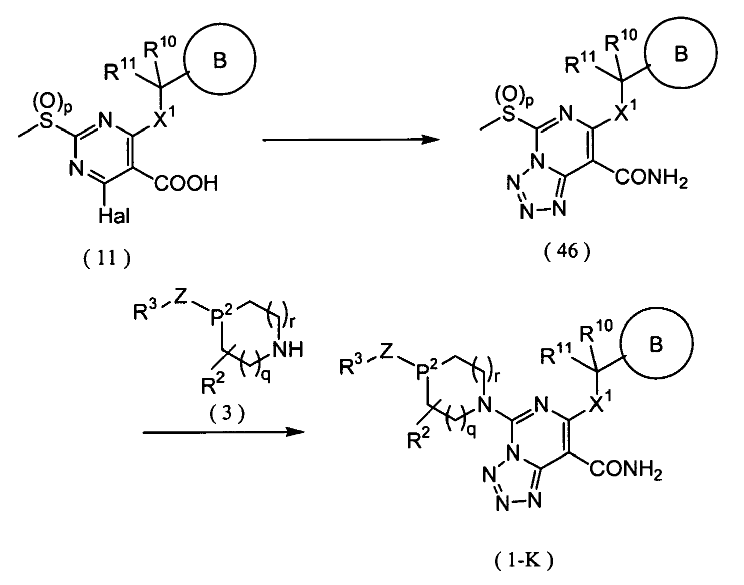 wherein each symbol is the same as described above.

Compound(46) is obtained by reacting the compound(11) with a metallic salt of azide(sodium azide, potassium azide etc.) in a solvent(DMF, DMSO, THF, dioxane etc.) at room temperature to 100°C for 1-12 hours.

Compound(1-K) is obtained by reacting Compound(46) with Compound(3) in the same manner as Method 1.

Method 18: Compound(1) wherein the ring A is a group (O), P¹ is N, X is -N(R⁷)- or -O- and Y is -C(R¹⁰)(R¹¹)- can be prepared by the following method; 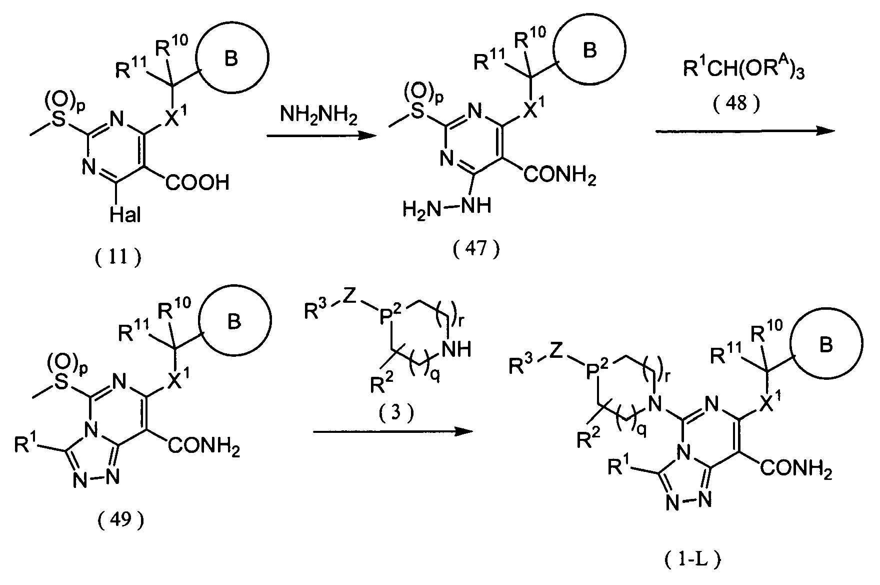 wherein each symbol is the same as described above.

Compound(47) is obtained by reacting Compound(11) with hydrazine in a solvent(DMF, DMSO, methanol, ethanol etc.) at 0-50°C for 1-24 hours.

Compound(49) is obtained by reacting Compound(47) with Compound(48) in a solvent(THF, DMF, DMSO, methanol, ethanol etc.) or without a solvent at room temperature to reflux temperature of the solvent for 1-24 hours.

Compound(1-L) is obtained by reacting Compound(49) with Compound(3) in the same manner as Method 1.

Method 19: Compound(1) wherein the ring A is a group (M), P¹ is N, X is -N(R⁷)- or -O- and Y is -C(R¹⁰)(R¹¹)- can be prepared by the following method; 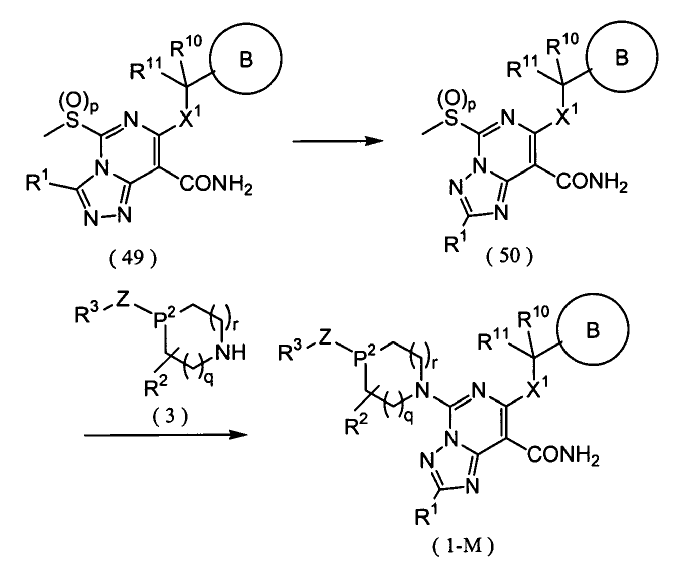 wherein each symbol is the same as described above.

Compound(50) is obtained by heating Compound(49) in a solvent(DMF, DMSO, THF, dioxane etc.).

Compound(1-M) is obtained by reacting Compound(50) with Compound(3) in the same manner as Method 1.

Method 20: Compound(1) wherein the ring A is a group (P), (Q) or (R) can be prepared by a method described in WO 01/83460 and EP 1195378 or by a method described above.

Method 21: Compound(6) wherein the ring A is a group (C) and Lv is halogen can be prepared by the following method; 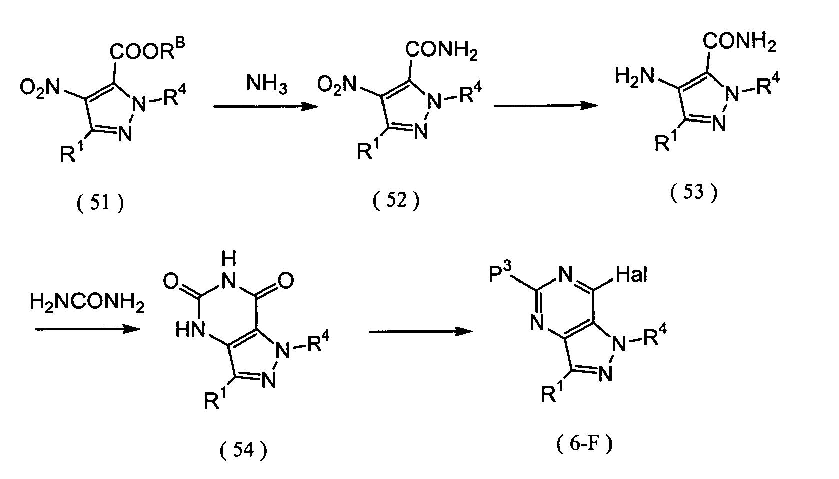 wherein R^{B} is hydrogen or alkyl, P³ is halogen and the other symbols are the same as described above.

Compound(51) is treated with ammonia water according to a conventional method to give Compound(52), and then Compound (52) is reduced in a solvent(e.g., water, methanol, ethanol, tert-butyl alcohol, THF, dioxane, ethyl acetate, acetic acid, xylene, DMF, DMSO or a mixture thereof) to give Compound(53). The reducing reaction can be carried out by using a reducing agent such as sodium borohydride, lithium borohydride, or lithium aluminum hydride etc., by using a metal such as iron, zinc or stannum etc., or by catalytic reduction using a transition metal such as palladium-carbon, platinum oxide, Raney nickel, rhodium or ruthenium etc. In addition, when a catalytic reduction is carried out, hydrogen source may be formic acid, ammonium formate, 1,4-cyclohexadiene etc. The present reaction can be carried out usually at -20 to 150°C for 30 minutes to 48 hours.

Compound(54) is obtained by reacting Compound(53) with urea at 100-250°C for 1-12 hours.

Compound(6-F) is obtained by reacting Compound(54) with a halogenating agent(phosphorous oxychloride, phosphorous oxybromide etc.) in a solvent(benzene, toluene, xylene, chloroform, methylene chloride, acetonitrile, DMF etc.) or without a solvent in the presence of a base(dimethylaniline, diethylaniline, triethylamine, collidine, pyridine, diisopropylethylamine etc.) if necessary, at room temperature to reflux temperature of the solvent for 1-12 hours.

Method 22: Compound(6) wherein the ring A¹ is a group (D) and Lv is halogen can be prepared by the following method; 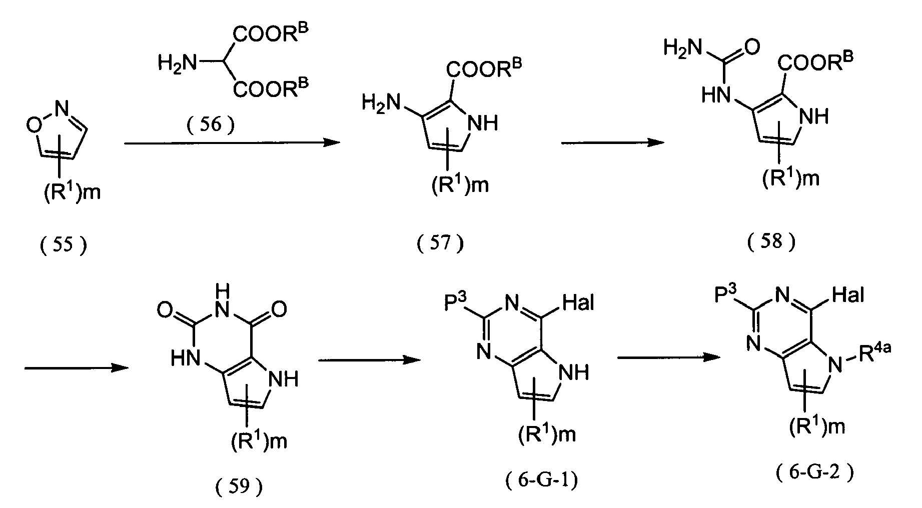 wherein R^{4a} is alkyl and the other symbols are the same as described above.

Compound(57) is obtained by reacting 3-oxopropionitrile derivative, which is produced by treating Compound(55) with a strong base(sodium methoxide, potassium methoxide, sodium ethoxide, potassium ethoxide etc.) in a solvent(methanol, ethanol etc.), with Compound(56) in the presence of a weak base(sodium acetate, potassium acetate, sodium carbonate, potassium carbonate etc.).

Compound(58) is obtained by reacting Compound(57) with a cyanate(sodium cyanate, potassium cyanate etc.) in a solvent(methanol, ethanol, acetic acid, water or a mixture thereof). The present reaction can be carried out at 0-100°C, preferably at room temperature for 1-12 hours.

Compound(59) is obtained by treating Compound(58) with a base(sodium hydroxide, potassium hydroxide, sodium carbonate, potassium carbonate etc.) in a solvent(water, methanol, ethanol, DMSO, DMF etc.). The present reaction can be carried out at 0-150°C, preferably at reflux temperature of the solvent for 1-12 hours.

Compound(6-G) is obtained by reacting Compound(59) with a halogenating agent in the same manner as Method 21.

Method 23: In the above methods, when the compound of the present invention, an intermediate thereof, or the starting compound has a functional group (hydroxy group, amino group, carboxy group, etc.), the functional group is protected with an ordinary protective group in the field of the organic synthetic chemistry in accordance with the method disclosed in "Protective Groups in Organic Synthesis" T. W. Greene, P. M. G. Wuts, John Wiley and Sons 1991, and then the reaction is carried out and is followed by cleavage of the protective group to give the object compound.

As the protective group, the protective groups described in the above text book and ordinarily used in the field of the organic synthetic chemistry are illustrated. For example, as protective groups of hydroxy group, tetrahydropyranyl, trimethylsilyl, tert-butyldimethylsilyl, benzyl, methoxymethyl, acetyl and so on, as protective groups of amino group, tert-butoxycarbonyl, benzyloxycarbonyl, 9-fluorenylmethoxycarbonyl, 2,2,2-trichloroethoxycarbonyl, tert-amyloxycarbonyl and so on, and as protective groups of carboxy group, alkyl group like methyl or ethyl, benzyl and so on are respectively illustrated.

Furthermore, after preparing the compound of the present invention or the intermediate thereof, the functional group is converted or modified in accordance with the conventional method. The following methods are illustrated.

### (1) Conversion of amino into amide.

The amino group can be converted into the corresponding amide group by reacting amino group with an acyl halide, or by condensing carboxy group with an amine in the presence of condensing agent.

### (2) Conversion of carboxy or ester thereof into carbamoyl.

The carboxy group can be converted into the corresponding carbamoyl group by converting carboxy group into acyl halide and then reacting it with an amine, by reacting carboxy group with an amine in the presence of a condensing agent, or by reacting the ester with an amine.

### (3) Hydrolysis of ester.

The ester can be converted into the corresponding carboxy by hydrolysis of ester in an alkali (sodium hydroxide, potassium hydroxide, etc.) or an acid (hydrochloric acid, sulfuric acid, etc.).

### (4) Conversion of carbamoyl into nitrile.

The carbamoyl can be converted into the corresponding nitrile by reacting carbamoyl with phosphorous oxychloride or trifluoroacetic anhydride.

### (5) N-Alkylation or N-phenylation.

The amino group can be converted into the corresponding mono- or di-alkylated amino group or phenylated amino group by reacting amino group with an alkyl halide or a phenyl halide.

In addition, the amino group can be converted into the corresponding mono- or di-alkylated amino group by reductive amination.

### (6) N-Sulfonylation.

The amino group can be converted into the corresponding alkylsulfonylamino group or phenylsulfonylamino group by reacting amino group with an alkylsulfonyl halide or a phenylsulfonyl halide.

### (7) Conversion of amine into ureido.

The amino group can be converted into an alkyl ureido by reacting amino group with alkyl isocyanate. The amino group can be converted into ureido by reacting amino group with carbamoyl halide or by reacting the isocyanate, which is converted from the amino, with an amine.

### (8) Conversion of aromatic nitro compound into aromatic amine.

The aromatic nitro compound can be converted into the corresponding aromatic amine by conventionally reducing it with a reducing agent such as a metal reducing agent (e.g., sodium borohydride, lithium borohydride, lithium aluminum hydride), metals (Fe, Zn, Sn, SnCl₂, Ti), or by catalytic reduction of it under transition metal catalyst (e.g., palladium-carbon, Pt, Raney-nickel). In case of catalytic reduction, ammonium formate, hydrazine and so on can be used as hydrogen source.

Furthermore, the compound of the present invention or the intermediate thereof prepared by the above methods is purified by the conventional method such as column chromatography, or recrystallization, etc. As the solvent for recrystallization, an alcohol solvent such as methanol, ethanol or 2-propanol, an ether solvent such as diethyl ether, an ester solvent such as ethyl acetate, an aromatic solvent such as toluene, a ketone solvent such as acetone, a hydrocarbon solvent such as hexane, water and so on, or a mixture thereof are illustrated. Furthermore, the compound of the present invention can be converted into its pharmaceutically acceptable salt by the conventional method and thereafter, can be subjected to recrystallization.

### EFFECT OF INVENTION

Since the compound of the present invention or its pharmaceutically acceptable salt has an activity for controlling the function of CCR4, or TARC/CCL17 and/or MDC/CCL22, it is useful as a prophylactic or treatment agent for allergic diseases, inflammatory diseases, autoimmune diseases and cancer diseases such as asthma (e.g., bronchial asthma), allergic rhinitis, allergic conjunctivitis, pollen allergy, dermatitis (atopic dermatitis, contact dermatitis, etc.), psoriasis, rheumatoid arthritis, systemic lupus erythematosus, multiple sclerosis, insulin dependent diabetes mellitus (IDDM), rejection on organ transplantation, inflammatory bowel disease (ulcerative colitis, Crohn's disease), interstitial crystitis, glomerulonephritis, sepsis, pain, adult T cell leukemia (ATL), malignant tumor, pulmonary fibrosis, eosinophilic pneumonia, pulmonary eosinophilic granuloma, cutaneous T cell lymphoma, ankylosing spondylitis, coronary artery disease, pemphigoid, Hodgkin's disease, etc.

The compound of the present invention or its pharmaceutically acceptable salt can be formulated in a medicament consisting of a therapeutically effective amount of said compound and a pharmaceutically acceptable carrier(s). The pharmaceutically acceptable carrier(s) are a diluent, a binder (syrup, gum arabic, gelatin, solbit, tragacanth gum, polyvinyl pyrrolidone, etc.), an excipient (lactose, sucrose, corn starch, potassium phosphate, solbit, glycine, etc.), a lubricant (magnesium stearate, talc, polyethylene glycol, silica, etc.), a disintegrant (potato starch), a humectant (sodium lauryl sulfate), and so on.

The compound of the present invention or its pharmaceutically acceptable salt can be orally or parenterally administered in an appropriate preparation form. The preparation suitable for oral application includes, for example solid preparations such as tablets, granules, capsules, powders, etc., solutions, suspensions, emulsions and so on. The preparation suitable for parenteral administration includes suppositories, injections or solutions for infusion containing distilled water for injection, physiological saline or an aqueous sucrose solution, preparations for inhalation and so on.

The dose of the compound of the present invention or its pharmaceutically acceptable salt varies depending on application route, age, body weight or condition of the patient, but usually, about 0.003 to 100mg/kg/day, preferably about 0.01 to 30mg/kg/day, and especially preferably about 0.05 to 10mg/kg/day.

### BEST MODE FOR CARRYING OUT THE INVENTION

The present invention is illustrated more in detail below by examples and reference examples, but not limited to these examples.

### EXAMPLE

### Example 1

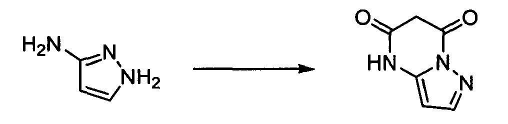

(1) A solution of sodium methylate (28%, 77.2 g) in methanol was added to a solution of 3-aminopyrazole(16.0 g) and dimethyl malonate(26.4 g) in methanol(500 mL) at room temperature and the mixture was refluxed under heating with stirring for 18 hours. After standing to cool, the reaction solution was concentrated under reduced pressure, allowed to stand for an hour, and insoluble material was filtered off and dried. The insoluble materials was dissolved in of water(500 mL) and pH was adjusted to 3 by adding 6N hydrochloric acid. The precipitated crystals were filtered, washed with water, ethanol and diethyl ether successively and dried to give 4H-pyrazolo[1,5-a]pyrimidine-5,7-dione(22.1 g) as a colorless powder.
   APCI-MS(m/e):152[M+H]⁺. 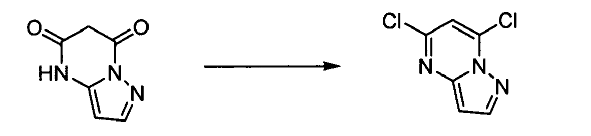
(2) Diethyl aniline(3.6 mL) and phosphorous oxychloride(9.3 mL) were added to 4H-pyrazolo[1,5-a]pyrimidine-5,7-dione(1.7 g) and the mixture was stirred at 70-75°C for an hour. the reaction solution was concentrated under reduced pressure and azeotropically distilled with toluene twice. The half amount of the residue was dissolved in ethyl acetate, poured into ice and an aqueous saturated sodium bicarbonate solution and then extracted with ethyl acetate. The organic layer was washed with brine, dried over sodium sulfate and filtered. The filtrate was concentrated under reduced pressure. The residue was purified with silica gel column chromatography(hexane/ethyl acetate=19/1 to 4/1) to give 5,7-dichloro-pyrazolo[1,5-a]pyrimidine(0.54 g) as a colorless solid.
   APCI-MS(m/e):188/190[M+H]⁺. 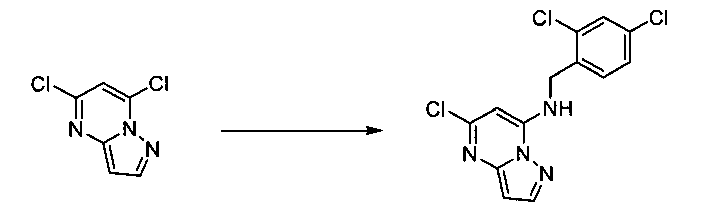
(3) 2,4-Dichlorobenzylamine(440 mg) and triethylamine(0.35 mL) were added to a solution of 5,7-dichloro-pyrazolo[1,5-a]pyrimidine(470 mg) in 1,4-dioxane(5 mL) and the mixture was stirred at 50-60°C for 4 hours. After standing to cool, an aqueous saturated sodium bicarbonate solution was added to the reaction solution and the mixture was extracted with ethyl acetate. The extract was dried over sodium sulfate and the solvent was distilled away. The residue was purified with silica gel column chromatography(hexane/ethyl acetate=9/1 to 4/1) to give (5-chloro-pyrazolo[1,5-a]pyrimidine-7-yl)-(2,4-dichlorobenzyl)-amine(697 mg) as a colorless solid.
   APCI-MS(m/e):327/329[M+H]⁺. 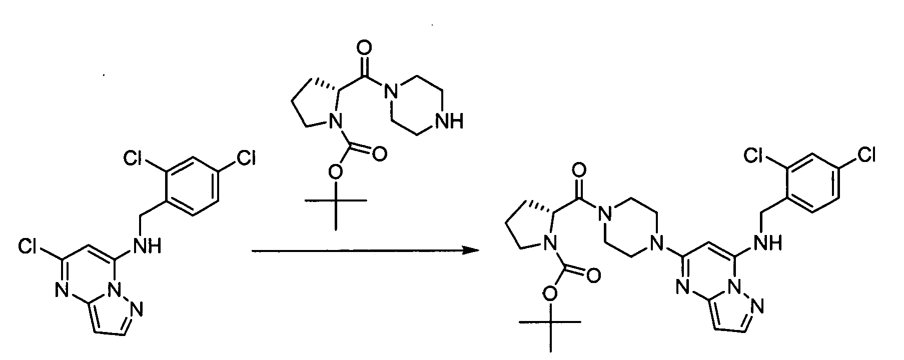
(4) 2-(Piperazin-1-carbonyl)-pyrrolidine-1-carboxylic acid tert-butyl ester(1.0 g) and diisopropylethylamine(0.31 mL) were added to a solution of (5-chloro-pyrazolo[1,5-a]pyrimidine-7-yl)-(2,4-dichlorobenzyl)-amine(290 mg) in 1,4-dioxane(3 mL) and the mixture was stirred under reflux for 17 hours. After standing to cool, ethyl acetate was added to the reaction solution and the mixture was washed with an aqueous saturated sodium bicarbonate solution and brine.
   The mixture was dried over sodium sulfate, filtered and the solvent was distilled away. The residue was purified with silica gel column chromatography(hexane/ethyl acetate=2/1 to 1/2) to give 2-{4-[7-(2,4-dichloro-benzylamino)-pyrazolo[1,5-a]pyrimidine-5-yl]-piperazine-1-carbonyl}-pyrrolidine-1-carboxylic acid tert-butyl ester(142 mg) as a colorless amorphous.
   APCI-MS(m/e):574/576[M+H]⁺. 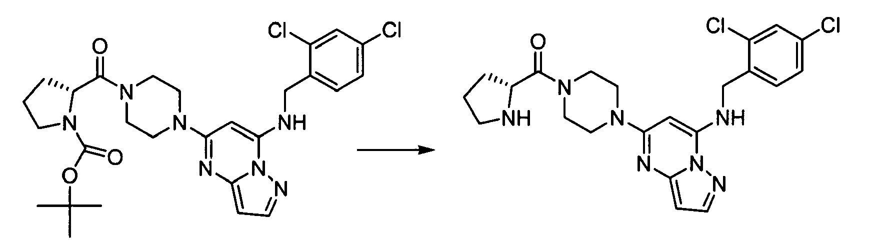
(5) 2-{4-[7-(2,4-Dichloro-benzylamino)-pyrazolo[1,5-a]pyrimidine-5-yl]-piperazine-1-carbonyl}-pyrrolidine-1-carboxylic acid tert-butyl ester(142 mg) was dissolved in methylene chloride(6 mL), trifluoroacetic acid(0.5 mL) was added thereto under ice-cooling and the mixture was stirred for 1.5 hours. Trifluoroacetic acid(0.5 mL) was further added thereto under ice-cooling and the mixture was stirred at room temperature for 4.5 hours. The reaction solution was concentrated under reduced pressure and ethyl acetate was added to the residue.
The mixture was washed with an aqueous saturated sodium bicarbonate solution and brine and dried over sodium sulfate. The solvent was distilled away, the residue was freeze-dried from tert-butanol to give {4-[7-(2,4-dichloro-benzylamino)-pyrazolo[1,5-a]pyrimidin-5-yl]-piperazin-1-yl}-pyrrolidin-2-yl-methanone(111 mg) as a colorless amorphous.
APCI-MS(m/e):474/476[M+H]⁺.

### Examples 2-15

The following compounds were obtained by reacting and treating in the same manner as Example 1.

**[Table 1]**

| | | | | | |
|---|---|---|---|---|---|
| 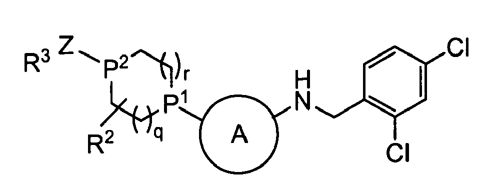 | | | | | |

| Example | R³ | Z | 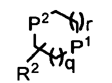 | Ring A | MS([M+H]⁺) |
|---|---|---|---|---|---|
| 2 | 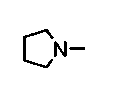 | -(CH₂)₂⁻ | 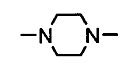 | 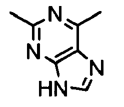 | 475/477, APCI |
| 3 | 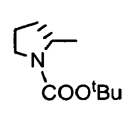 | -CO- | 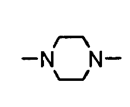 | 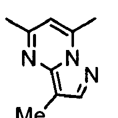 | 588/590, APCI |
| 4 | 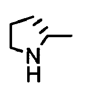 | -CO⁻ | 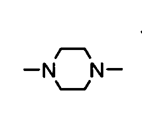 | 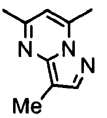 | 488/490, APCI |
| 5 | 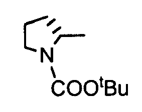 | -CO⁻ | 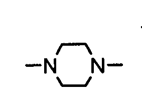 | 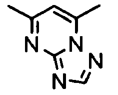 | 575/577, APCI |
| 6 | 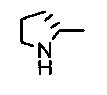 | -CO⁻ | 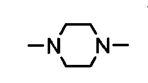 | 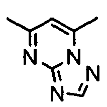 | 475/477, APCI |
| 7 | 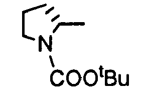 | -CO⁻ | 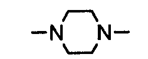 | 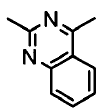 | 585/587, APCI |
| 8 | 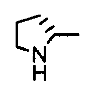 | -CO⁻ | 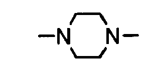 | 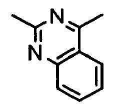 | 485/487, APCI |
| 9 | HO⁻ | -CH₂⁻ | 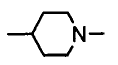 | 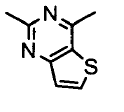 | 423/425, APCI |
| 10 | 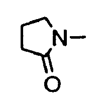 | -CH₂CO⁻ | 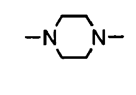 | 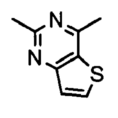 | 519/521, APCl |

**[Table 2]**

| | | | | | |
|---|---|---|---|---|---|
| 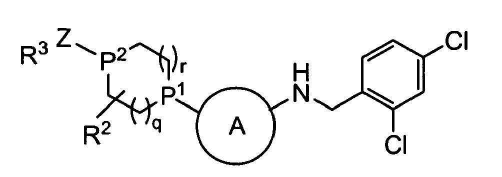 | | | | | |

| Example | R³ | Z | 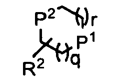 | Ring A | MS([M+H]⁺) |
|---|---|---|---|---|---|
| 11 | 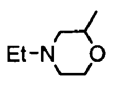 | -CO⁻ | 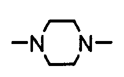 | 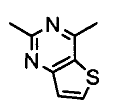 | 535/537, APCI |
| 12 | 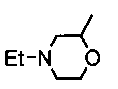 | -CO⁻ | 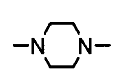 | 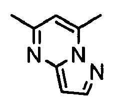 | 518/520, APCI |
| 13 | 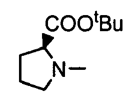 | -CH₂CO⁻ | 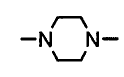 | 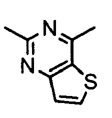 | 605/607, APCI |
| 14 | 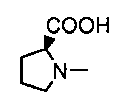 | -CH₂CO⁻ | 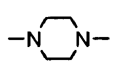 | 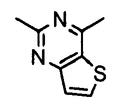 | 549/551, APCI |
| 15 | 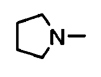 | -CH₂CO⁻ | 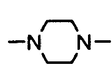 | 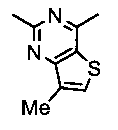 | 519/521, APCl |

### Example 16

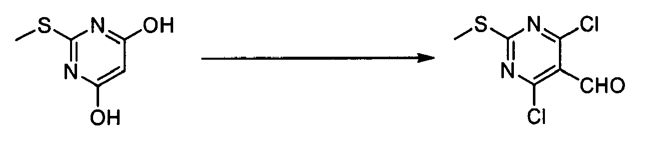

(1) Phosphorous oxychloride(144.1 g) was cooled to 0°C and DMF(28 mL) was added dropwise. The mixture was stirred at room temperature for 30 minutes, and 4,6-dihydroxy-2-mercaptopyrimidine(20 g) was added thereto. After stirring at room temperature for an hour, the reaction solution was stirred under reflux for 6 hours. The reaction solution was concentrated, toluene was added to the residue and the mixture was concentrated again. Water and ethyl acetate were added to the residue and the organic layer was separated and dried. The solvent was concentrated, the residue was purified with silica gel column chromatography(hexane/ethyl acetate=95/5 to 80/20) to give 4,6-dichloro-2-methyl-sulfanylpyridine-5-carboaldehyde(11.62 g) as a colorless powder.
   GC-MS:222/224[M]. 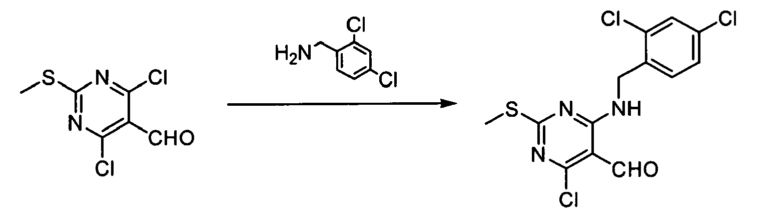
(2) A solution of 2,4-dichlorobenzylamine(1.89 g) and triethylamine(1.36 g) in methanol(10 mL) was added dropwise to a solution of 4,6-dichloro-2-methylsulfanylpyridine-5-carboaldehyde(2.0 g) in methanol(80 mL) cooled at 0°C. The reaction mixture was stirred at room temperature for an hour, an aqueous saturated sodium bicarbonate solution and ethyl acetate were added thereto, and the organic layer was separated. The organic layer was dried and the solvent was concentrated. Diisopropyl ether and hexane were added to the residue and the precipitates were filtered and dried to give 4-chloro-6-(2,4-dichlorobenzylamino)-2-methylsulfanylpyrimidine-5-carboaldehyde(2.88 g) as a colorless powder.
   APCI-MS(m/e):362/364[M+H]⁺. 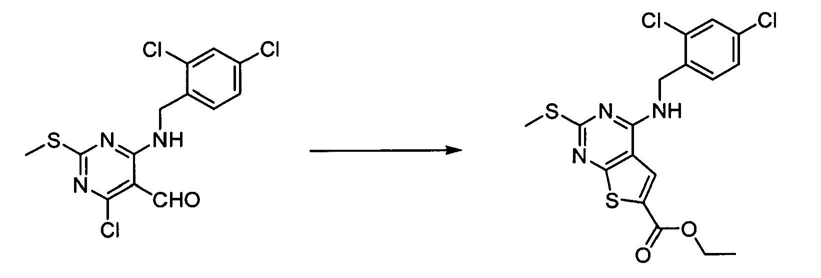
(3) Ethyl thioglycolate(0.4 mL) and triethylamine(0.92 mL) were added dropwise to a solution of 4-chloro-6-(2,4-dichlorobenzylamino)-2-methylsulfanylpyrimidine-5-carboaldehyde(1.20 g) in ethanol(15 mL) at room temperature and the mixture was stirred at 80°C for 2 hours. After standing to cool, precipitated crystals were filtered, washed with diisopropyl ether and dried. The crude crystals were dissolved in ethyl acetate, washed with water, dried over magnesium sulfate and the solvent was distilled away. The obtained crystals were washed with diethyl ether and dried to give 4-(2,4-dichloro-benzylamino)-2-methylsulfanyl-thieno[2,3-d]pyrimidine-6-carboxylic acid ethyl ester(1.13 g) as a colorless solid
   APCI-MS(m/e):428/430[M+H]⁺. 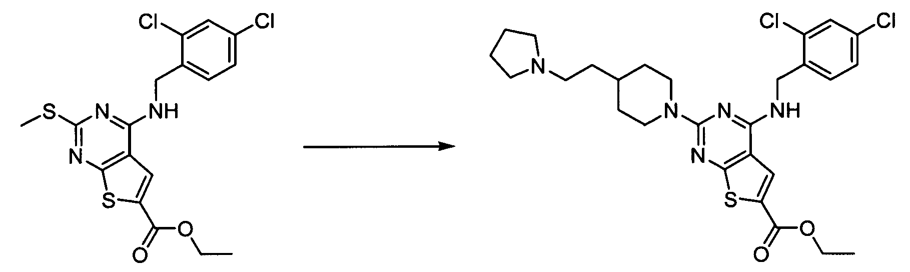
(4) m-Chloroperbenzoic acid(781 mg) was added to a solution of 4-(2,4-dichloro-benzylamino)-2-methylsulfanyl-thieno[2,3-d]pyrimidine-6-carboxylic acid ethyl ester(1.13 g) in DMF(20 mL)/chloroform(4 mL) at 0°C and the mixture was stirred for 2 hours. 4-(2-Pyrrolidinoethyl)piperidine(719 mg) and triethylamine(0.55 mL) were added to the reaction solution and the mixture was stirred at room temperature for a day and at 55°C for a day. After standing to cool, an aqueous 2% sodium carbonate solution was added to the reaction solution, and the mixture was extracted with ethyl acetate, dried over magnesium sulfate and filtered. The residue was purified with silica gel column chromatography(hexane/ethyl acetate=100/0 to 75/25) to give 4-(2,4-dichloro-benzylamino)-2-[4-(2-pyrrolidin-1-yl-ethyl)-piperidin-1-yl]-thieno[2,3-d]pyrimidine-6-carboxylic acid ethyl ester(1.14 g) as a pale yellow powder.
   APCI-MS(m/e):562/564[M+H]⁺.

### Example 17

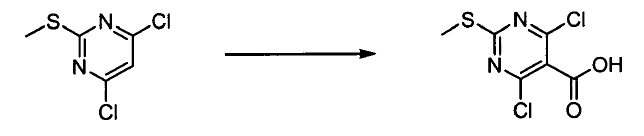

(1) n-Butyl lithium(1.7M hexane solution, 295 mL) was added dropwise to a solution of diisopropylamine(47.7 g) in THF(1.4 L) at -78°C under nitrogen atmosphere for 30 minutes and stirred for additional 30 minutes. A solution of 4,6-dichloro-2-methylsulfanyl-pyrimidine(40.0 g) in THF(200 mL) was added dropwise to the reaction solution for an hour and stirred for an additional hour. The reaction solution was poured into dry ice(800 g) slowly and the mixture was stirred for 2 hours. Hydrochloric acid(1N) was added slowly, ethyl acetate was added thereto and the mixture was washed with water and brine. The aqueous layer was extracted with ethyl acetate, the combined organic layer was dried over magnesium sulfate and the solvent was distilled away. Hexane was added and the solid was triturated and dried to give 4,6-dichloro-2-methylsulfanyl-pyrimidine-5-carboxylic acid(45.51 g) as a pale brown solid.
   APCI-MS(m/e):237/239[M-H]⁻. 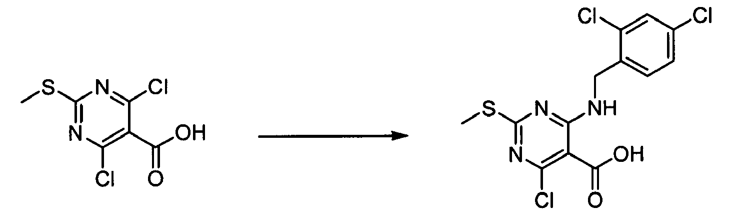
(2) A solution of 2,4-dichlorobenzylamine(33.5 g) in DMF(270 mL) was added dropwise to a solution of 4,6-dichloro-2-methylsulfanyl-pyrimidine-5-carboxylic acid(45.5 g) and triethylamine(53.1 g) in DMF(270 mL) for 30 minutes and the mixture was stirred at room temperature for 4 hours. An aqueous citric acid solution was added to the reaction solution and ethyl acetate/hexane was further added. The organic layer was washed with water and brine, dried over sodium sulfate and the solvent was distilled away. The residue was crystallized from ethyl acetate/hexane to give 4-chloro-6-(2,4-dichlorobenzylamino)-2-methylsulfanyl-pyrimidine-5-carboxylic acid(69.5 g) as pink crystals.
   APCI-MS(m/e):378/380[M-H]⁻. 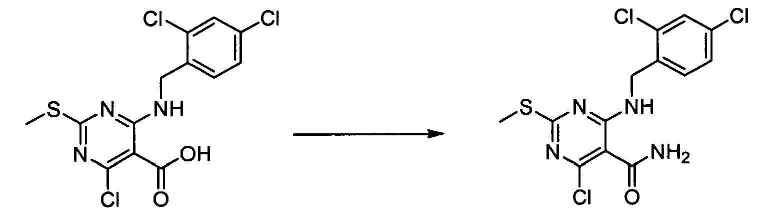
(3) Thionyl chloride(136 mL) was added to 4-chloro-6-(2,4-dichlorobenzylamino)-2-methylsulfanyl-pyrimidine-5-carboxylic acid(80.5 g) and the mixture was refluxed under heating for an hour. After standing to cool, the reaction solution was concentrated under reduced pressure. Methylene chloride(400 mL) was added to the residue, a solution of ammonia/1,4-dioxane was added thereto under ice-cooling and the mixture was stirred at room temperature for 3 hours. The reaction solution was washed with a saturated aqueous sodium bicarbonate solution and brine and dried over sodium sulfate. NH Silica gel was added, filtered and the solvent was distilled away. the residue was recrystallized from ethyl acetate/hexane to give 4-chloro-6-(2,4-dichlorobenzylamino)-2-methylsulfanyl-pyrimidine-5-carboxylic amide(54.6 g) as pale brown crystals.
   APCI-MS(m/e):377/379[M+H]⁺. 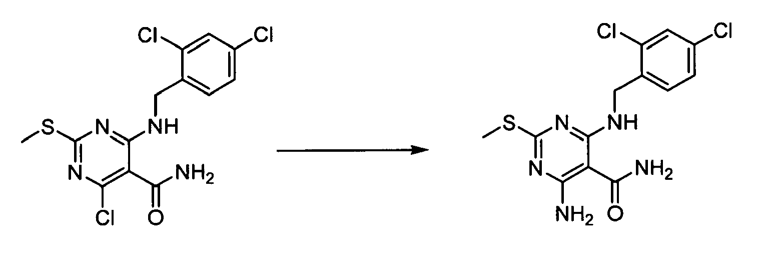
(4) A solution of 4-chloro-6-(2,4-dichlorobenzylamino)-2-methylsulfanyl-pyrimidine-5-carboxylic amide(20.0 g) in 1,4-dioxane(250 mL) was stirred and bubbled with ammonia gas at room temperature. A temperature of the solution was risen to 110°C and the stirring was continued for a week while being bubbled with ammonia gas sometimes. After standing to cool to room temperature, the mixture was refluxed under heating for an hour. After standing to cool, chloroform was added to the reaction solution, an insoluble material was filtered, washed with chloroform and dried. The insoluble material was dissolved in ethyl acetate/tetrahydrofuran and washed with a saturated aqueous sodium bicarbonate solution and brine. The mixture was dried over sodium sulfate and the solvent was distilled away to give 4-amino-6-(2,4-dichlorobenzylamino)-2-methylsulfanyl-pyrimidine-5-carboxylic amide(2.44 g) as a pale brown powder.
   APCI-MS(m/e):358/360[M+H]⁺. 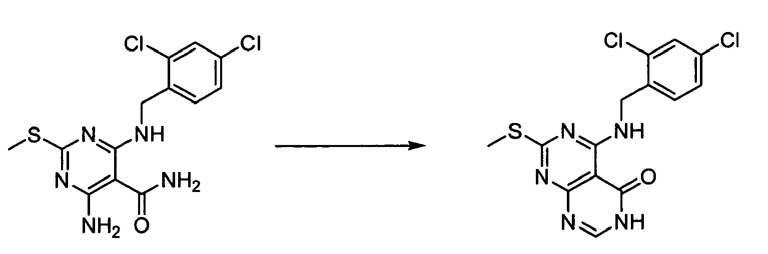
   4-Amino-6-(2,4-dichlorobenzylamino)-2-methylsulfanyl-pyrimidine-5-carboxylic amide(2.44 g), triethyl orthoformate(3.3 mL) and acetic anhydride(19.2 mL) were stirred at 120°C for 5 hours. After standing to cool, the reaction solution was concentrated under reduced pressure. Ethyl acetate/hexane was added to the residue, and a solid was triturated, washed with diisopropyl ether and dried to give 5-(2,4-dichlorobenzylamino)-7-methylsulfanyl-3H-pyrimido[4,5-d]pyrimidin-4-one(770 mg) as a pale yellow powder.
   APCI-MS(m/e):368/370[M+H]⁺. 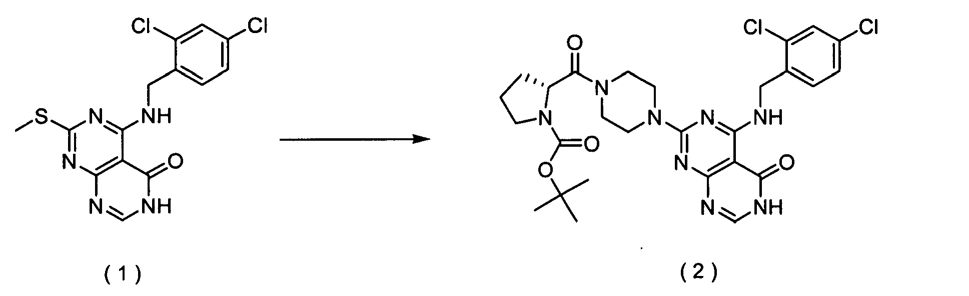
(6) Compound(2) was obtained by reacting and treating Compound(1) in the same manner as Example 16(2).
   APCI-MS(m/e):603/605[M+H]⁺. 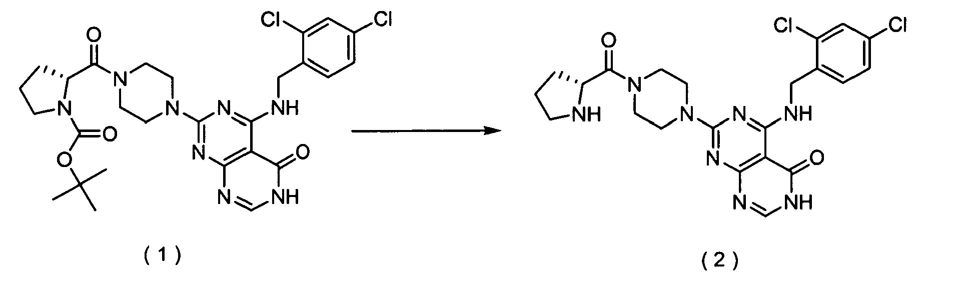
(7) Compound(2) was obtained by reacting and treating Compound(1) in the same manner as Example 1(5).
   APCI-MS(m/e):503/505[M+H]⁺.

### Example 18-20

The following compounds were obtained in the same manner as the aforementioned examples.

**[Table 3]**

| | | | | | |
|---|---|---|---|---|---|
| 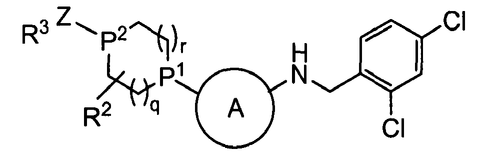 | | | | | |

| Example | R³ | Z | 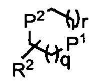 | Ring A | MS([M+H]⁺) |
|---|---|---|---|---|---|
| 18 | 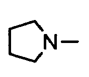 | -(CH₂)₂⁻ | 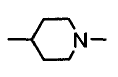 | 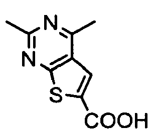 | 534/536, APCI |
| 19 | 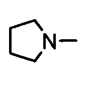 | -(CH₂)₂⁻ | 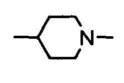 | 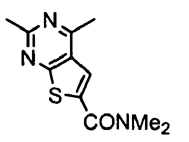 | 561/563, APCI |
| 20 | 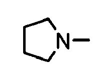 | -(CH₂)₂⁻ | 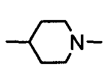 | 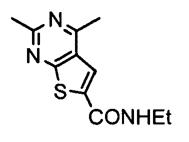 | 561/563, APCI |

### Example 21

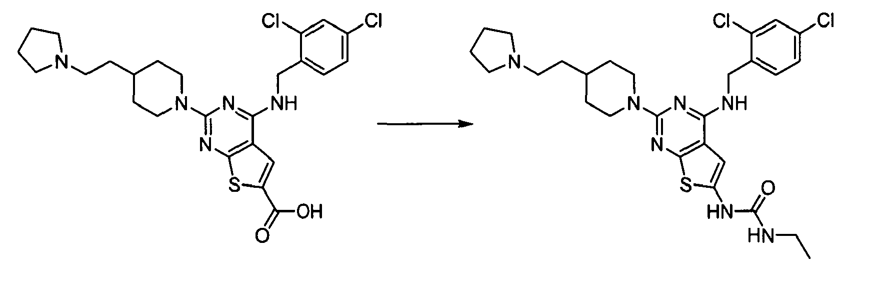

Diphenylphosphoryl azide(0.12 mL) and triethylamine(0.08 mL) were added to a solution of 4-(2,4-dichlorobenzylamino)-2-[4-(2-pyrrolidin-1-yl-ethyl)-piperidin-1-yl]-thieno[2,3-d]pyrimidine-6-carboxylic acid(150 mg) in dimethylacetamide(1 mL) and the mixture was stirred at 60°C for 3 hours. Further, an aqueous solution of ethylamine(70%, 0.05 m 1) was added thereto, the mixture was allowed to stand at room temperature overnight and chloroform was added to the reaction solution. The mixture was washed with a saturated aqueous sodium bicarbonate solution and brine, dried over magnesium sulfate and the solvent was distilled away. The residue was purified with thin layer silica gel chromatography(chloroform/methanol=19/1) to give 1-{4-(2,4-dichlorobenzylamino)-2-[4-(2-pyrrolidin-1-yl-ethyl)-piperidin-1-yl]-thieno[2,3-d]pyrimidine-6-yl}-3-ethyl-urea(2.3 mg) as an orange solid.
APCI-MS(m/e):576/578[M+H]⁺.

### Example 22

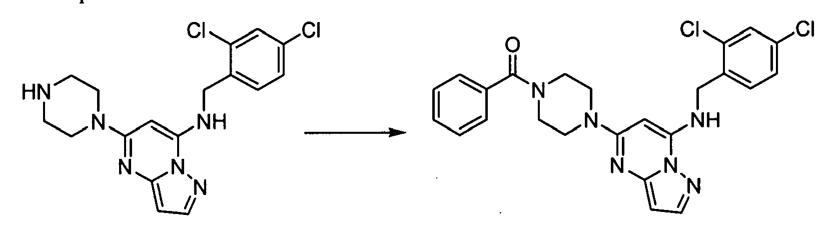

Benzoyl chloride(28 mg) was added to a solution of (2,4-dichlorobenzyl)-(5-piperazin-1-yl-pyrazolo[1,5-a]pyrimidin-7-yl)-amine(40 mg) in THF(1 mL) at room temperature and the mixture was stirred for 30 minutes. To the reaction solution was added ethyl acetate, the mixture was washed with a saturated aqueous sodium bicarbonate solution, dried over magnesium sulfate and the solvent was distilled away. The residue was recrystallized from ethyl acetate to give {4-[7-(2,4-dichlorobenzylamino)-pyrazolo[1,5-a]pyrimidin-5-yl]-piperazin-1-yl}-phenyl-methanone(36 mg) as colorless crystals.
APCI-MS(m/e):481/483[M+H]⁺.

### Example 23-40

The following compounds were obtained by the reaction and treatment in the same manner as the aforementioned examples.

**[Table 4]**

| | | | | | |
|---|---|---|---|---|---|
| 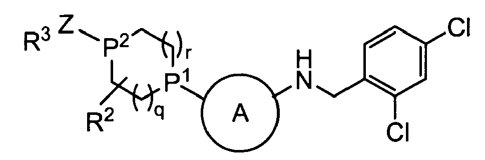 | | | | | |

| Example | R¹ | Z | 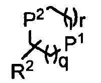 | Ring A | MS([M+H]⁺) |
|---|---|---|---|---|---|
| 23 | 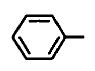 | -SO₂⁻ | 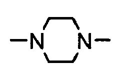 | 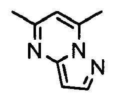 | 517/519, APCI |
| 24 | 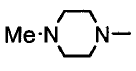 | -CO⁻ | 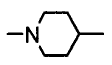 | 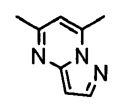 | 502/504, APCI |
| 25 | 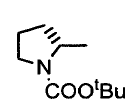 | -CO⁻ | 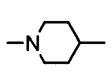 | 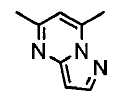 | 573/575, APCI |
| 26 | 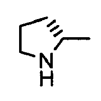 | -CO⁻ | 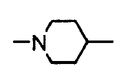 | 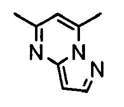 | 473/475, APCI |
| 27 | 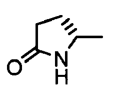 | -CO⁻ | 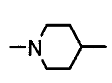 | 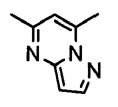 | 487/489, APCI |
| 28 | 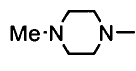 | -CO⁻ | 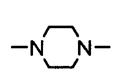 | 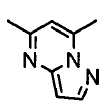 | 503/505, APCI |
| 29 | 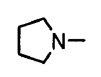 | -CO⁻ | 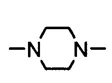 | 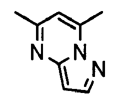 | 474/476, APCI |
| 30 | 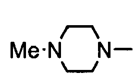 | -CO⁻ | 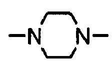 | 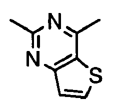 | 520/522, APCI |
| 31 | 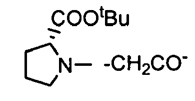 | | 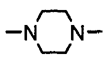 | 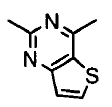 | 605/607, APCI |
| 32 | 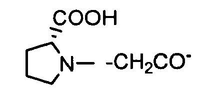 | | 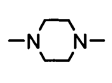 | 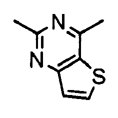 | 549/551, APCI |

**[Table 5]**

| | | | | | |
|---|---|---|---|---|---|
| 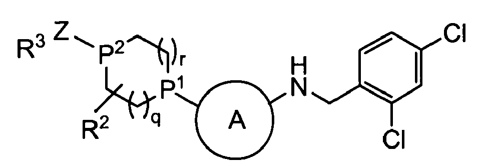 | | | | | |

| Example | R¹ | Z | 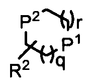 | Ring A | MS([M+H]⁺) |
|---|---|---|---|---|---|
| 33 | 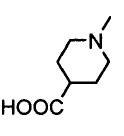 | -CH₂CO⁻ | 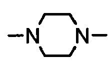 | 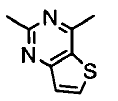 | 563/565, APCI |
| 34 | 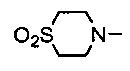 | -CH₂CO⁻ | 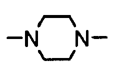 | 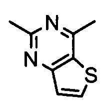 | 569/571, APCI |
| 35 | 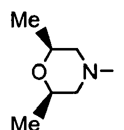 | -CH₂CO⁻ | 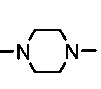 | 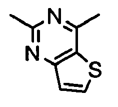 | 549/551, APCI |
| 36 | 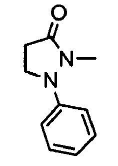 | -CH₂CO⁻ | 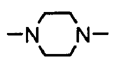 | 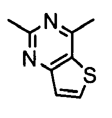 | 596/598, APCI |
| 37 | 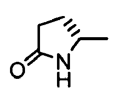 | -CO⁻ | 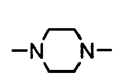 | 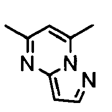 | 488/490, APCI |
| 38 | 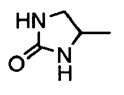 | -CO⁻ | 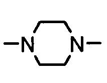 | 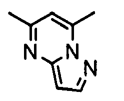 | 489/491, APCI |
| 39 | 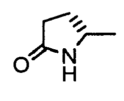 | -CO⁻ | 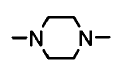 | 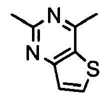 | 505/507, APCI |
| 40 | 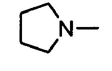 | -CH2⁻ | 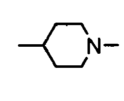 | 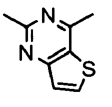 | 476/478, APCI |

### Example 41

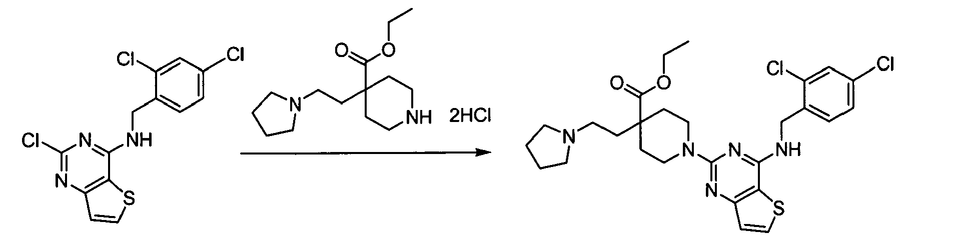

(1) (2-Chlorothieno[3,2-d]pyrimidin-4-yl)-(2,4-dichlorobenzyl)-amine(199 mg), 4-(2-pyrrolidin-1-yl-ethyl)-piperidine-4-carboxylic acid ethyl ester(770 mg) and diisopropylethylamine(1.01 mL) were dissolved in 1,4-dioxane(3 mL) and the mixture was stirred under microwave irradiation at 150°C for 3 hours. After standing to cool, a saturated aqueous sodium bicarbonate solution was added to the reaction solution and the mixture was extracted with ethyl acetate three times.
   The combined organic layer was dried over magnesium sulfate and the solvent was distilled away. The residue was purified with a silica gel column chromatography(chloroform/methanol = 100/0 to 85/15) to give 1-[4-(2,4-dichlorobenzylamino)-thieno[3,2-d]pyrimidin-2-yl]-4-(2-pyrrolidin-1-yl-ethyl)-piperidine-4-carboxylic acid ethyl ester(193 mg) as a pale yellow oily substance.
   APCI-MS(m/e):562/564[M+H]⁺. 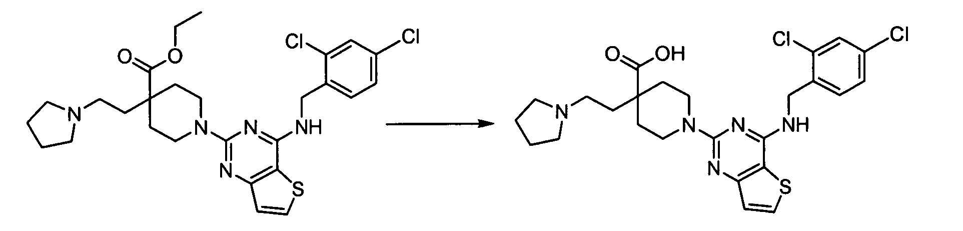
(2) A 2N sodium hydroxide aqueous solution(1.35 mL) was added to a solution of 1-[4-(2,4-dichlorobenzylamino)-thieno[3,2-d]pyrimidin-2-yl]-4-(2-pyrrolidin-1-yl-ethyl)-piperidine-4-carboxylic acid ethyl ester(152 mg) in ethanol(2.7 mL) and the mixture was refluxed under heating overnight. After standing to cool, 1N hydrochloric acid was added, the reaction solution was neutralized and the solvent was distilled away. Chloroform/methanol(4/1) mixture was added, an insoluble material was filtered and the filtrate was concentrated. The residue was purified with a silica gel column chromatography(chloroform/methanol=90/10 to 40/60), the precipitated solid was washed with methanol and dried to give 1-[4-(2,4-dichlorobenzylamino)-thieno[3,2-d]pyrimidin-2-yl]-4-(2-pyrrolidin-1-yl-ethyl)-piperidine-4-carboxylic acid(84 mg) as a pale yellow solid.
   APCI-MS(m/e):534/536[M+H]⁺.

### Example 42

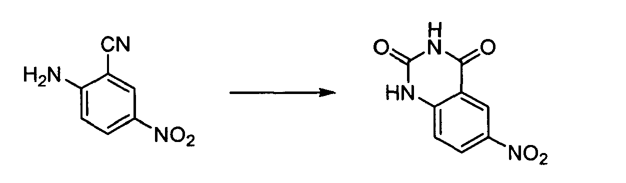

(1) DBU(32.7 g) was added to a solution of 2-amino-5-nitrobenzonitrile(11.69 g) in DMF(135 mL) at room temperature and the mixture was stirred under carbon dioxide atmosphere at room temperature overnight. The reaction solution was cooled to 0°C, and 1N hydrochloric acid(1350 mL) was added dropwise. The precipitated crystals were filtered, washed with diethyl ether and dried to give 6-nitro-1H-quinazolin-2,4-dione(14.13 g) as a yellow powder.
   APCI-MS(m/e):206[M-H]⁻. 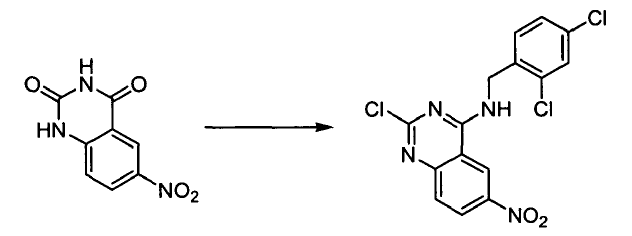
(2) Phosphorous oxychloride(41.45 g) was added to 6-nitro-1H-quinazolin-2,4-dione(3.0 g) and the mixture was stirred under reflux overnight. The reaction solution was concentrated, toluene was added to the residue and it was concentrated again. Chloroform was added to the residue, the precipitate was filtered, and water was added to the filtrate and it was separated. The organic layer was dried and the solvent was concentrated. Acetonitrile(50 mL) was added to the residue and the mixture was cooled to 0°C, a solution of 2,4-dichlorobenzylamine(1.06 g) in acetonitrile(20 mL) was added dropwise. After stirring at 0°C for 15 minutes, chloroform and water was added to the reaction solution. The organic layer was separated , dried and the solvent was concentrated. Diisopropyl ether was added to the residue, the precipitate was filtered and dried to give (2-chloro-6-nitro-quinazolin-4-yl)-(2,4-dichlorobenzyl)-amine(832 mg) as a yellow powder.
   APCI-MS(m/e):383/385[M+H]⁺. 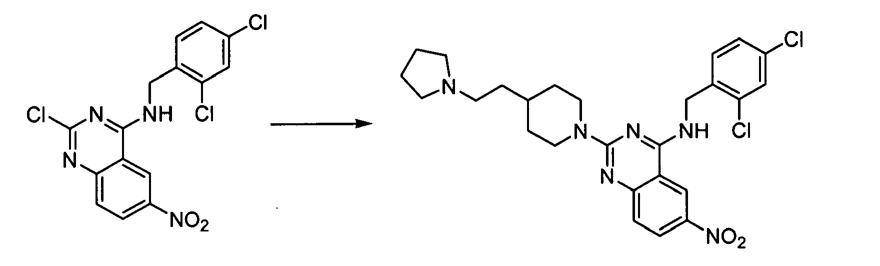
(3) Diisopropylethylamine(153 mg) was added to a suspension of (2-chloro-6-nitroquinazolin-4-yl)-(2,4-dichlorobenzyl)-amine(226.3 mg) and 4-(2-pyrrodinoethyl)piperidine(161 mg) in isopropyl alcohol(7 mL) and chloroform(1 mL), and the mixture was stirred under reflux for 2 hours. The reaction solution was concentrated and a saturated aqueous sodium bicarbonate solution and chloroform were added to the residue. The organic layer was separated, dried and the solvent was concentrated. Diisopropyl ether was added to the residue, the precipitate was filtered and dried to give (2,4-dichloro-{6-nitro-2-[4-(2-pyrrolidin-1-yl-ethyl)-piperidin-1-yl]-quinazolin-4-yl}-amine(238 mg) as a yellow powder.
   APCI-MS(m/e):529/531 [M+H]⁺. 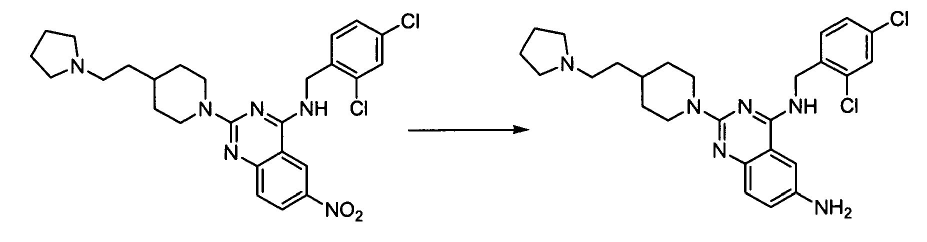
(4) Tin chloride dihydrate(3.48 g) was added to a solution of (2,4-dichloro-{6-nitro-2-[4-(2-pyrrolidin-1-yl-ethyl)-piperidin-1-yl]-quinazolin-4-yl}-amine(2.33 g) in ethanol(100 mL) and the mixture was stirred under reflux for 2 hours. The reaction solution was concentrated and a saturated aqueous sodium bicarbonate solution and chloroform were added to the residue. After an insoluble material was filtered, the organic layer was separated, dried and concentrated. The residue was purified with a NH silica gel column chromatography(ethyl acetate/methanol=99/1 to 95/5) to give {4-(2,4-dichlorobenzylamino)-2-[4-(2-pyrrolidin-1-ylethyl)piperidin-1-yl]quinazolin-6-yl}amine(1.1 g) as a red powder.
   APCI-MS(m/e):499/501[M+H]⁺. 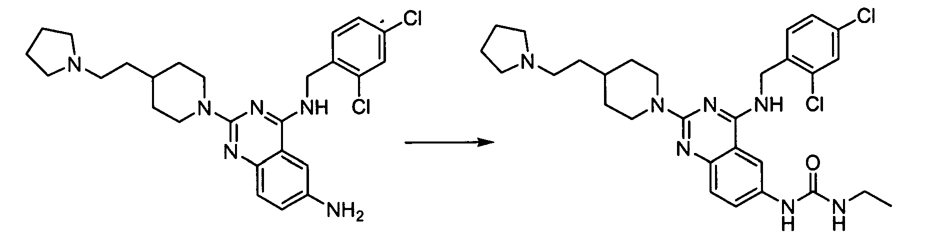
(5) Ethyl isocyanate(99.5 mg) was added to a solution of {4-(2,4-dichlorobenzylamino)-2-[4-(2-pyrrolidin-1-ylethyl)piperidin-1-yl]quinazolin-6-yl}amine(350 mg) in THF(30 mL) and the mixture was stirred at room temperature overnight. Ethyl isocyanate(48 mg) was further added thereto and the mixture was stirred at room temperature overnight. After the reaction solution was concentrated, the residue was purified with a NH silica gel column chromatography(chloroform/methanol=99/1) and then with a thin layer NH silica gel column chromatography(ethyl acetate/methanol=19/1). Diisopropyl ether was added to the residue, the precipitate was filtered and dried to give 1-{4-(2,4-dichlorobenzylamino)-2-[4-(2-pyrrolidin-1-ylethyl)-piperidin-1-yl]-quinazolin-6-yl}-3-ethyl urea(125 mg) as a yellow powder.
   APCI-MS(m/e):570/572[M+H]⁺.

### Example 43

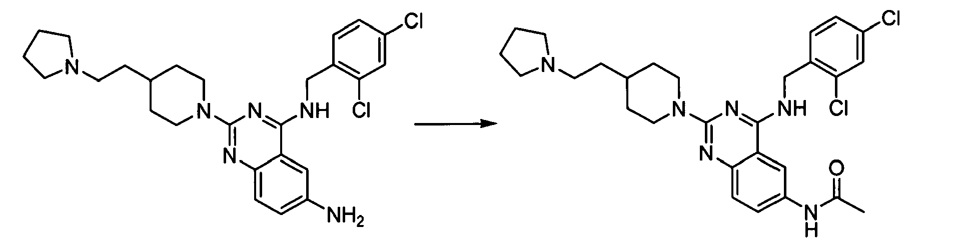

Acetyl chloride(14 mg) and triethylamine(18.2 mg) were added to a solution of {4-(2,4-dichlorobenzylamino)-2-[4-(2-pyrrolidin-1-ylethyl)piperidin-1-yl]quinazolin-6-yl}amine(30 mg) in methylene chloride(3 mL) and the mixture was stirred at room temperature for 2 days. A saturated aqueous sodium bicarbonate solution and chloroform were added to the reaction solution and the organic layer was separated, dried and the solvent was concentrated. After the residue was dissolved in methanol, activated charcoal was added and the mixture was filtered. The filtrate was purified with a thin layer NH silica gel column chromatography(chloroform/methanol=9/1) to give N-{4-(2,4-dichlorobenzylamino)-2-[4-(2-pyrrolidin-1-yl-ethyl)-piperidin-1-yl]-quinazolin-6-yl}-acetamide(15 mg) as an orange powder.
APCI-MS(m/e):541/543[M+H]⁺.

### Example 44-81

The following compounds were obtained by the reaction and treatment in the same manner as the aforementioned examples.

**[Table 6]**

| | | |
|---|---|---|
| 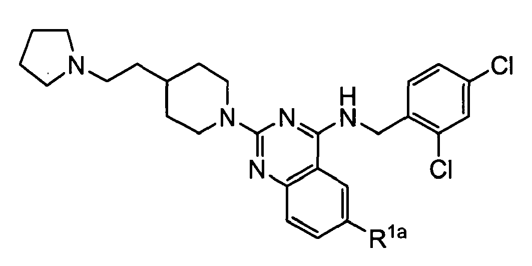 | | |

| Example | R^{1a} | MS([M+H]⁺) |
|---|---|---|
| 44 | 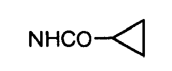 | 567/569 APCI |
| 45 | NHCOCH₂OMe | 571, ESI |
| 46 | NHCONEt₂ | 598, ESI |
| 47 | 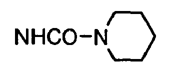 | 610, ESI |
| 48 | 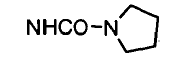 | 596, ESI |
| 49 | NHCOOMe | 557, ESI |
| 50 | 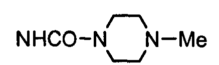 | 625, ESI |
| 51 | NHCOO(CH₂)₂OMe | 601, ESI |
| 52 | NHCOOEt | 571, ESI |
| 53 | 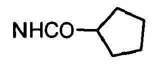 | 595, ESI |
| 54 | NHSO₂Me | 577, ESI |
| 55 | NHSO₂iPr | 605, ESI |
| 56 | NHCONHiPr | 584, ESI |

**[Table 7]**

| | | | | |
|---|---|---|---|---|
| 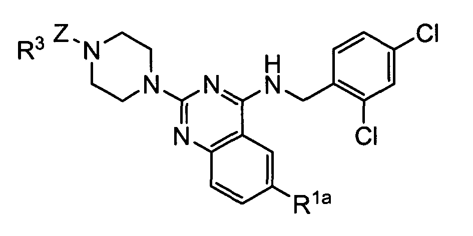 | | | | |

| Example | R³ | Z | R^{1a} | MS([M+H]⁺) |
|---|---|---|---|---|
| 57 | 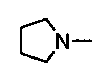 | -CH₂CO⁻ | -NO₂ | 544/546, APCI |
| 58 | 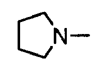 | -CH₂CO⁻ | -NH₂ | 514/516, APCI |
| 59 | 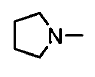 | -CH₂CO⁻ | -NHCONHEt | 585/587, APCI |
| 60 | 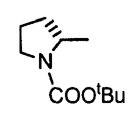 | -CO' | -NO₂ | 630/632, APCI |
| 61 | 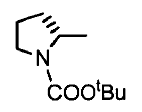 | -CO⁻ | -NH₂ | 600/602, APCI |
| 62 | 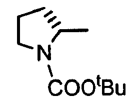 | -CO⁻ | -NHCONHEt | 671/673, APCI |
| 63 | 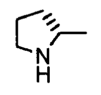 | -CO⁻ | -NHCONHEt | 571/573, APCI |
| 64 | 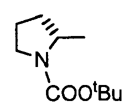 | -CO⁻ | -H | 585/587, APCI |
| 65 | 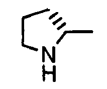 | -CO⁻ | -H | 485/487, APCI |

**[Table 8]**

| | | | | |
|---|---|---|---|---|
| 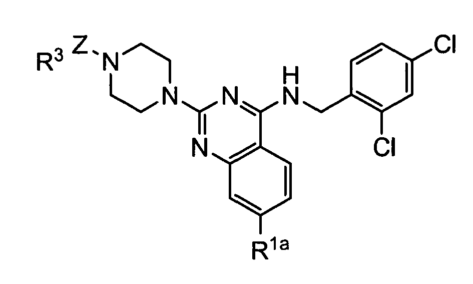 | | | | |

| Example | R³ | Z | R^{1a} | MS([M+H]⁺) |
|---|---|---|---|---|
| 66 | 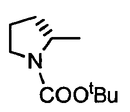 | -CO⁻ | -NO₂ | 630/632, APCI |
| 67 | 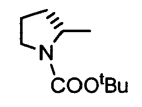 | -CO⁻ | -NHOH | 616/618, APCI |
| 68 | 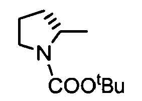 | -CO⁻ | -NH₂ | 600/602, APCI |
| 69 | 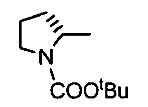 | -CO⁻ | -NHCONHEt | 671/673, APCI |
| 70 | 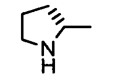 | -CO⁻ | -NHCONHEt | 571/573, APCI |

**[Table 9]**

| | | | | |
|---|---|---|---|---|
| 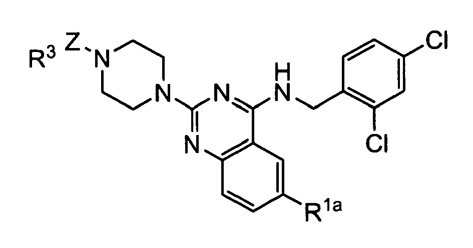 | | | | |

| Example | R³ | Z | R^{1a} | MS([M+H]⁺) |
|---|---|---|---|---|
| 71 | 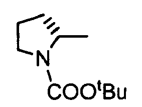 | -CO⁻ | -COOMe | 643/645, APCI |
| 72 | 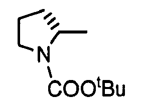 | -CO⁻ | -COOH | 629/631, APCI |
| 73 | 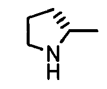 | -CO⁻ | -COOH | 529/531, APCI |
| 74 | 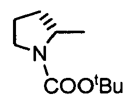 | -CO⁻ | -CONHEt | 656/658, APCI |
| 75 | 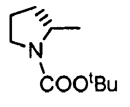 | -CO⁻ | -CONH₂ | 628/630, APCI |
| 76 | 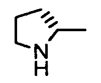 | -CO⁻ | -CONHEt | 556/558, APCI |
| 77 | 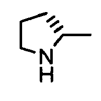 | -CO⁻ | -CONH₂ | 528/530, APCI |

**[Table 10]**

| | | | | |
|---|---|---|---|---|
| 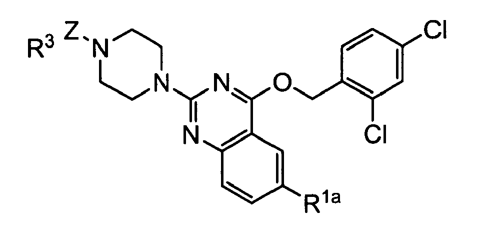 | | | | |

| Example | R³ | Z | R^{1a} | MS([M+H]⁺) |
|---|---|---|---|---|
| 78 | 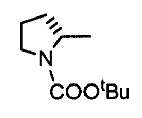 | -CO⁻ | -NO₂ | 631/633, APCI |
| 79 | 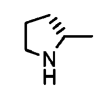 | -CO' | -NO₂ | 531/533, APCI |
| 80 | 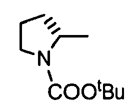 | -CO⁻ | -NHCONHEt | 672/674, APCI |
| 81 | 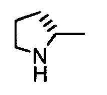 | -CO⁻ | -NHCONHEt | 572/574, APCI |

### Example 82

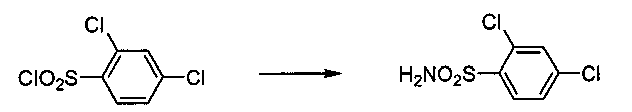

(1) A solution of 2,4-dichlorobenzenesulfonyl chloride(5.0 g) in THF(80 mL) was cooled to 0°C, 28% aqueous ammonia(40 mL) was added thereto and the mixture was stirred at room temperature for an hour. The reaction solution was concentrated and ethyl acetate and hydrochloric acid were added. The organic layer was separated, dried and concentrated. The residue was recrystallized from ethyl acetate and diisopropyl ether to give 2,4-dichlorobenzenesulfonamide(4.23 g) as colorless crystals.
   APCI-MS(m/e):224/226[M-H]⁻. 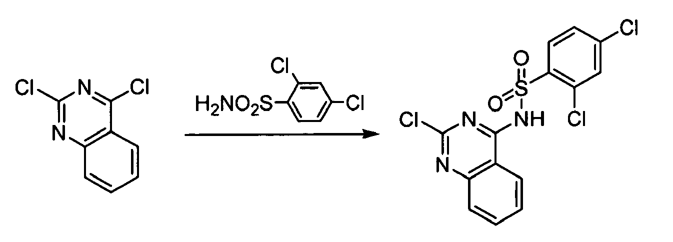
(2) Sodium hydride(160 mg) was added to a solution of 2,4-dichlorobenzenesulfonamide(904 mg) in DMSO(10 mL) and the mixture was stirred at room temperature for 30 minutes. The reaction solution was cooled to 0°C, 2,4-dichloroquinazoline(597.1 mg) was added and the mixture was stirred at room temperature for 3 hours. Ethyl acetate and phosphate buffer(pH 6) were added to the reaction solution and the organic layer was separated, dried and the solvent was concentrated. Diethyl ether was added to the residue, the precipitate was filtered and dried to give 2,4-dichloro-N-(2-chloro-quinazolin-4-yl)-benzenesulfonamide(164 mg) as a colorless powder.
   APCI-MS(m/e):388/390[M+H]⁺. 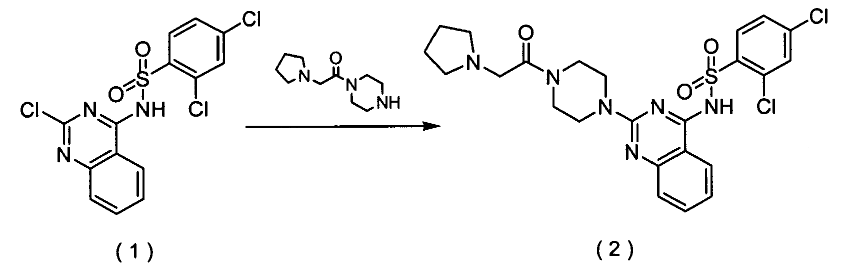
(3) Compound(2) was obtained by reacting and treating Compound(1) in the same manner as Example 42(3).
   APCI-MS(m/e):549/551 [M+H]⁺.

### Example 83

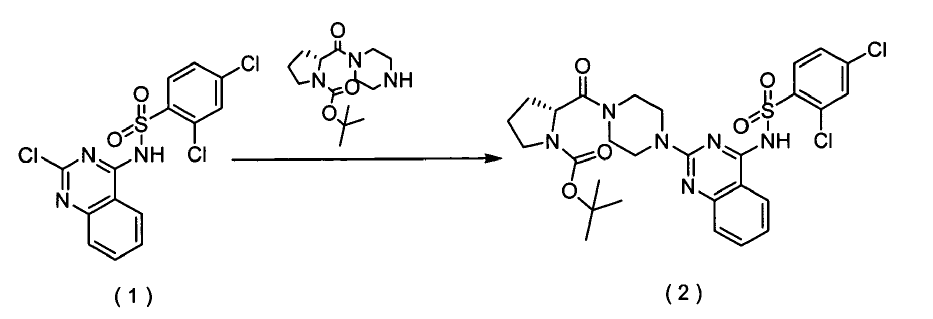

(1) Compound(2) was obtained by reacting and treating Compound(1) in the same manner as Example 1(4).
   APCI-MS(m/e):635/637[M+H]⁺. 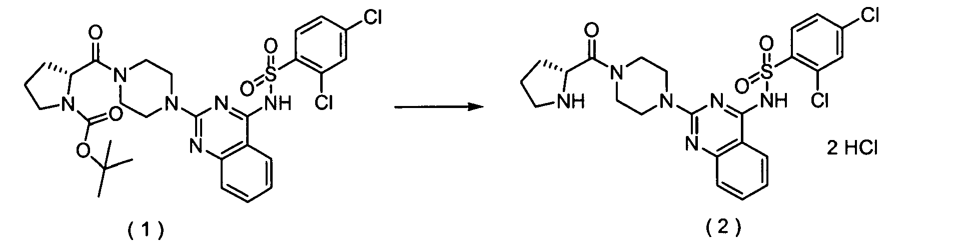
(2) Compound(2) was obtained by reacting and treating Compound(1) in the same manner as Example 1(5).
   APCI-MS(m/e):535/537[M+H]⁺.

### Example 84

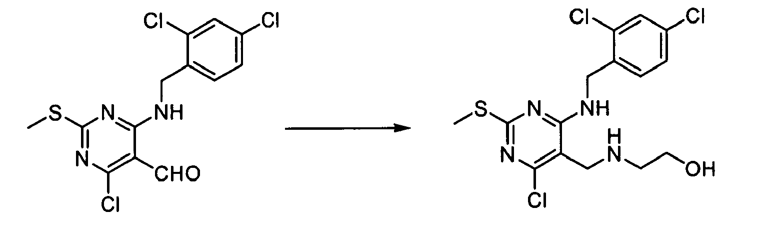

(1) 2-Aminoethanol(111 mg) was added to a solution of 4-chloro-6-(2,4-dichlorobenzylamino)-2-methylsulfanylpyrimidine-5-carboaldehyde(600 mg) in methylene chloride(30 mL) and the mixture was stirred at room temperature for 30 minutes. Sodium triacetoxyborohydride(1.05 g) and acetic acid(0.284 mL) were further added to the mixture and it was stirred at room temperature overnight. A saturated aqueous sodium bicarbonate solution and chloroform were added to the reaction solution and the organic layer was separated, dried and the solvent was concentrated. The residue was purified with a silica gel column chromatography(chloroform/methanol=100/0 to 19/1) to give 2-{[4-chloro-6-(2,4-dichlorobenzylamino)-2-methylsulfanylpyrimidin-5-ylmethyl]amino} ethanol(86.3 mg) as a colorless powder.
   APCI-MS(m/e):407/409[M+H]⁺. 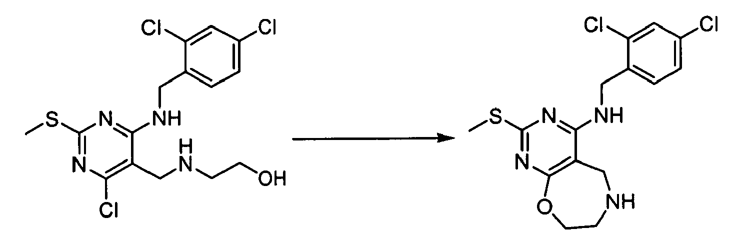
(2) Sodium hydride(60%, 10.2 mg) was added to a solution of 2-{[4-chloro-6-(2,4-dichlorobenzylamino)-2-methylsulfanylpyrimidin-5-ylmethyl]amino]ethanol(86.3 mg) in THF(6 mL) and the mixture was refluxed under heating with stirring for 3 hours. Water and chloroform were added to the reaction solution and the organic layer was separated, dried and concentrated. The residue was purified with a thin layer column chromatography(chloroform/methanol=9/1) to give (2,4-dichlorobenzyl)-2-(methylsulfanyl-5,6,7,8-tetrahydro-9-oxa-1,3,6-triazabenzocyclohepten-4-yl)amine(28.5 mg) as a colorless powder.
   APCI-MS(m/e):371/373[M+H]⁺. 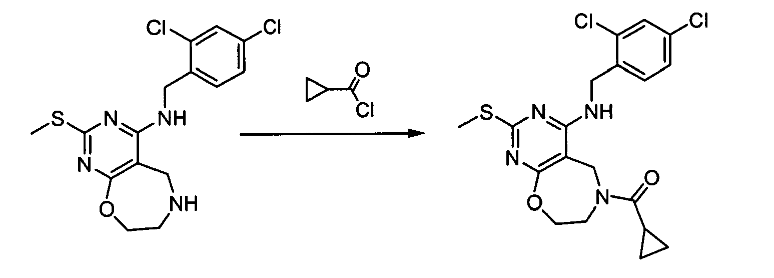
(3) Cyclopropanecarbonyl chloride(12 mg) and triethylamine(15.5 mg) were added to a suspension of (2,4-dichlorobenzyl)-2-(methylsulfanyl-5,6,7,8-tetrahydro-9-oxa-1,3,6-triazabenzocyclohepten-4-yl)amine(28.5 mg) in THF(5 mL) and the mixture was stirred at room temperature for an hour. A saturated aqueous sodium bicarbonate solution and chloroform were added to the reaction solution and the organic layer was separated, dried and concentrated. The residue was purified with a thin layer column chromatography(ethyl acetate) to give cyclopropyl-[4-(2,4-dichlorobenzylamino)-2-methylsulfanyl-7,8-dihydro-5H-9-oxa-1,3,6-triazabenzocyclohepten-6-yl]methanone(21.9 mg) as a colorless powder.
   APCI-MS(m/e):439/441 [M+H]⁺. 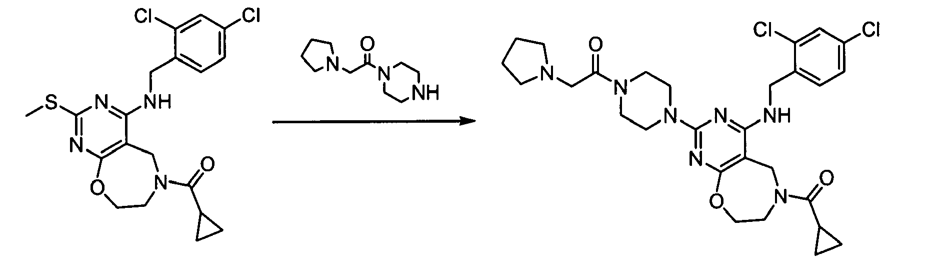
(4) A solution of cyclopropyl-[4-(2,4-dichlorobenzylamino)-2-methylsulfanyl-7,8-dihydro-5H-9-oxa-1,3,6-triazabenzocyclohepten-6-yl]methanone(21.9 mg) in chloroform(3 mL) was cooled to 0°C, m-chloroperbenzoic acid(75%, 20 mg) was added thereto and the mixture was stirred at room temperature for 2 hours. After the reaction solution was concentrated, 1-piperazin-1-yl-2-pyrrolidin-1-ylethanone(259 mg) was added to the residue and the mixture was radiated at 150°C for an hour using a microwave reaction apparatus. The reaction solution was cooled to room temperature, and water and chloroform were added thereto. The organic layer was separated, dried and the solvent was concentrated. The residue was purified with a thin layer silica gel column chromatography(chloroform/methanol=9/1) and further with a NH thin layer silica gel column chromatography(ethyl acetate/methanol=10/1) to give 1-{4-[6-cyclopropanecarbonyl-4-(2,4-dichlorobenzylamino)-5,6,7,8-tetraunhydro-9-oxa-1,3,6-triazabenzocycloheptan-2-yl]-piperazin-1-yl}-2-pyrrolidin-1-yl-ethanone(3 mg) as a yellow powder.
   APCI-MS(m/e):588/590[M+H]⁺.

### Example 85-177

The following compounds were obtained by the reaction and treatment in the same manner as the aforementioned examples.

**[Table 11]**

| | | | |
|---|---|---|---|
| 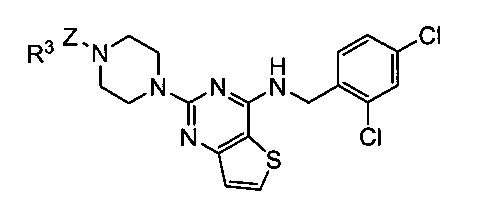 | | | |

| Example | R³ | Z | MS([M+H]⁺) |
|---|---|---|---|
| 85 | 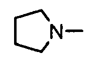 | -CH₂CO⁻ | 505/507, APCI |
| 86 | 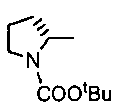 | -CO⁻ | 591/593, APCI |
| 87 | 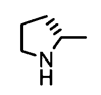 | -CO⁻ | 491/493, APCI |
| 88 | 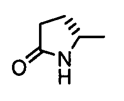 | -CO⁻ | 505/507, APCI |
| 89 | MeSO₂NH⁻ | -CH₂CO⁻ | 529, ESI |
| 90 | 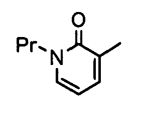 | -CO⁻ | 557, ESI |
| 91 | 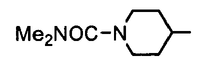 | -CO⁻ | 576, ESI |
| 92 | 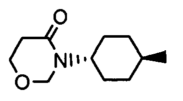 | -CO⁻ | 603, ESI |
| 93 | 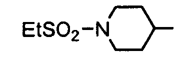 | -CO⁻ | 597, ESI |
| 94 | 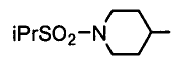 | -CO⁻ | 611, ESI |
| 95 | 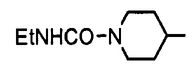 | -CO⁻ | 576, ESI |
| 96 | 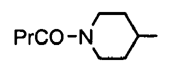 | -CO⁻ | 575, ESI |
| 97 | 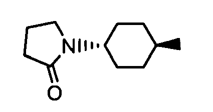 | -CO⁻ | 587, ESI |

**[Table 12]**

| | | | |
|---|---|---|---|
| 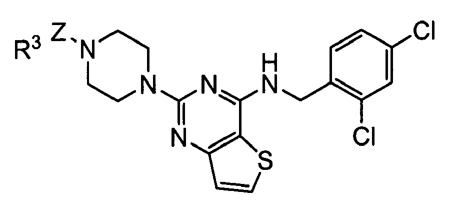 | | | |

| Example | R³ | Z | MS([M+H]⁺) |
|---|---|---|---|
| 98 | 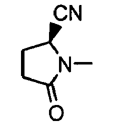 | -CH₂CO⁻ | 544, ESI |
| 99 | 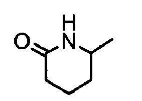 | -CO⁻ | 519, ESI |
| 100 | 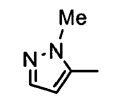 | -CO⁻ | 502, ESI |
| 101 | 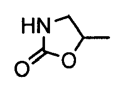 | -CH₂CO⁻ | 521, ESI |
| 102 | 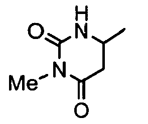 | -CO⁻ | 548, ESI |
| 103 | 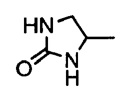 | -CH₂CO⁻ | 520, ESI |
| 104 | 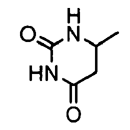 | -CO⁻ | 534, ESI |
| 105 | 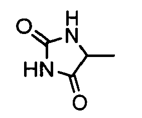 | -CH₂CO⁻ | 534, ESI |
| 106 | 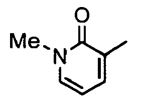 | -CO⁻ | 529, ESI |
| 107 | 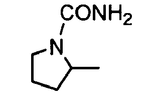 | -CH₂CO⁻ | 534, ESI |

**[Table 13]**

| | | | |
|---|---|---|---|
| 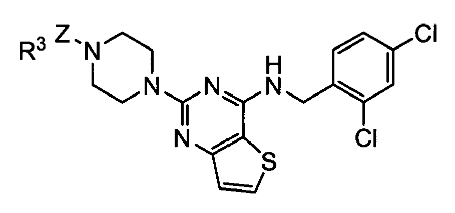 | | | |

| Example | R³ | Z | MS([M+H]⁺) |
|---|---|---|---|
| 108 | 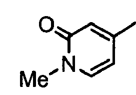 | -CO⁻ | 529, ESI |
| 109 | 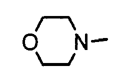 | -CH₂CO⁻ | 521, ESI |
| 110 | 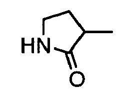 | -CO⁻ | 505, ESI |
| 111 | 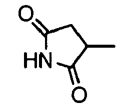 | -CH₂CO⁻ | 533, ESI |
| 112 | 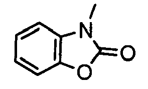 | -(CH₂)₂CO⁻ | 583, ESI |
| 113 | 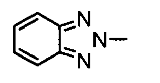 | -CH₂CO⁻ | 553, ESI |
| 114 | 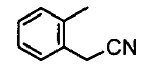 | -CO⁻ | 537, ESI |
| 115 | 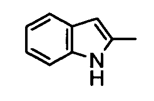 | -CO⁻ | 537, ESI |
| 116 | 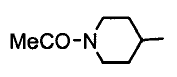 | -CO⁻ | 547, ESI |
| 117 | 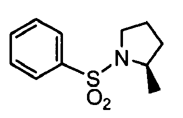 | -CO⁻ | 631, ESI |
| 118 | 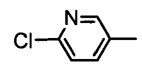 | -CO⁻ | 533, ESI |
| 119 | 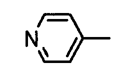 | -CO⁻ | 499, ESI |

**[Table 14]**

| | | | |
|---|---|---|---|
| 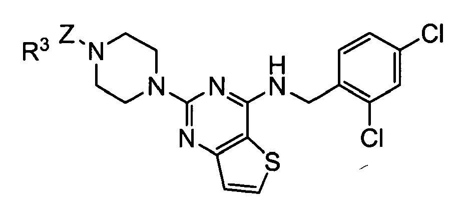 | | | |

| Example | R³ | Z | MS([M+H]⁺) |
|---|---|---|---|
| 120 | 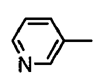 | -CH₂CO⁻ | 513, ESI |
| 121 | 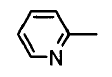 | -CH₂CO⁻ | 513, ESI |
| 122 | MeCONH⁻ | -CH₂CO⁻ | 493, ESI |
| 123 | 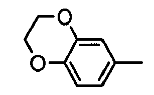 | -CO' | 556, ESI |
| 124 | 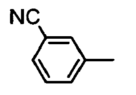 | -CO⁻ | 523, ESI |
| 125 | 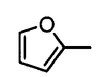 | -CO' | 488, ESI |
| 126 | 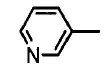 | -CO' | 499, ESI |
| 127 | 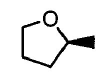 | -CO⁻ | 492, ESI |
| 128 | 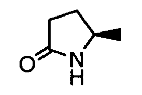 | -CO⁻ | 505, ESI |
| 129 | 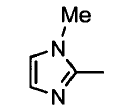 | -CO⁻ | 502, ESI |
| 130 | 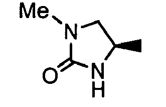 | -CO⁻ | 520, ESI |
| 131 | 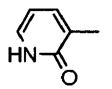 | -CH₂CO⁻ | 529, ESI |
| 132 | 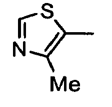 | -CO⁻ | 519, ESI |

**[Table 15]**

| | | | |
|---|---|---|---|
| 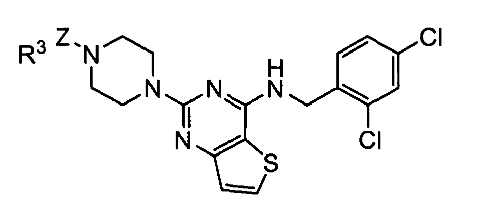 | | | |

| Example | R³ | Z | MS([M+H]⁺) |
|---|---|---|---|
| 133 | 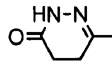 | -CO⁻ | 518, ESI |
| 134 | 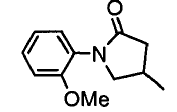 | -CO⁻ | 611, ESI |
| 135 | 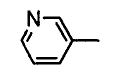 | -(CH₂)₂CO⁻ | 527, ESI |
| 136 | 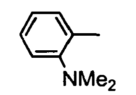 | -CO⁻ | 541, ESI |
| 137 | 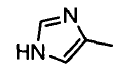 | -CH₂CO⁻ | 502, ESI |
| 138 | 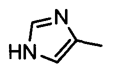 | -CO⁻ | 488, ESI |
| 139 | 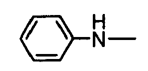 | -CH₂CO⁻ | 527, ESI |
| 140 | 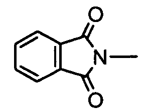 | -CH₂CO⁻ | 581, ESI |
| 141 | 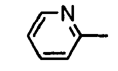 | -CO⁻ | 499, ESI |
| 142 | 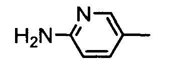 | -CO⁻ | 514, ESI |
| 143 | 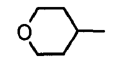 | -CH₂CO⁻ | 520, ESI |
| 144 | 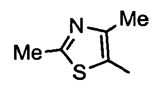 | -CO⁻ | 533, ESI |

**[Table 16]**

| | | | |
|---|---|---|---|
| 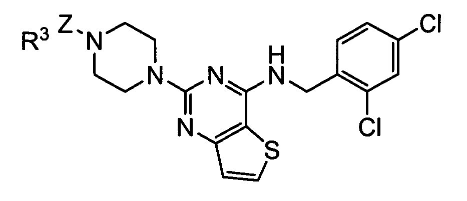 | | | |

| Example | R³ | Z | MS([M+H]⁺) |
|---|---|---|---|
| 145 | 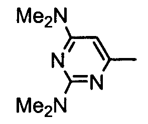 | -CO' | 586, ESI |
| 146 | 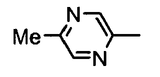 | -CO' | 514, ESI |
| 147 | 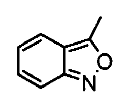 | -CO' | 539, ESI |
| 148 | 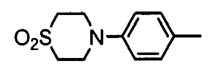 | -CO' | 631, ESI |
| 149 | 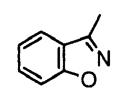 | -CH₂CO⁻ | 553, ESI |
| 150 | 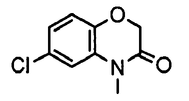 | -CH₂CO⁻ | 617, ESI |
| 151 | 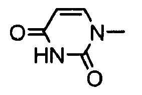 | -CH₂CO⁻ | 546, ESI |
| 152 | 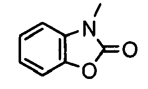 | -CH₂CO⁻ | 569, ESI |
| 153 | 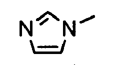 | -(CH₂)₂CO⁻ | 516, ESI |
| 154 | 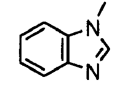 | -CH₂CO⁻ | 552, ESI |
| 155 | 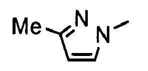 | -(CH₂)₂CO⁻ | 530, ESI |
| 156 | 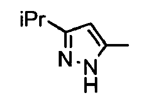 | -CO' | 530, ESI |

**[Table 17]**

| | | | |
|---|---|---|---|
| 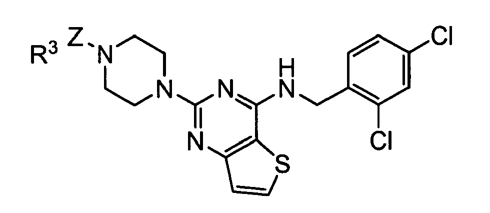 | | | |

| Example | R³ | Z | MS([M+H]⁺) |
|---|---|---|---|
| 157 | 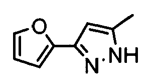 | -CO' | 554, ESI |
| 158 | 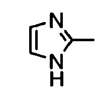 | -CO' | 488, ESI |
| 159 | 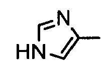 | -(CH₂)₂CO⁻ | 516, ESI |
| 160 | 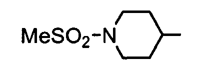 | -CO' | 583, ESI |
| 161 | 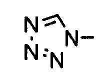 | -CH₂CO⁻ | 504, ESI |
| 162 | HO⁻ | -(CH₂)₂CO⁻ | 466, ESI |
| 163 | 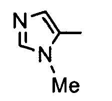 | -(CH₂)₂CO⁻ | 530, ESI |
| 164 | 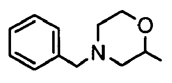 | -CO⁻ | 597, ESI |

**[Table 18]**

| | | | |
|---|---|---|---|
| 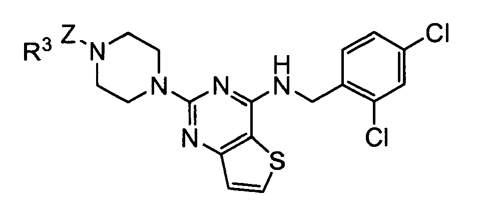 | | | |

| Example | R³ | Z | MS([M+H]⁺) |
|---|---|---|---|
| 165 | 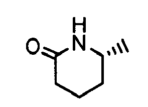 | -CO⁻ | 519, ESI |
| 166 | 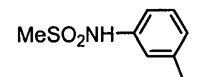 | -CO⁻ | 591, ESI |
| 167 | 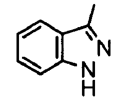 | -CO' | 538, ESI |
| 168 | 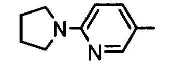 | -CO⁻ | 568, ESI |
| 169 | MeO⁻ | -(CH₂)₂CO⁻ | 480, ESI |
| 170 | 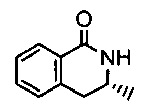 | -CO' | 567, ESI |
| 171 | 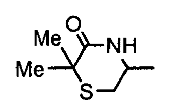 | -CO' | 565, ESI |
| 172 | 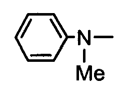 | -CH₂CO⁻ | 541/543, APCI |

**[Table 19]**

| | | | | | |
|---|---|---|---|---|---|
| 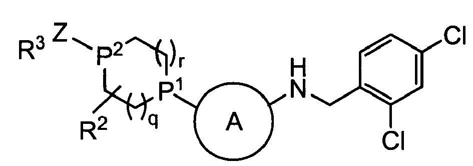 | | | | | |

| Example | R³ | Z | 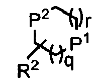 | Ring A | MS([M+H]⁺) |
|---|---|---|---|---|---|
| 173 | 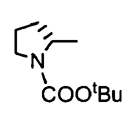 | -CO⁻ | 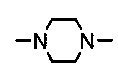 | 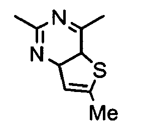 | 605/607, APCI |
| 174 | 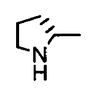 | -CO⁻ | 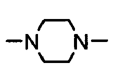 | 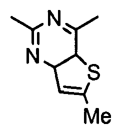 | 505/507, APCI |
| 175 | 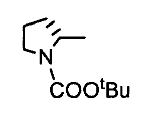 | -CO⁻ | 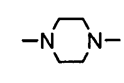 | 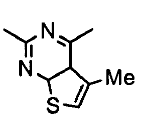 | 605/607, APCI |
| 176 | 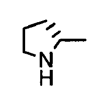 | -CO⁻ | 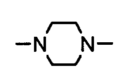 | 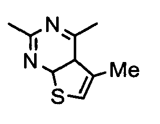 | 505/507, APCI |
| 177 | 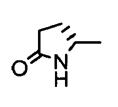 | -CO⁻ | 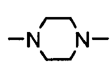 | 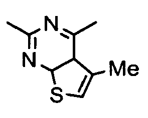 | 519/521, APCI |

### Example 178

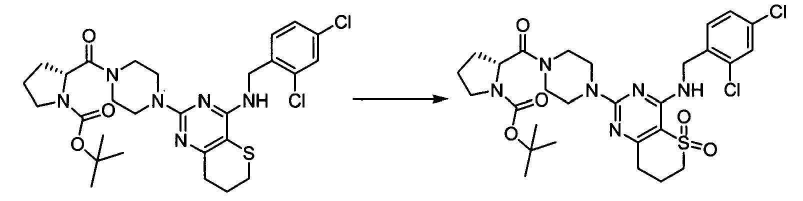

m-Chloroperbenzoic acid(75%, 74.1 mg) was added to a solution of (R)-2-{4-[4-(2,4-dichlorobenzylamino)-7,8-dihydro-6H-thiopyrano[3,2-d]pyrimidin-2-yl]piperazin-1-carbonyl}pyrrolidine-1-carboxylic acid tert-butyl ester(110.7 mg) in chloroform(5 mL) and the mixture was stirred at room temperature 1.5 hours. A saturated aqueous sodium thiosulfate solution and a saturated aqueous sodium bicarbonate solution were added to the reaction solution, the mixture was stirred for 10 minutes, and the organic layer was separated, dried and concentrated. The residue was purified with a thin layer silica gel column chromatography(ethyl acetate) to give 2-{4-[4-(2,4-dichlorobenzylamino)-5,5-dioxo-5,6,7,8-tetrahydro-λ-6-thienopyrimidine[3,2-d]pyrimidin-2-yl]-piperidin-1-carbonyl}pyrrolidine-1-carboxylic acid tert-butyl ester(59.4 mg) as a colorless powder.
APCI-MS(m/e):639/641[M+H]⁺.

### Examples 179-202

The following compounds were obtained by the reaction and treatment in the same manner as the aforementioned examples.

**[Table 20]**

| | | | | |
|---|---|---|---|---|
| 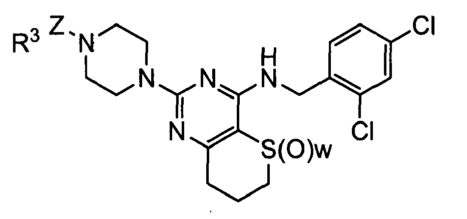 | | | | |

| Example | R³ | Z | w | MS([M+H]⁺) |
|---|---|---|---|---|
| 179 | 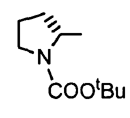 | -CO' | 0 | 607/609, APCI |
| 180 | 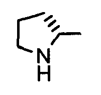 | -CO' | 0 | 507/509, APCI |
| 181 | 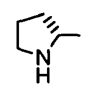 | -CO' | 2 | 539/541, APCI |

**[Table 21]**

| | | | | | |
|---|---|---|---|---|---|
| 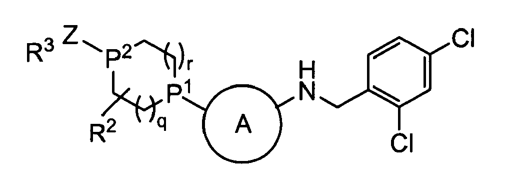 | | | | | |

| Example | R³ | Z | 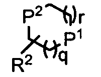 | Ring A | MS([M+H]⁺) |
|---|---|---|---|---|---|
| 182 | 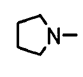 | -CO⁻ | 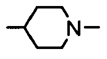 | 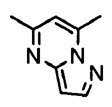 | 473/475, APCI |
| 183 | 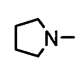 | -CH₂CO⁻ | 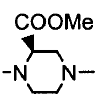 | 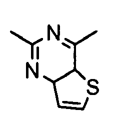 | 563/565, APCI |
| 184 | 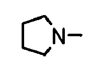 | -CH₂CO⁻ | 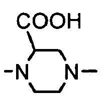 | 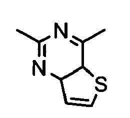 | 549/551, APCI |
| 185 | 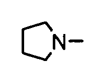 | -CH₂CO⁻ | 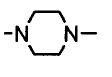 | 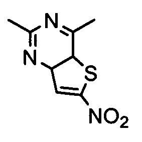 | 550/552, APCI |
| 186 | 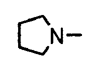 | -CH₂CO⁻ | 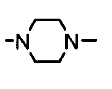 | 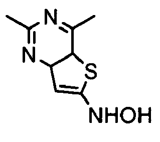 i | 536/538, APCI |
| 187 | 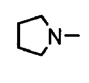 | -CH₂CO⁻ | 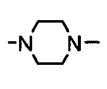 | 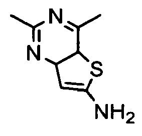 | 520/522, APCI |
| 188 | 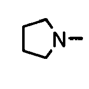 | -CH₂CO⁻ | 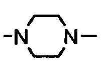 | 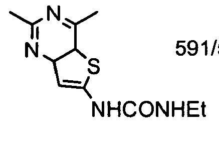 | 591/593, APCI |

**[Table 22]**

| | | | | | |
|---|---|---|---|---|---|
| 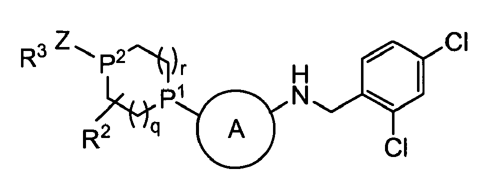 | | | | | |

| Example | R³ | Z | 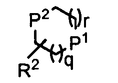 | Ring A | MS([M+H]⁺) |
|---|---|---|---|---|---|
| 189 | 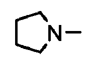 | -(CH₂)₂⁻ | 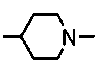 | 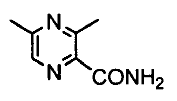 | 477/479, APCI |
| 190 | 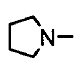 | -(CH₂)₂⁻ | 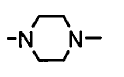 | 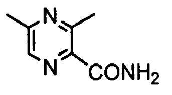 | 478/480, APCI |
| 191 | 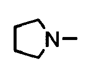 | -(CH₂)₂⁻ | 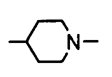 | 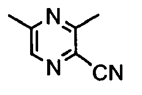 | 459/461, APCI |
| 192 | 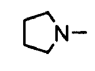 | -(CH₂)₂⁻ | 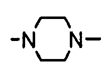 | 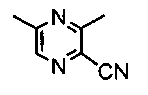 | 460/462, APCI |
| 193 | 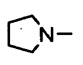 | -(CH₂)₂⁻ | 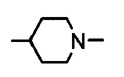 | 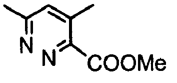 | 492/494, APCI |
| 194 | 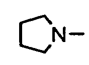 | -(CH₂)₂⁻ | 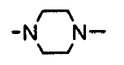 | 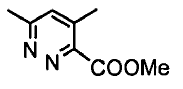 | 493/495, APCI |
| 195 | 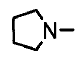 | -(CH₂)₂⁻ | 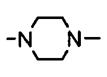 | 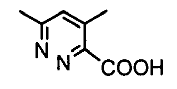 | 479/481, APCI |
| 196 | 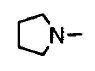 | -(CH₂)₂⁻ | 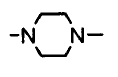 | 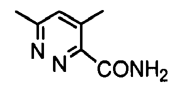 | 478/480, APCI |
| 197 | 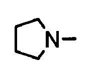 | -CH₂CO⁻ | 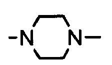 | 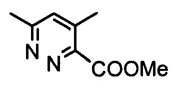 | 507/509, APCI |
| 198 | 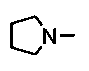 | -CH₂CO⁻ | 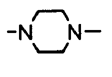 | 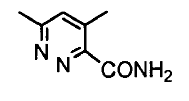 | 492/494, APCI |

**[Table 23]**

| | | | | | |
|---|---|---|---|---|---|
| 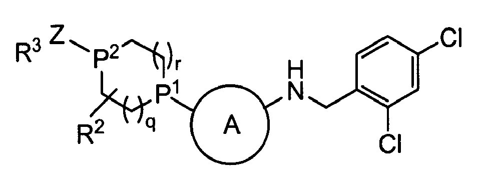 | | | | | |

| Example | R³ | Z | 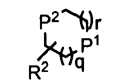 | Ring A | MS([M+H]⁺) |
|---|---|---|---|---|---|
| 199 | 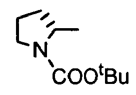 | -CO⁻ | 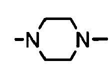 | 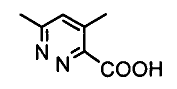 | 579/581, ESI |
| 200 | 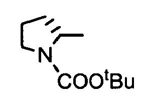 | -CO⁻ | 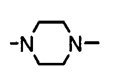 | 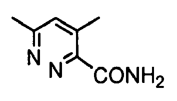 | 578/580, APCI |
| 201 | 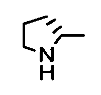 | -CO⁻ | 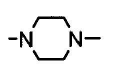 | 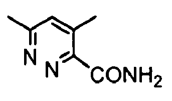 | 478/480, APCI |
| 202 | 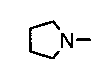 | -(CH₂)₂⁻ | 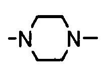 | 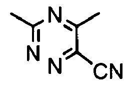 | 461/463, APCI |

### Example 203

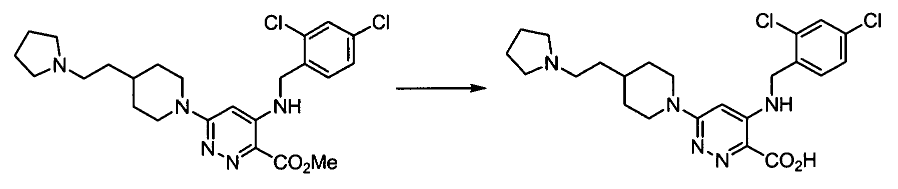

(1) A 2N sodium hydroxide aqueous solution(5 mL) was added to a solution of 4-(2,4-dichlorobenzylamino)-6-[4-(2-pyrrolidin-1-ylethyl)piperidin-1-yl]pyridazine-3-carboxylic acid methyl ester(566.8 mg) in dioxane(5 mL) and the mixture was stirred at room temperature for an hour. The reaction solution was neutralized by adding 2N hydrochloric acid, and chloroform and phosphate buffer were added thereto. After the organic layer was separated, dried and concentrated, THF and diethyl ether were added to the residue. The precipitate was filtered and dried to give 4-(2,4-dichlorobenzylamino)-6-[4-(2-pyrrolidin-1-ylethyl)piperidin-1-yl]pyridazine-3-carboxylic acid(500 mg) as a colorless powder.
   ESI-MS(m/e):478/480[M+H]⁺. 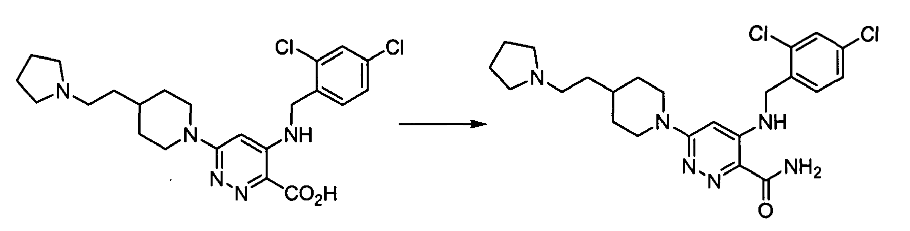
(2) N,N'-Carbonyldiimidazole(71.3 mg) was added to a suspension of 4-(2,4-dichlorobenzylamino)-6-[4-(2-pyrrolidin-1-ylethyl)piperidin-1-yl]pyridazine-3-carboxylic acid(140 mg) in DMF(3 mL) and DMSO(3 mL) and the mixture was stirred at room temperature for 3 days. Ammonia water(28%, 0.2 mL) was further added to the reaction solution and the mixture was stirred at room temperature overnight. Water and ethyl acetate were added to the reaction mixture and the organic layer was separated, washed with water, dried and concentrated. The residue was purified with a thin layer NH silica gel column chromatography(chloroform/methanol=50/1). Ethyl acetate and diisopropyl ether were added to the residue and the precipitate was filtered and dried to give 4-(2,4-dichlorobenzylamino)-6-[4-(2-pyrrolidin-1-ylethyl)piperidin-1-yl]pyridazine-3-carboxylic amide(20 mg) as a pale yellow powder.
   APCI-MS(m/e):477/479[M+H]⁺.

### Example 204

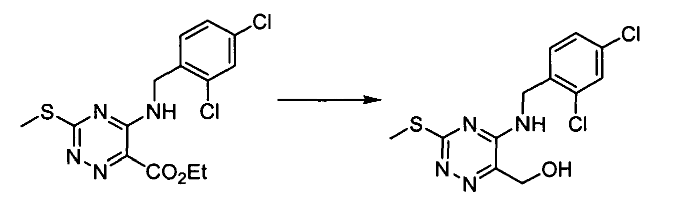

(1) A suspension of lithium aluminum hydride(508 mg) in THF(40 mL) was cooled to 0°C, a solution of 5-(2,4-dichlorobenzylamino)-3-methylsulfanyl-[1,2,4]triazin-6-carboxylic acid ethyl ester(2.5 g) in THF(17 mL) was added dropwise thereto and the mixture was stirred at room temperature for 30 minutes. After the reaction solution was cooled to 0°C, a saturated aqueous ammonium sulfate solution(1.6 mL) was added dropwise and the mixture was stirred at 0°C for 10 minutes. Further THF(20 mL) was added and a saturated aqueous ammonium sulfate solution(0.5 mL) was added, and the mixture was stirred at room temperature for 30 minutes. The reaction solution was filtered and the filtrate was concentrated. The residue was purified with a silica gel column chromatography(chloroform/methanol=90/1), ethyl acetate was added to the residue and the precipitate was filtered and dried to give 5-(2,4-dichlorobenzylamino)-3-methylsulfanyl-[1,2,4]triazin-6-yl]-methanol(500 mg) as a colorless powder.
   APCI-MS(m/e):331/333[M+H]⁺. 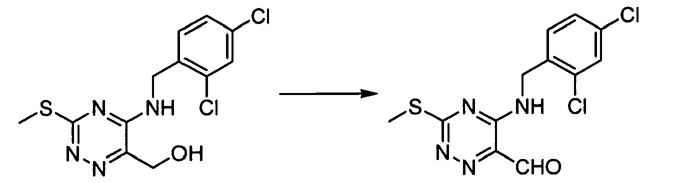
(2) Manganese dioxide(85%, 8.18 g) was added to a solution of 5-(2,4-dichlorobenzylamino)-3-methylsulfanyl-[1,2,4]triazin-6-yl]-methanol(1.32 g) in methylene chloride(50 mL) and THF(50 mL) and the mixture was stirred at room temperature for 3 days. The reaction solution was filtered and the filtrate was concentrated. The residue was purified with a silica gel column chromatography(hexane/ethyl acetate=95/5 to 75/25) to give 5-(2,4-dichlorobenzylamino)-3-methylsulfanyl-[1,2,4]triazine-6-carboaldehyde(520 mg) as a pale yellow powder.
   APCI-MS(m/e):329/331[M+H]⁺. 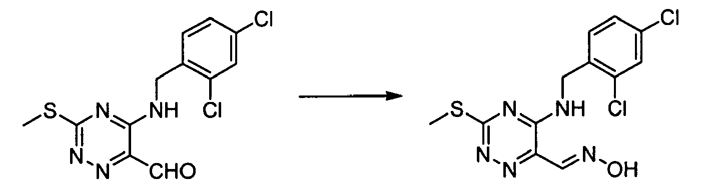
(3) Hydroxylammonium chloride(164.7 mg) and sodium acetate(324 mg) were added to a solution of 5-(2,4-dichlorobenzylamino)-3-methylsulfanyl-[1,2,4]triazine-6-carboaldehyde(520 mg) in methanol(30 mL) and the mixture was stirred at room temperature overnight. After the reaction solution was concentrated, water and chloroform were added to the residue. The aqueous layer was separated, extracted with chloroform twice and the combined organic layer was dried and concentrated. Diethyl ether was added to the residue and the precipitate was filtered and dried to give 5-(2,4-dichlorobenzylamino)-3-methylsulfanyl-[1,2,4]triazine-6-carboaldehyde oxime(445 mg) as a colorless powder.
   APCI-MS(m/e):344/346[M+H]⁺. 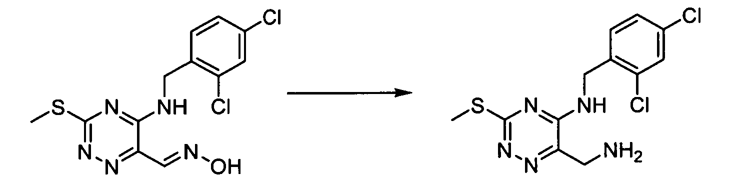
(4) Lithium aluminum hydride(36.4 mg) was added to a solution of 5-(2,4-dichlorobenzylamino)-3-methylsulfanyl-[1,2,4]triazine-6-carboaldehyde oxime(276 mg) in THF(10 mL) cooled to 0°C and the mixture was stirred at room temperature for an hour. The reaction solution was cooled to 0°C, lithium aluminum hydride(36.4 mg) was added and the mixture was stirred at room temperature overnight. After the reaction solution was cooled to 0°C and a saturated aqueous ammonium sulfate solution(0.56 mL) was added thereto, the reaction solution was filtered and the filtrate was concentrated. The residue was purified with a thin layer silica gel column chromatography(chloroform/methanol=80/20) to give 6-aminomethyl-3-methylsulfanyl-[1,2,4]triazin-5-yl)-(2,4-dichlorobenzyl)amine(42.6 mg) as red viscous oily substance.
   APCI-MS(m/e):330/332[M+H]⁺. 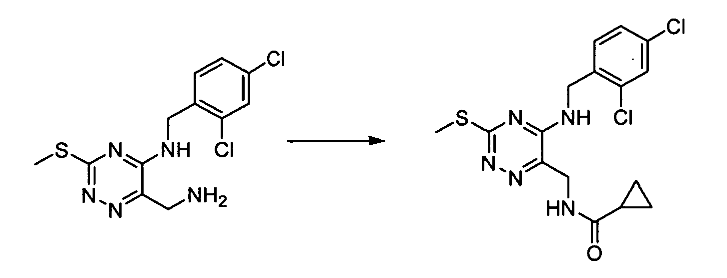
(5) Cyclopanecarbonyl chloride(14.6 mg) was added to a solution of (6-aminomethyl-3-methylsulfanyl-[1,2,4]triazin-5-yl)-(2,4-dichlorobenzyl)amine(42.6 mg) in THF(5 mL) and the mixture was stirred at room temperature for 10 minutes. A saturated aqueous sodium bicarbonate solution and chloroform were added to the reaction solution and the organic layer was separated, dried and concentrated. The residue was purified with a thin layer silica gel column chromatography(chloroform/methanol=95/5) to give cyclopropanecarboxylic acid [5-(2,4-dichlorobenzylamino)-3-methylsulfanyl-[1,2,4]triazin-6-yl]methyl]amide(36.7 mg) as a colorless powder.
   APCI-MS(m/e):398/400[M+H]⁺. 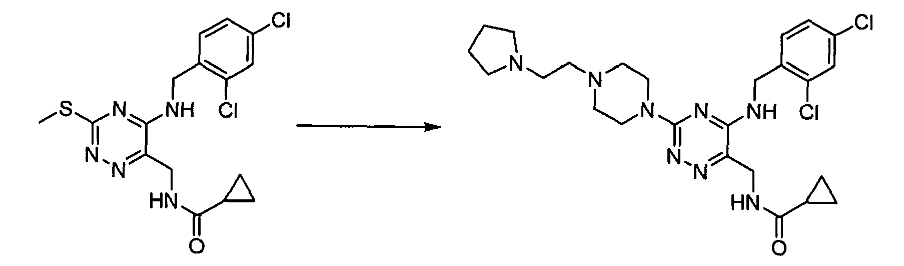
(6) A solution of cyclopropanecarboxylic acid [5-(2,4-dichlorobenzylamino)-3-methylsulfanyl-[1,2,4]triazin-6-yl]methyl]amide(36.7 mg) in chloroform(5 mL) was cooled to 0°C and a solution of m-chloroperbenzoic acid(75%, 27.6 mg) in chloroform(5 mL) was dropwise added thereto and the mixture was stirred at room temperature for an hour. 1-(2-Pyrrolidin-1-yl-ethyl)piperazine(43.8 mg), triethylamine(0.017 ml) and dioxane(20 ml) were added to the reaction solution, and the mixture was stirred at 100°C overnight. The reaction solution was concentrated and chloroform and a saturated aqueous potassium carbonate solution were added to the residue. The organic layer was separated, dried and concentrated. The residue was purified with a NH thin layer silica gel column chromatography(chloroform/methanol=95/5). Diisopropyl ether was added to the residue and the precipitate was filtered and dried to give cyclopropanecarboxylic acid{5-(2,4-dichlorobenzylamino)-3-[4-(2-pyrrolidin-1-yl-ethyl)-piperazin-1-yl]-[1,2,4]triazin-6-ylmethyl}-amide(17.4 mg) as a colorless powder.
   APCI-MS(m/e):533/535[M+H]⁺.

### Example 205

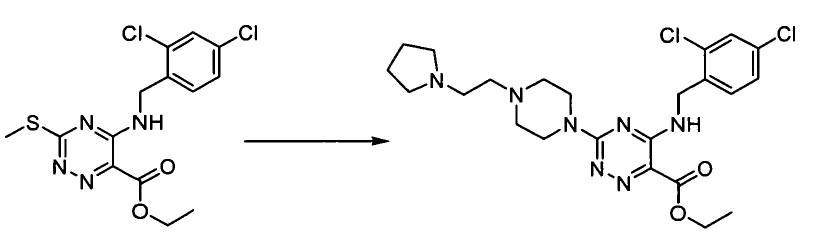

m-Chloroperbenzoic acid(3.91 g) was added to a solution of 5-(2,4-dichlorobenzylamino)-3-methylsulfanyl[1,2,4]triazin-6-carboxylic acid ethyl ester(5.40 g) in chloroform(100 mL) at 0°C, and the mixture was stirred at room temperature for 0.5 hour. A solution of 1-(2-pyrrolidin-1-ylethyl)piperazine(700 mg) and triethylamine(708 mg) in chloroform(50 mL) was added dropwise to the reaction solution for 30 minutes and the mixture was stirred at room temperature for a day. After the reaction solution was concentrated under reduced pressure, ethyl acetate was added to the residue and the organic solution was washed with a saturated aqueous sodium bicarbonate solution, water and brine, dried over magnesium sulfate and the solvent was distilled away. The residue was purified with a silica gel column chromatography (chloroform/methanol=100/0 to 87/13) to give an orange solid, which was recrystallized from ethyl acetate/hexane to give 5-(2,4-dichlorobenzylamino)-3-[4-(2-pyrrolidin-1-ylethyl)piperazin-1-yl]-[1,2,4]triazine-6-carboxylic acid ethyl ester(4.33 g) as pale yellow crystals.
APCI-MS(m/e):508/510[M+H]⁺.

### Examples 206-218

The following compounds were obtained by the reaction and treatment in the same manner as the aforementioned examples.

**[Table 24]**

| | | | | | | | |
|---|---|---|---|---|---|---|---|
| 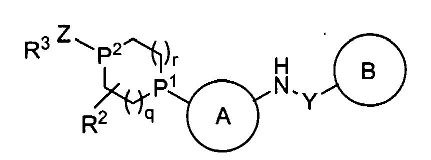 | | | | | | | |

| Example | R³ | Z | 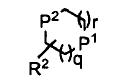 | Ring A | Y | Ring B | MS([M+H]⁺) |
|---|---|---|---|---|---|---|---|
| 206 | 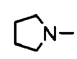 | -(CH₂)₂⁻ | 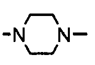 | 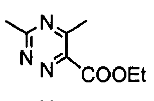 | -CH₂⁻ | 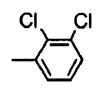 | 508/510, APCI |
| 207 | 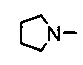 | -(CH₂)₂⁻ | 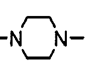 | 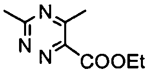 | -CH₂⁻ | 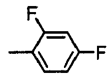 | 476, APCI |
| 208 | 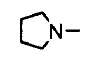 | -(CH₂)₂⁻ | 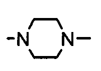 | 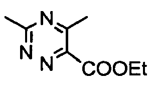 | -CH₂⁻ | 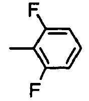 | 476, APCI |
| 209 | 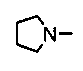 | -(CH₂)₂⁻ | 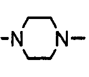 | 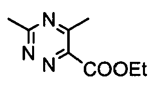 | -(CH₂)₂⁻ | 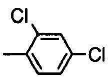 | 522/524, APCI |
| 210 | 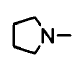 | -COCH₂⁻ | 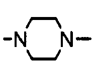 | 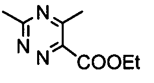 | -CH₂⁻ | 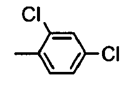 | 522/524, APCI |
| 211 | 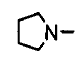 | -(CH₂)₂⁻ | 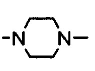 | 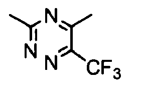 | -CH₂⁻ | 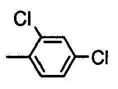 | 504/506, APCI |
| 212 | 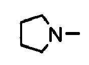 | -(CH₂)₂⁻ | 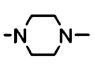 | 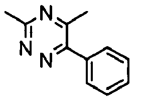 | -CH₂⁻ | 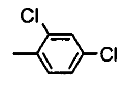 | 512/514, APCI |
| 213 | 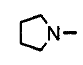 | -(CH₂)₂⁻ | 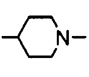 | 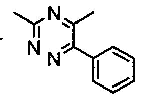 | -CH₂⁻ | 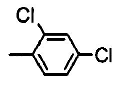 | 511/513, APCI |

**[Table 25]**

| | | | | | |
|---|---|---|---|---|---|
| 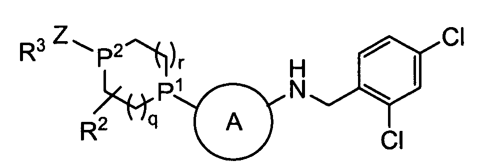 | | | | | |

| Example | R³ | Z | 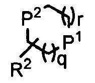 | Ring A | MS([M+H]⁺) |
|---|---|---|---|---|---|
| 214 | 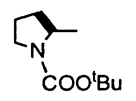 | -CO⁻ | 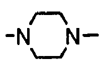 | 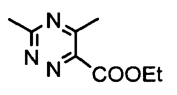 | 585/587, APCI |
| 215 | 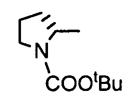 | -CO⁻ | 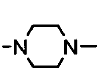 | 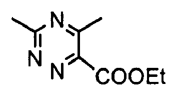 | 585/587, APCI |
| 216 | 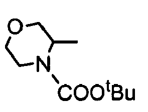 | -CO⁻ | 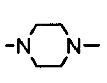 | 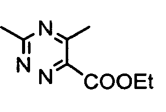 | 624/626, APCI |
| 217 | 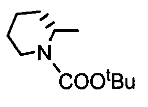 | -CO⁻ | 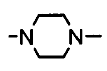 | 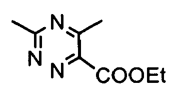 | 622/624, APCI |
| 218 | 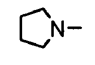 | -CH₂CO⁻ | 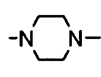 | 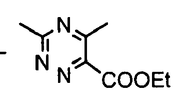 | 522/524, APCI |

### Example 219

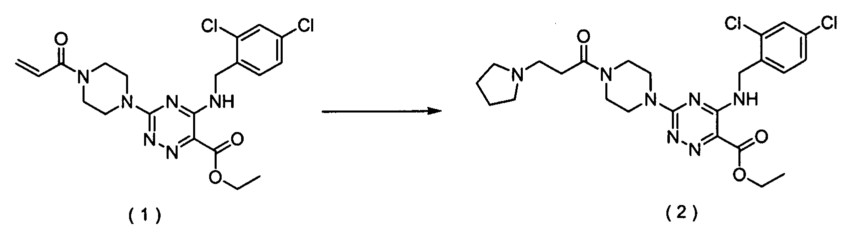

Pyrrolidine(15 µl) was added to a solution of 3-(4-acryloyl-piperazin-1-yl)-5-(2,4-dichlorobenzylamino)-[1,2,4]triazin-6-carboxylic acid ethyl ester(51 mg) in methylene chloride(5 mL) and the mixture was stirred at room temperature. Pyrrolidine(50 µl) was further added thereto and the mixture was stirred at room temperature overnight. The solvent was distilled away and the residue was purified with a silica gel column chromatography (chloroform/methanol=90/10 to 30/70) to give 5-(2,4-dichlorobenzylamino)-3-[4-(3-pyrrolidin-1-yl-propionyl)-piperazin-1-yl]-[1,2,4]triazin-6-carboxylic acid ethyl ester(37 mg) as a pale yellow oily substance. APCI-MS(m/e):536/538[M+H]⁺.

### Examples 220-254

The following compounds were obtained by the reaction and treatment in the same manner as the aforementioned examples.

**[Table 26]**

| | | | | | | | | |
|---|---|---|---|---|---|---|---|---|
| 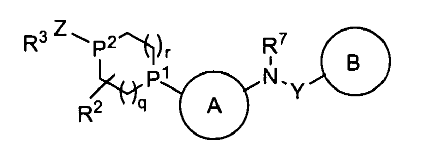 | | | | | | | | |

| Example | R³ | Z | 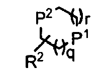 | Ring A | R⁷ | Y | Ring B | MS([M+H]⁺) |
|---|---|---|---|---|---|---|---|---|
| 220 | 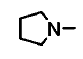 | -NHCO- | 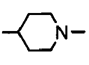 | 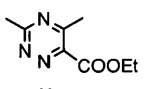 | H | -CH₂⁻ | 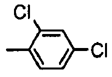 | 522/524, APCI |
| 221 | 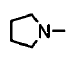 | -(CH₂)₂⁻ | 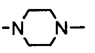 | 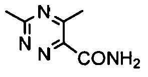 | H | -CH₂⁻ | 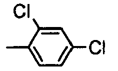 | 479/481, APCI |
| 222 | 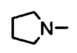 | -(CH₂)₂⁻ | 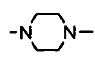 | 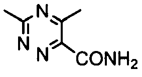 | H | -CH₂⁻ | 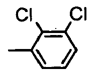 | 479/481, APCI |
| 223 | 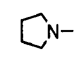 | -(CH₂)₂⁻ | 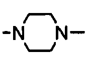 | 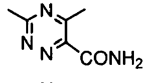 | H | -CH₂⁻ | 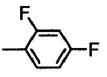 | 447, APCI |
| 224 | 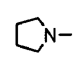 | -(CH₂)₂⁻ | 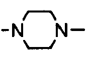 | 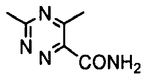 | H | -CH₂⁻ | 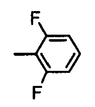 | 447, APCI |
| 225 | 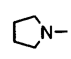 | -(CH₂)₂⁻ | 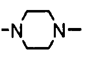 | 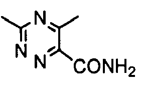 | H | -(CH₂)₂⁻ | 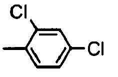 | 493/495, APCI |
| 226 | 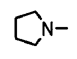 | -COCH₂⁻ | 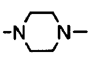 | 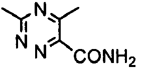 | H | -CH₂⁻ | 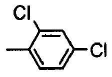 | 493/495, APCI |
| 227 | 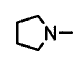 | -CO⁻ | 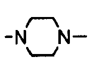 | 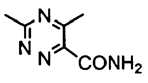 | H | -CH₂⁻ | 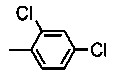 | 479/481, APCI |
| 228 | 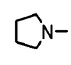 | -CH₂CO⁻ | 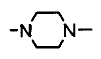 | 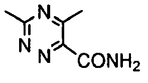 | Me | -CH₂⁻ | 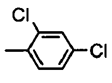 | 507/509, APCI |
| 229 | 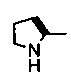 | -CO⁻ | 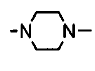 | 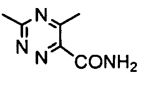 | H | -CH₂⁻ | 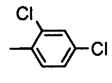 | 479/481, APCI |
| 230 | 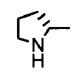 | -CO- | 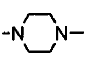 | 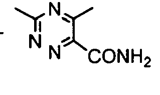 | H | -CH₂⁻ | 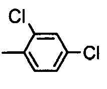 | 479/481, APCI |

**[Table 27]**

| | | | | | |
|---|---|---|---|---|---|
| 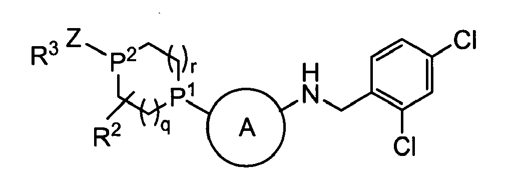 | | | | | |

| Example | R³ | Z | 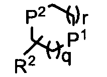 | Ring A | MS([M+H]⁺) |
|---|---|---|---|---|---|
| 231 | 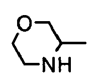 | -CO⁻ | 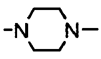 | 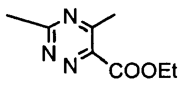 | 524/526, APCI |
| 232 | 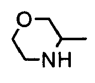 | -CO⁻ | 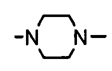 | 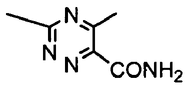 | 495/497, APCI |
| 233 | 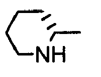 | -CO⁻ | 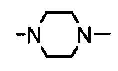 | 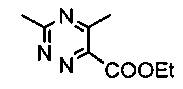 | 522/524, APCI |
| 234 | 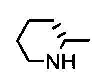 | -CO⁻ | 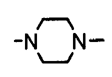 | 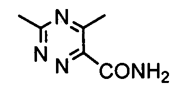 | 493/495, APCI |
| 235 | 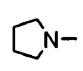 | -CH₂CO⁻ | 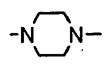 | 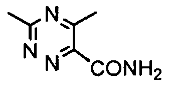 | 493/495, APCI |
| 236 | 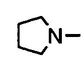 | -(CH₂)₂CO⁻ | 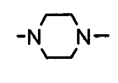 | 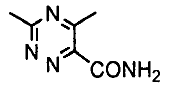 | 507/509, APCI |
| 237 | 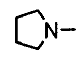 | -NHCO⁻ | 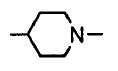 | 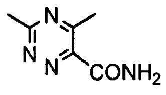 | 493/495, APCI |
| 238 | 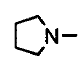 | -(CH₂)₂⁻ | 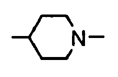 | 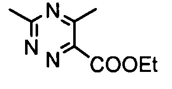 | 507/509, APCI |
| 239 | 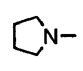 | -(CH₂)₂⁻ | 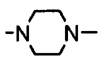 | 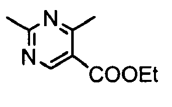 | 507/509, APCI |

**[Table 28]**

| | | | | | | |
|---|---|---|---|---|---|---|
| 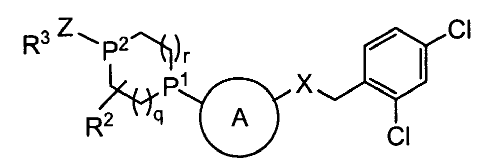 | | | | | | |

| Example | R³ | Z | 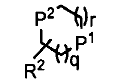 | Ring A X | | MS([M+H]⁺) |
|---|---|---|---|---|---|---|
| 240 | 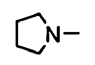 | -CH₂CO⁻ | 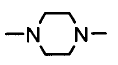 | 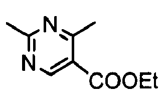 | -O- | 522/524, APCI |
| 241 | 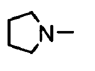 | -(CH₂)₂⁻ | 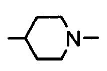 | 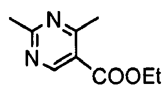 | -NH- | 506/508, APCI |
| 242 | 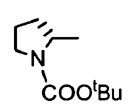 | -CO⁻ | 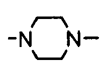 | 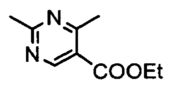 | -NH- | 607/609, APCI |
| 243 | 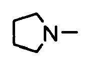 | -(CH₂)₂⁻ | 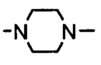 | 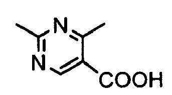 | -NH- | 479/481, APCI |
| 244 | 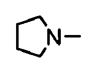 | -CH₂CO⁻ | 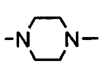 | 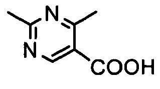 | -O- | 494/496, APCI |
| 245 | 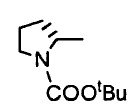 | -CO⁻ | 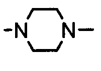 | 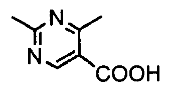 | -NH- | 579/581, APCI |
| 246 | 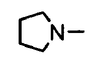 | -(CH₂)₂⁻ | 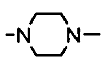 | 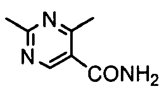 | -NH- | 478/480, APCI |
| 247 | 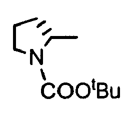 | -CO⁻ | 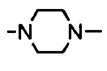 | 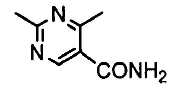 | -NH- | 578/580, APCI |
| 248 | 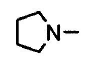 | -CH₂CO⁻ | 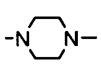 | 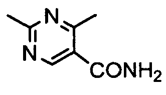 | -O- | 493/495, APCI |
| 249 | 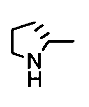 | -CO⁻ | 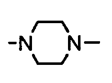 | 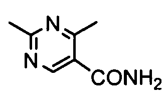 | -NH- | 478/480, APCI |
| 250 | 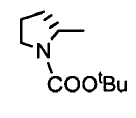 | -CONHCH₂- | 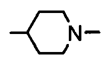 | 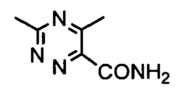 | -NH- | 607/609, APCI |

**[Table 29]**

| | | | | | |
|---|---|---|---|---|---|
| 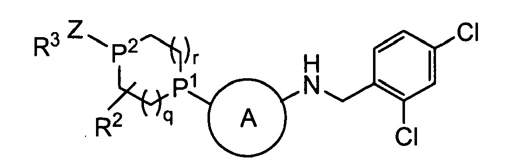 | | | | | |

| Example | R³ | Z | 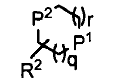 | Ring A | MS([M+H]⁺) |
|---|---|---|---|---|---|
| 251 | 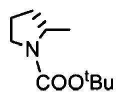 | -CONH⁻ | 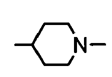 | 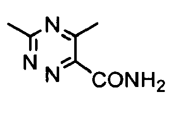 | 593/595, APCI |
| 252 | 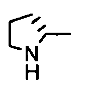 | -CONHCH₂⁻ | 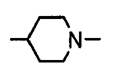 | 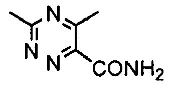 | 507/509, APCI |
| 253 | 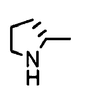 | -CONH⁻ | 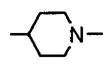 | 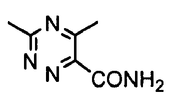 | 493/495, APCI |
| 254 | 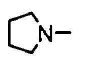 | -(CH₂)₂⁻ | 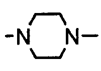 | 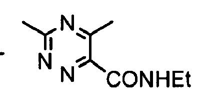 | 507/509, APCI |

### Example 255

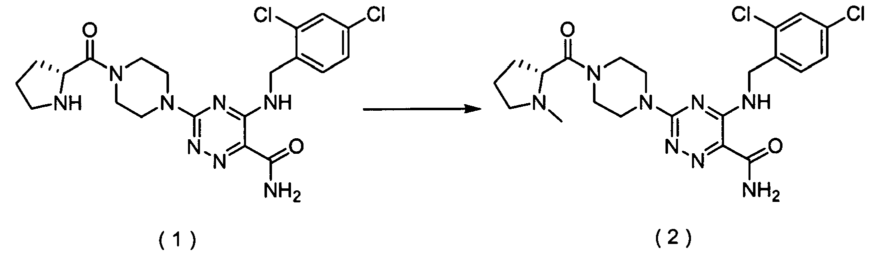

Sodium triacetoxyborohydride(261 mg) was added to a solution of 5-(2,4-dichlorobenzylamino)-3-[4-(pyrrolidin-2-carbonyl)-piperazin-1-yl]-[1,2,4]triazin-6-carboxylic amide(116 mg), acetic acid(21 µl) and paraformaldehyde(29.6 mg) in THF(5 mL) at 0°C, and the mixture was stirred at room temperature overnight. Paraformaldehyde(26 mg), acetic cid(21 µl) and Sodium triacetoxyborohydride(515 mg) were added thereto and the mixture was stirred at room temperature for 2 days. Water was added to the reaction solution and the solvent was distilled away. The residue was purified with a silica gel column chromatography (chloroform/methanol=100/0 to 20/80) twice and with a NH silica gel column chromatography(ethyl acetate/methanol=100/0 to 90/10) once to give 5-(2,4-dichlorobenzylamine)-3-[4-(1-methyl-pyrrolidin-2-carbonyl)-piperazin-1-yl]-[1,2,4]triazine-6-carboxylic amide(5 mg) as a colorless solid.
APCI-MS(m/e):493/495[M+H]⁺.

### Example 256

The following compounds were obtained by the reaction and treatment in the same manner as Example 255. 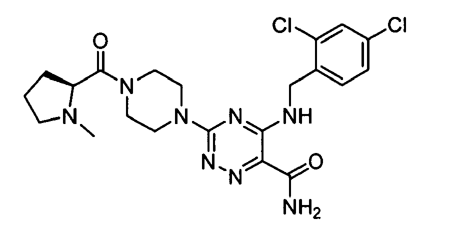 APCI-MS(m/e):493/495[M+H]⁺.

### Example 257

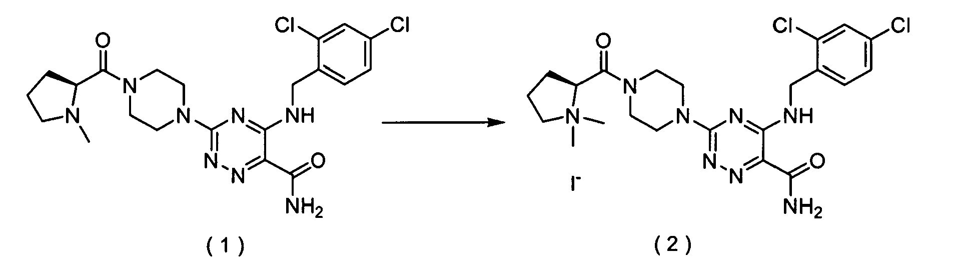

Methyl iodide(10 µl) was added to a solution of 5-(2,4-dichlorobenzylamine)-3-[4-(1-methyl-pyrrolidin-2-carbonyl)-piperazin-1-yl]-[1,2,4]triazine-6-carboxylic amide(27 mg) in THF(1.5 mL) and the mixture was stirred at room temperature overnight. The solvent was distilled away and the residue was recrystallized from methanol-ethyl acetate to give2-{4-[6-carbamoyl-5-(2,4-dichlorobenzylamino)-[1,2,4]triazin-3-yl]-piperazin-1-carbonyl}-1,1-dimethyl-pyrrolidinium iodide(21 mg) as pale yellow crystals.
APCI-MS(m/e):507/509[M+H]⁺.

### Example 258

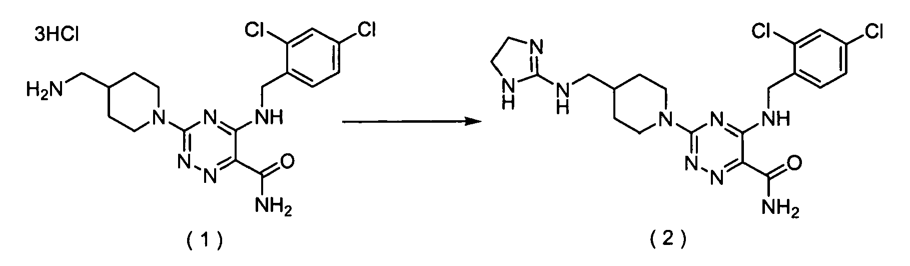

2-(4-Aminomethyl-piperidin-1-yl)-4-(2,4-dichlorobenzylamino)-pyrimidine-5-carboxylic amide(95 mg), 2-methylsulfanyl-4,5-dihydro-1H-imidazole(155 mg) and triethylamine(325 µl) were dissolved in ethanol(4 mL) and the mixture was stirred at 60°C for 6 days. The reaction solution was concentrated under reduced pressure and the residue was purified with a thin layer silica gel column chromatography. Diisopropyl ether was added to the solid residue and it was triturated to give 4-(2,4-dichlorobenzylamino)2-{4-[(4,5-dihydro-1H-imidazol-2-ylamino)-methyl]-piperidin-1-yl}-pyrimidine-5-carboxylic amide(15 mg) as a pale yellow solid.
APCI-MS(m/e):478/480[M+H]⁺.

### Examples 259-294

The following compounds were obtained by the reaction and treatment in the same manner as the aforementioned examples.

**[Table 30]**

| | | | | | |
|---|---|---|---|---|---|
| 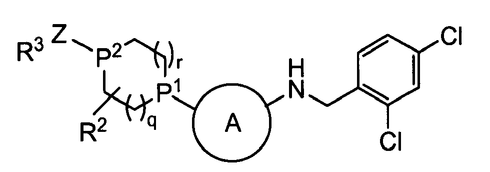 | | | | | |

| Example | R³ | Z | 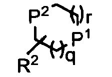 | Ring A | MS([M+H]⁺) |
|---|---|---|---|---|---|
| 259 | 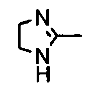 | -NH⁻ | 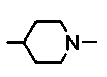 | 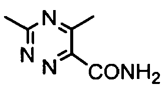 | 464/466, APCI |
| 260 | 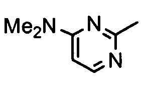 | -NH⁻ | 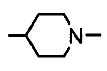 | 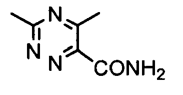 | 517/519, APCI |
| 261 | 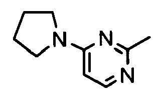 | -NH⁻ | 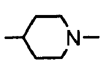 | 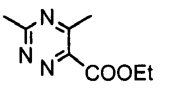 | 572/574, APCI |
| 262 | 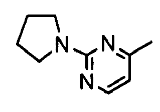 | -NH⁻ | 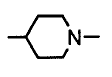 | 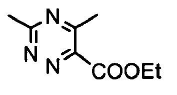 | 572/574, APCI |
| 263 | 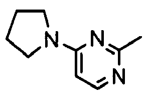 | -NH⁻ | 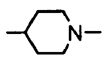 | 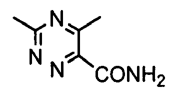 | 543/545, APCI |
| 264 | 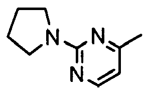 | -NH⁻ | 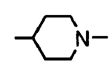 | 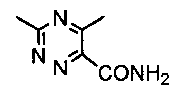 | 543/545, APCI |
| 265 | 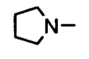 | -(CH₂)₂⁻ | 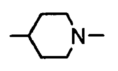 | 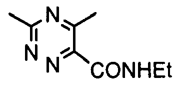 | 506/508, APCI |
| 266 | 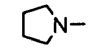 | -(CH₂)₂⁻ | 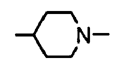 | 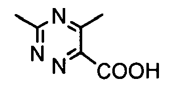 | 479/481, APCI |
| 267 | 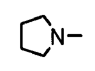 | -(CH₂)₂⁻ | 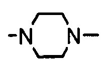 | 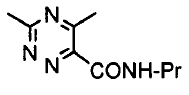 | 521/523, APCI |
| 268 | 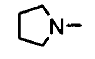 | -(CH₂)₂⁻ | 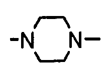 | 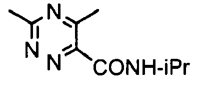 | 521/523, APCI |
| 269 | 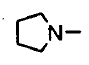 | -(CH₂)₂⁻ | 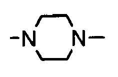 | 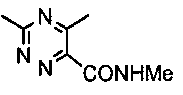 | 493/495, APCI |
| 270 | 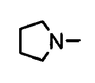 | -(CH₂)₂⁻ | 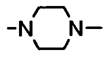 | 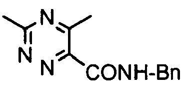 | 569/571, APCI |

**[Table 31]**

| | | | | | |
|---|---|---|---|---|---|
| 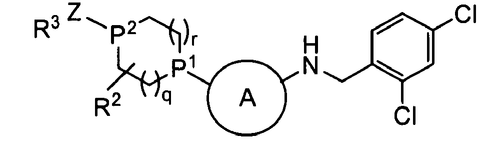 | | | | | |

| Example | R³ | Z | 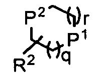 | Ring A | MS([M+H]⁺) |
|---|---|---|---|---|---|
| 271 | 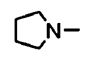 | -(CH₂)₂⁻ | 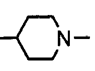 | 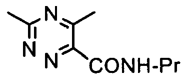 | 520/522, APCI |
| 272 | 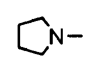 | -(CH₂)₂⁻ | 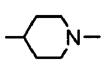 | 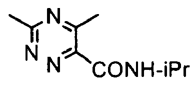 | 520/522, APCI |
| 273 | 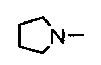 | -(CH₂)₂⁻ | 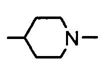 | 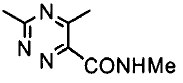 | 492/494, APCI |
| 274 | 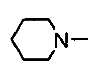 | -(CH₂)₂⁻ | 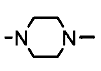 | 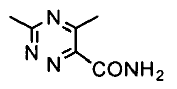 | 493/495, APCI |
| 275 | Me₂N- | -(CH₂)₂⁻ | 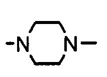 | 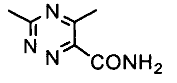 | 453/455, APCI |
| 276 | Et₂N- | -(CH₂)₂⁻ | 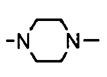 | 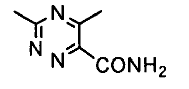 | 481/483, APCI |
| 277 | 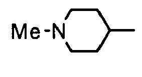 | -CH₂⁻ | 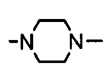 | 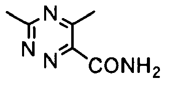 | 493/495, APCI |
| 278 | (iPr)₂N- | -(CH₂)₂⁻ | 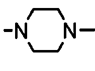 | 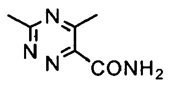 | 509/511, APCI |
| 279 | 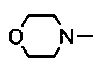 | -(CH₂)₂⁻ | 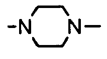 | 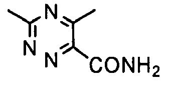 | 495/497, APCI |
| 280 | 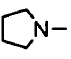 | -(CH₂)₂⁻ | 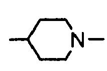 | 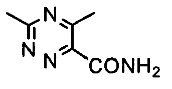 | 478/480, APCI |
| 281 | 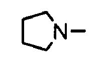 | -(CH₂)₂⁻ | 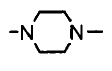 | 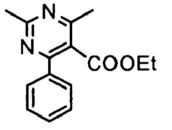 | 583/585, APCI |
| 282 | 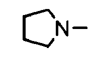 | -(CH₂)₂⁻ | 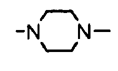 | 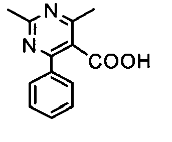 | 555/557, APCI |

**[Table 32]**

| | | | | | |
|---|---|---|---|---|---|
| 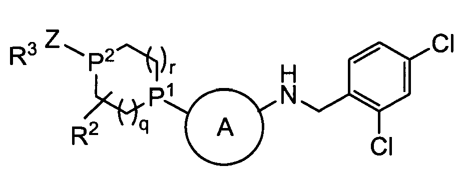 | | | | | |

| Example | R³ | Z | 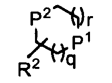 | Ring A | MS([M+H]⁺) |
|---|---|---|---|---|---|
| 283 | 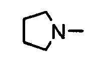 | -(CH₂)₂⁻ | 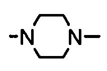 | 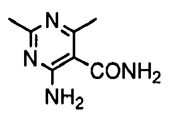 | 493/495, APCI |
| 284 | 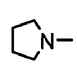 | -(CH₂)₂⁻ | 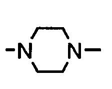 | 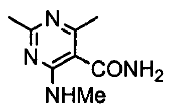 | 507/509, APCI |
| 285 | 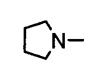 | -(CH₂)₂⁻ | 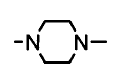 | 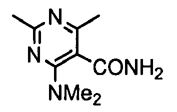 | 521/523, APCI |
| 286 | 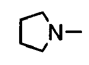 | -(CH₂)₂⁻ | 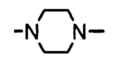 | 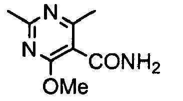 | 508/510, APCI |
| 287 | 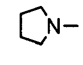 | -(CH₂)₂⁻ | 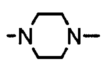 | 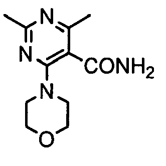 | 563/565, APCI |

**[Table 33]**

| | | | | | |
|---|---|---|---|---|---|
| 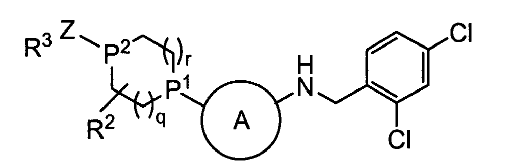 | | | | | |

| Example | R³ | Z | 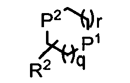 | Ring A | MS([M+H]⁺) |
|---|---|---|---|---|---|
| 288 | 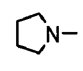 | -(CH₂)₂⁻ | 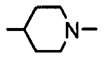 | 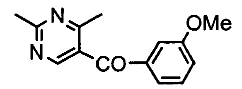 | 568/570, APCI |
| 289 | 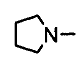 | -CH₂CO⁻ | 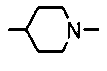 | 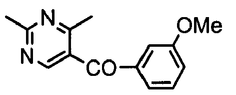 | 583/585, APCI |
| 290 | 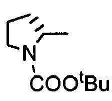 | -CO⁻ | 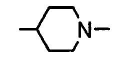 | 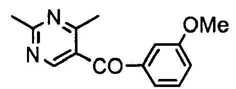 | 669/671, APCI |
| 291 | 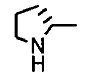 | -CO⁻ | 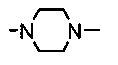 | 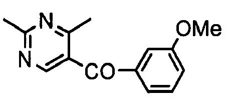 | 569/571, APCI |
| 292 | 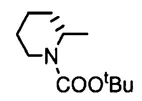 | -CO⁻ | 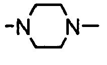 | 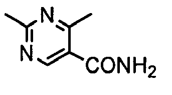 | 592/594, APCI |
| 293 | 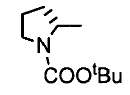 | -CO⁻ | 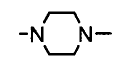 | 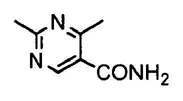 | 578/580, APCI |
| 294 | 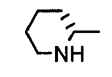 | -CO⁻ | 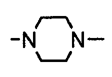 | 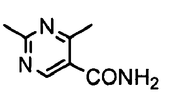 | 492/494, APCI |

### Example 295

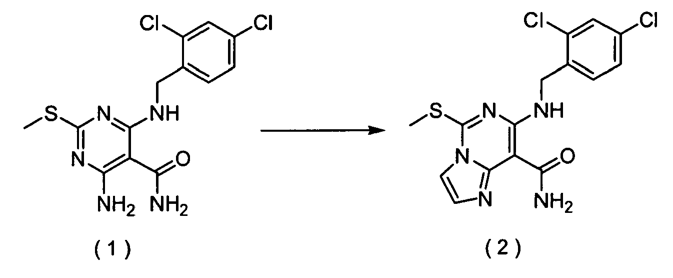

(1) Chloroacetoaldehyde(0.344 mL) was added to a solution of 4-amino-6-(2,4-dichlorobenzylamino)-2-methylsulfanyl-pyrimidine-5-carboxylic amide(502 mg) in 1,4-dioxane(70 mL) and the mixture was stirred at 100°C overnight. The reaction solution was concentrated under reduced pressure, ethyl acetate was added to the residue and the organic solution was washed with a saturated aqueous sodium bicarbonate solution and brine, dried and concentrated. The residue was purified with silica gel column chromatography(hexane/ethyl acetate=100/0 to 35/65) to give 7-(2,4-dichlorobenzylamino)-5-methylsulfanyl-imidazo[1,2-c]pyrimidine-8-carboxylic amide(108 mg) as a yellow solid.
   APCI-MS(m/e):382/384[M+H]⁺. 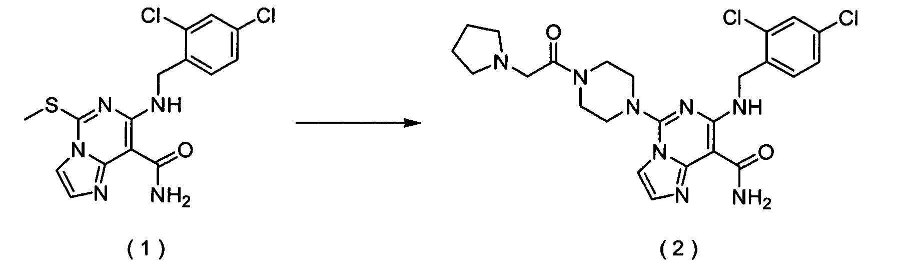
(2) 7-(2,4-dichlorobenzylamino)-5-methylsulfanyl-imidazo[1,2-c]pyrimidine-8-carboxylic amide(15 mg) and 1-piperidin-1-yl-2-pyrrolidin-1-yl-ethanone(56 mg) were dissolved in dimethylacetamide(0.2 mL) and the mixture was stirred at 80-100°C for 9 hours. Further 1-piperidin-1-yl-2-pyrrolidin-1-yl-ethanone(25 mg) was added and the mixture was stirred at 100°C for 3 hours. After standing to cool, an aqueous sodium bicarbonate solution was added and the mixture was extracted with ethyl acetate/diethyl ether. The combined organic layer was washed with brine, dried over magnesium sulfate and the solvent was distilled away. The residue was purified with a thin layer NH silica gel chromatography(hexane/ethyl acetate=1/2) to give 7-(2,4-dichlorobenzylamino)-5-[4-(2-pyrrolidin-1-yl-acetyl)-piperazin-1-yl]-imidazo[1,2-c]pyrimidine-8-carboxylic amide(5 mg) as pale yellow crystals.
   APCI-MS(m/e):531/533[M+H]⁺.

### Example 296

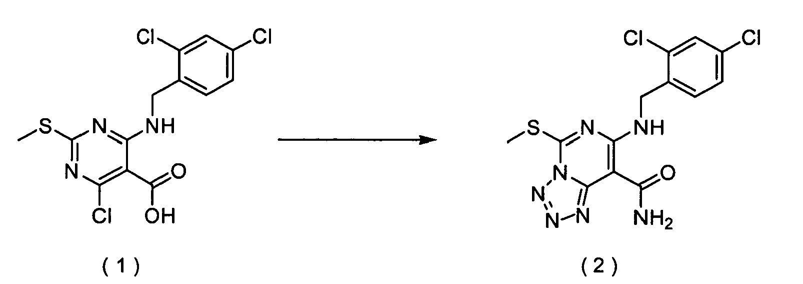

(1) Sodium azide(17.2 mg) was added to a solution of 4-amino-6-(2,4-dichlorobenzylamino)-2-methylsulfanyl-pyrimidine-5-carboxylic acid(100 mg) in DMF(2 mL) and the mixture was stirred at 50°C for an hour. Ethyl acetate was added to the reaction mixture, and the mixture was washed with water and brine, dried over sodium sulfate and the solvent was distilled away. The residue was washed with hexane and dried to give 7-(2,4-dichlorobenzylamino)-5-methylsulfanyl-tetrazolo[1,5-c]pyrimidine-8-carboxylic amide(85 mg) as a pale yellow solid.
   APCI-MS(m/e):384/386[M+H]⁺. 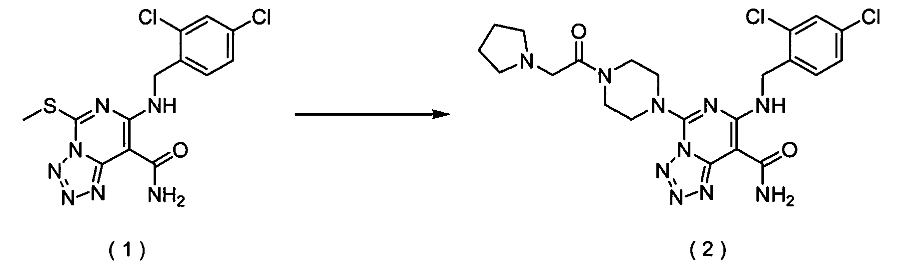
(2) 7-(2,4-dichlorobenzylamino)-5-[4-(2-pyrrolidin-1-yl-acetyl)-piperidin-1-yl]-tetrazolo[1,5-c]pyrimidine-8-carboxylic amide was obtained by reacting and treating 7-(2,4-dichlorobenzylamino)-5-methylsulfanyl-tetrazolo[1,5-c]pyrimidine-8-carboxylic amide in the same manner as Example 295(2).
   APCI-MS(m/e):533/535[M+H]⁺.

### Examples 297 and 298

The following compounds were obtained by the reaction and treatment in the same manner as the aforementioned examples.

**[Table 34]**

| | | | |
|---|---|---|---|
| 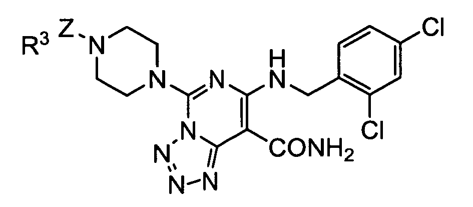 | | | |

| Example | R³ | Z | MS([M+H]⁺) |
|---|---|---|---|
| 297 | 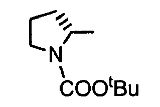 | -CO⁻ | 619/621, APCI |
| 298 | 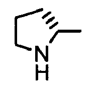 | -CO⁻ | 519/521, APCI |

### Example 299

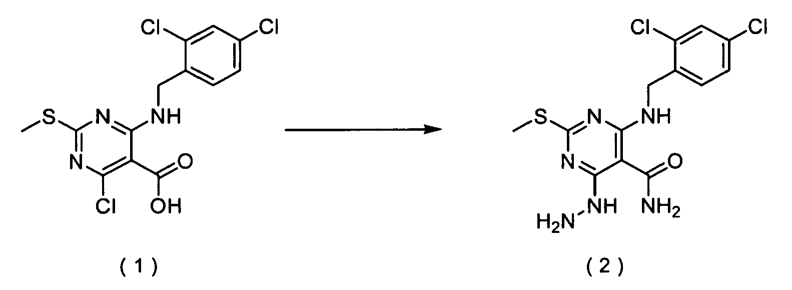

(1) Hydrazine hydrate(0.66 g) was added to a solution of 4-amino-6-(2,4-dichlorobenzylamino)-2-methylsulfanyl-pyrimidine-5-carboxylic acid(1.0 g) in 1,4-dioxane(20 mL) and the mixture was stirred at room temperature. Ethyl acetate was added thereto, and the mixture was washed with a saturated aqueous sodium bicarbonate solution and brine, dried over magnesium sulfate and the solvent was distilled away. The residue was washed with hexane and dried to give 4-(2,4-dichlorobenzylamino)-6-hydrazino-2-methylsulfanyl-pyrimidine-5-carboxylic amide(1.01 g) as a colorless solid.
   APCI-MS(m/e):373/375[M+H]⁺. 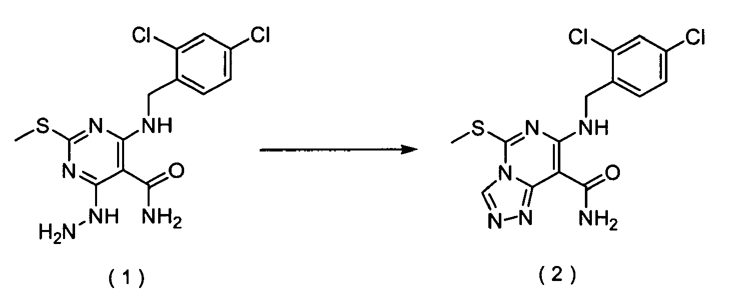
(2) Methyl orthoformate(29.6 g) was added to a solution of 4-(2,4-dichlorobenzylamino)-6-hydrazino-2-methylsulfanyl-pyrimidine-5-carboxylic amide(2.66 g) in DMF(70 mL) and the mixture was stirred at room temperature for an hour and at 55°C for 3.5 hours. After standing to cool, diisopropyl ether/ethyl acetate(9/1) was added thereto and the precipitated crystals were filtered. It was washed with diisopropyl ether and dried to give 7-(2,4-dichlorobenzylamino)-5-methylsulfanyl-[1,2,4]triazolo[4,3-c]pyrimidine-8-carboxylic amide(1.51 g) as yellow crystals.
   APCI-MS(m/e):383/385[M+H]⁺. 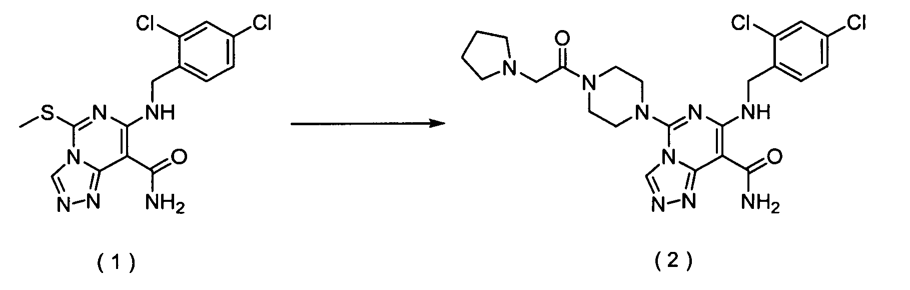
(3) 7-(2,4-dichlorobenzylamino)-5-[4-(2-pyrrolidin-1-yl-acetyl)-piperazin-1-yl][1,2,4]triazolo[4,3-c]pyrimidine-8-carboxylic amide was obtained by reacting and treating 7-(2,4-dichlorobenzylamino)-5-methylsulfanyl-[1,2,4]triazolo[4,3-c]pyrimidine-8-carboxylic amide in the same manner as Example 295(2).
   APCI-MS(m/e):532/534[M+H]⁺.

### Examples 300-303

The following compounds were obtained by the reaction and treatment in the same manner as the aforementioned examples.

**[Table 35]**

| | | | |
|---|---|---|---|
| 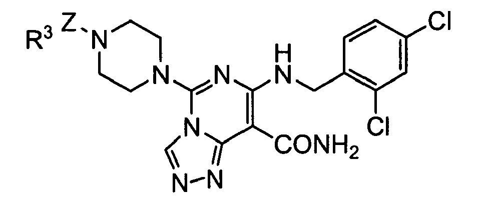 | | | |

| Example | R³ | Z | MS([M+H]⁺) |
|---|---|---|---|
| 300 | 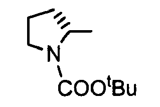 | -CO⁻ | 618/620, APCI |
| 301 | 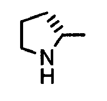 | -CO⁻ | 518/520, APCI |

**[Table 36]**

| | | | |
|---|---|---|---|
| 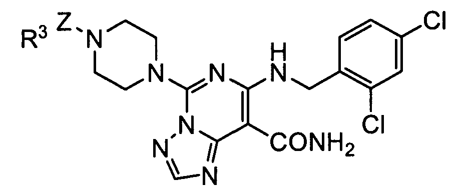 | | | |

| Example | R³ | Z | MS([M+H]⁺) |
|---|---|---|---|
| 302 | 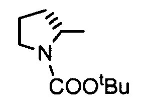 | -CO⁻ | 618/620, APCI |
| 303 | 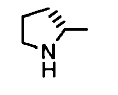 | -CO⁻ | 518/520, APCI |

### Example 304

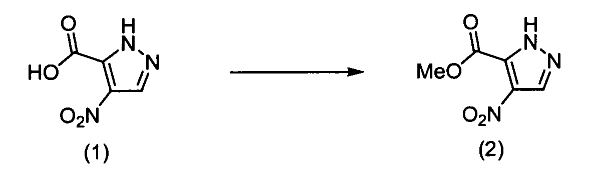

(1) Concentrated sulfuric acid(10.1 mL) was added to a suspension of 4-nitro-3-pyrazole(1)(51.06 g) in methanol(515 mL) and the mixture was refluxed under heating with stirring overnight. The reaction solution was ice-cooled and made mildly alkaline by adding a saturated aqueous sodium bicarbonate solution, and methanol was distilled away under reduced pressure. The residue was extracted with ethyl acetate six times, the combined organic layer was dried and the solvent was distilled away. The residue was triturated with ethyl acetate/hexane and dried to give Compound(2)(47.61 g) as a colorless powder.
   ESI-MS(m/e):170[M-H]⁻ 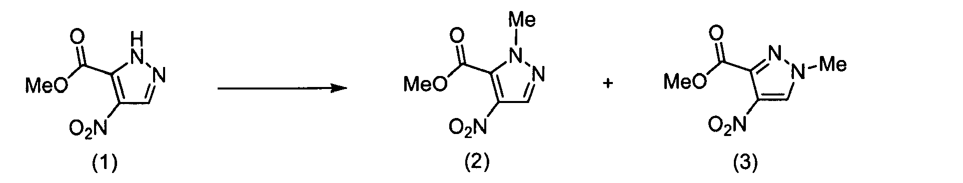
(2) Metallic sodium(6.71 g) was added carefully and slowly to thoroughly dried methanol(830 mL) and the solution was ice-cooled after the fragment of metallic sodium was completely dissolved. Compound(1)(43.61 g) was added to the solution, then methyl iodide(43.64 mL) was added and the mixture was stirred at 60-70°C for 4 hours. The reaction solution was concentrated under reduced pressure, chloroform was added to the residue and the mixture was washed with a saturated aqueous sodium bicarbonate solution and brine, dried and the solvent was distilled away. The residue was purified with a silica gel column chromatography(hexane/ethyl acetate=4/1 to 1/1) to give Compound(2)(17.0 g) as a yellow oily substance and Compound(3)(26.8 g) as a colorless powder. Compound(2):
   APCI-MS(m/e):186[M+H]⁺,
   ¹HNMR(500MHZ/DMSO-d₆ )δ(ppm):3.95(s,3H),4.00(s,3H),8.37(s,1H). Compound(3):
   APCI-MS(m/e):186[M+H]⁺,
   ¹HNMR(500MHZ/DMSO-d₆ )δ(ppm):3.94(s,3H),4.00(s,3H),8.95(s,1H). 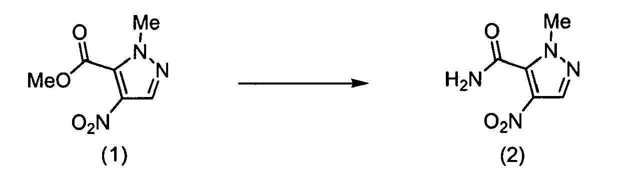
(3) Compound(1)(17.0 g) was added to 28% ammonia water(285 mL) and the mixture was stirred at 60°C for 6 hours. After standing to cool, the reaction solution was concentrated under reduced pressure to give Compound(2)(16.0 g) as a colorless solid.
   ESI-MS(m/e):169[M-H]⁻. 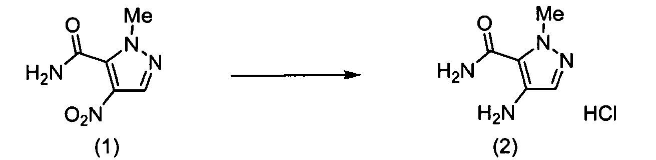
(4) 10% Palladium-carbon was added to a suspension of Compound(1)(19.8 g) in methanol(350 mL) and the mixture was stirred under hydrogen pressure(50 psi) at room temperature overnight using a medium pressure reduction apparatus. The reaction solution was poured into 4N hydrochloric acid/ethyl acetate(120 mL) and the insoluble materials were filtered through Celite. The filtrate was concentrated under reduced pressure, and the residue was triturated with ethyl acetate and dried to give Compound(2)(19.95 g) as a pale orange powder.
   APCI-MS(m/e):141 [M+H]⁺. 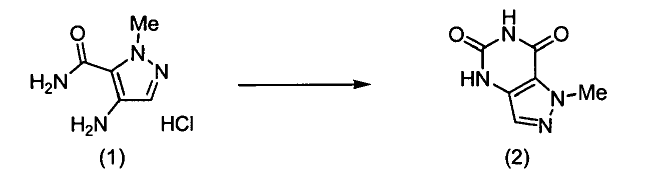
(5) Compound(1)(17.3 g) and urea(32.9 g) were put in a reaction vessel and were stirred at 200°C for an hour. After standing to cool, a warm 1N aqueous sodium hydroxide solution(330 mL) was added and dissolved. The mixture was ice-cooled and adjusted to pH 4 by adding acetic acid(55 mL). The precipitated crystals were filtered and washed with water three times and diethyl ether once, and dried to give Compound(2)(12.6 g) as a colorless powder.
   APCI-MS(m/e):189[M+Na]⁺. 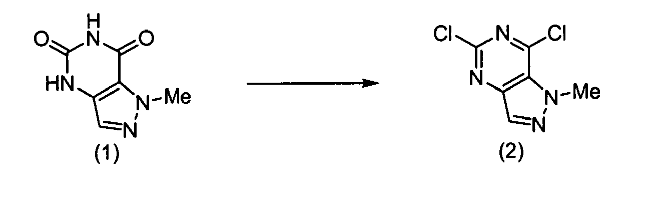
(6) Diethyl aniline(16.9 mL) was slowly added to a suspension of Compound(1)(14.0 g) and phosphorous oxychloride(84 mL) and the mixture was refluxed under heating with stirring for 22 hours. After standing to cool, the reaction solution was concentrated under reduced pressure and azeotropically distilled with toluene twice. The residue was slowly added to ice water, chloroform was added and the mixture was stirred. The mixture was separated and the chloroform layer was washed with brine, dried and the solvent was distilled away. The residue was purified with a silica gel column chromatography(hexane/ethyl acetate=10/1 to 2/1) to give Compound(2)(15.3 g) as a yellow solid.
   APCI-MS(m/e):203/205[M+H]⁺. 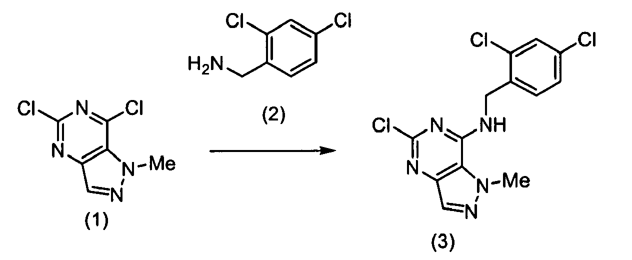
(7) Compound(2)(18.6 g) and triethylamine(17.8 g) were successively added to a solution of Compound(1)(14.3 g) in 1,4-dioxane(260 mL) and the mixture was stirred at room temperature for 4 hours. Ethyl acetate was added to the reaction solution and the mixture was washed with a saturated aqueous sodium bicarbonate solution and brine, dried and the solvent was distilled away. The residue was purified with a silica gel column chromatography(hexane/ethyl acetate=2/1 to 1/2). The product was triturated in diethyl ether and dried to give Compound(2)(20.0 g) as a colorless powder.
   APCI-MS(m/e):342/344[M+H]⁺. 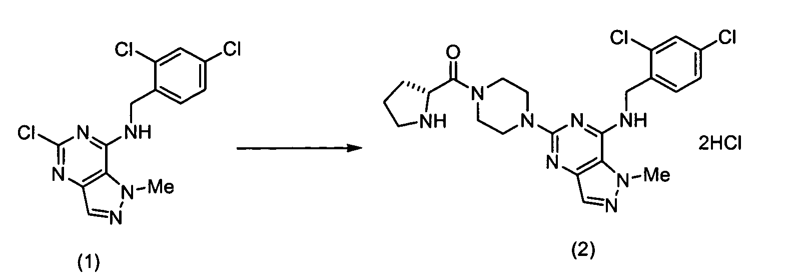
(8) Compound(2) was obtained by reacting and treating Compound(1) in the same manner as Example 1(4) and (5).
   APCI-MS(m/e):489/491 [M+H]⁺.

### Example 305

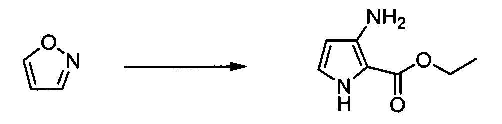

(1) A solution of sodium ethoxide in ethanol(21w%, 369 g) was slowly added to a solution of isoxazole(75 g) in ethanol(300 mL) at 8°C or lower for 2 hours and the mixture was stirred for 45 minutes. Acetic acid(22.5 g), 2-aminodimalonic acid diethyl hydrochloride(143 g) and sodium acetate(61.4 g) were successively added to the reaction solution and the mixture was stirred at room temperature overnight. The mixture was concentrated under reduced pressure, chloroform was added to the residue and the mixture was washes with water and dried. Ethanol(1.14 L) and a solution of sodium ethoxide in ethanol(21 w%, 262 mL) were added to the residue and the mixture was stirred overnight. To the reaction solution was added acetic acid(42.2 g) and the mixture was concentrated under reduced pressure. Water was added to the residue and the mixture was adjusted to pH 7 by adding a saturated aqueous sodium bicarbonate solution and extracted with chloroform. The extract was dried, and purified with a silica gel column chromatography(hexane/ethyl acetate=2/1) to give 3-amino-1H-pyrrole-2-carboxylic acid ethyl ester(60.7 g)
   APCI-MS(m/e):155[M+H]⁺. 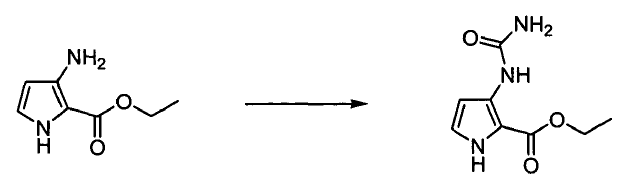
(2) 3-Amino-1H-pyrrole-2-carboxylic acid ethyl ester (82.6 g) was dissolved in acetic acid (500 mL) and water (50 mL) and an aqueous potassium cyanate (130.4 g) solution (250 mL) was added thereto for an hour, and the mixture was further stirred at room temperature for an hour. The reaction solution was concentrated, and water(500 mL) and ethyl acetate(200 mL) were added, the mixture was neutralized by adding potassium carbonate. The precipitate was filtered, washed with water and ethyl acetate and dried to give 3-ureido-1H-pyrrolo-2-carboxylic acid ethyl ester(63.8 g).
   APCI-MS(m/e):198[M+H]⁺. 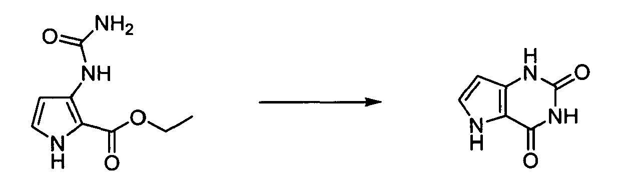
(3) A 6% aqueous sodium hydroxide solution (950 mL) was added to 3-ureido-1H-pyrrolo-2-carboxylic acid ethyl ester(69.4 g) and the mixture was refluxed under heating with stirring for 30 minutes. After standing to cool, the reaction mixture was adjusted to pH 6 by adding conc.hydrochloric acid and the precipitate was filtered after stirring. It was washed with a little amount of water and methanol, dried under reduced pressure and azeotropically distilled with toluene to give 1,5-dihydro-pyrrolo[3,2-d]pyrimidin-2,4-dione(32 g).
   APCI-MS(m/e):152[M+H]⁺. 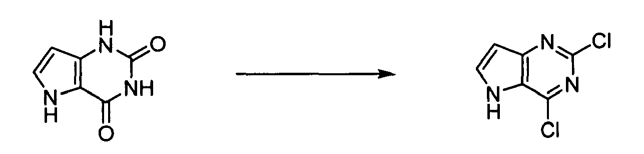
(4) A 1N aqueous sodium hydroxide solution (231 mL) was added to 1,5-dihydro-pyrrolo[3,2-d]pyrimidin-2,4-dione(35.0 g) and the mixture was stirred for a while, then it was concentrated under reduced pressure and the residue was azeotropically distilled after adding toluene. Phenyl phosphonic dichloride(239 g) was slowly added to the residue and the mixture was heated to 180°C and stirred for 3 hours after exothermic reaction was over. Further phenyl phosphonic dichloride(100 g) was slowly added and the mixture was stirred overnight. The reaction solution was poured into ice water slowly with stirring, and it was extracted with ethyl acetate. The organic layer was washed with a sodium bicarbonate solution and dried. The residue was crystallized with ethyl acetate and diisopropyl ether to give 2,4-dichloro-5H-pyrrolo[3,2-d]pyrimidine(19.96 g).
   APCI-MS(m/e):188/190[M+H]⁺ . 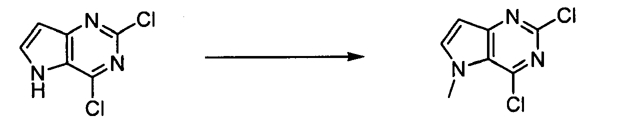
(5) 60% Sodium hydride(5.08 g) was added to a solution of 2,4-dichloro-5H-pyrrolo[3,2-d]pyrimidine(19.9 g) in acetonitrile(530 mL) under ice-cooling and the mixture was stirred at room temperature for 30 minutes. Methyl iodide(18.03 g) was added dropwise to the reaction solution at 8°C, and the mixture was stirred at room temperature overnight. The insoluble material was filtered through Celite and the filtrate was concentrated. The residue was washed with diisopropyl ether to give 2,4-dichloro-5-methyl-5H-pyrrolo[3,2-d]pyrimidine(11.0 g).
   APCI-MS(m/e):202/204[M+H]⁺. 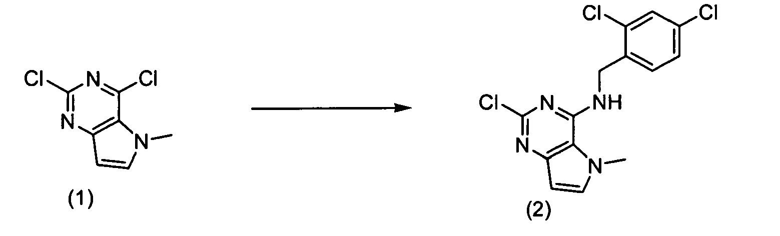
(6) (2-Chloro-5-methyl-5H-pyrrolo[3,2-d]pyrimidin-4-yl)-(2,4-dichlorobenzyl)-amine(2)(20.6 g) was obtained by reacting and treating Compound(1) in the same manner as Example 1(3).
   APCI-MS(m/e):341/343[M+H]⁺. 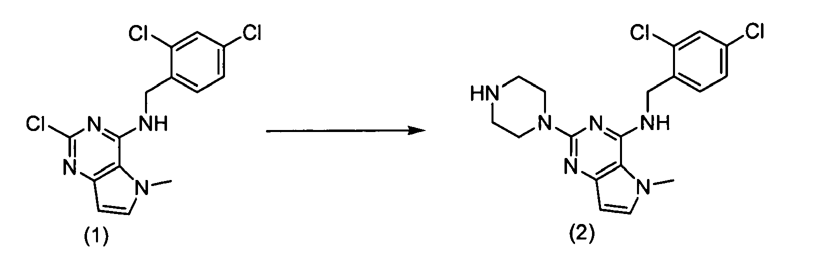
(7) Compound(2) was obtained by reacting and treating Compound(1) in the same manner as Example 1(4).
   APCI-MS(m/e):391/393[M+H]⁺. 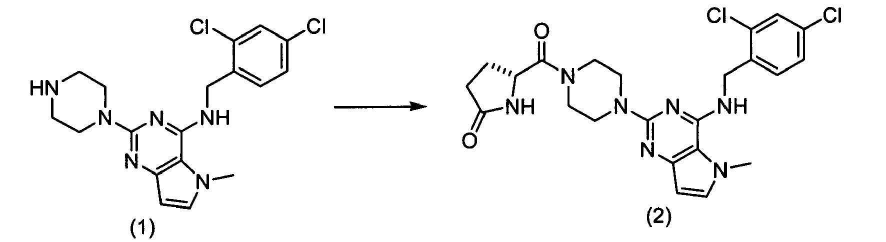
(8) Compound(2) was obtained by reacting and treating Compound(1) in the same manner as Example 22.
   APCI-MS(m/e):502/504[M+H]⁺.

### Example 306

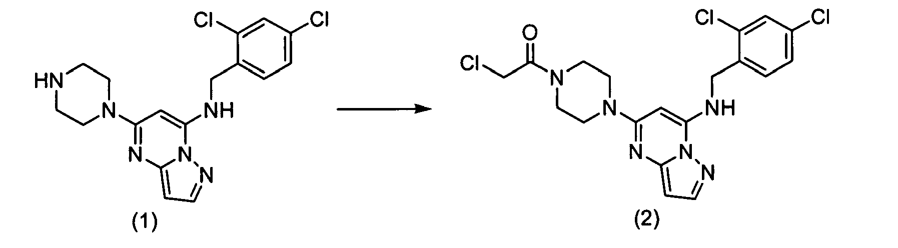

(1) Chloroacetyl chloride(224 mg) was added dropwise to a suspension of Compound(1)(679 mg) and triethylamine(276 µl) in THF(20 mL) under ice-cooling, and the mixture was stirred for an hour. Chloroform was added to the reaction solution, the mixture was washed with water, dried and the solvent was distilled away. The residue was triturated with diethyl ether and dried to give Compound(2)(686 mg) as a pink powder.
   APCI-MS(m/e):453/455[M+H]⁺. 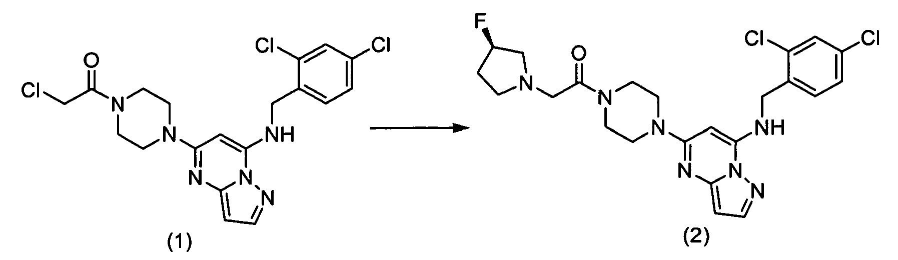
(2) Compound(1)(100 mg), (R)-3-fluoropyrrolidine hydrochloride(33 mg) and potassium carbonate powder(76 mg) were added to a solution of acetonitrile(2 mL) and 1,4-dioxane(2 mL), and the mixture was stirred at room temperature for 1.5 hours. Ethyl acetate was added to the reaction solution, the mixture was washed with brine, dried and the solvent was distilled away. The residue was purified with a silica gel column chromatography(chloroform/methanol=100/0 to 95/5) to give Compound(2)(107 mg) as a colorless powder.
   APCI-MS(m/e):506/508[M+H]⁺.

### Example 307

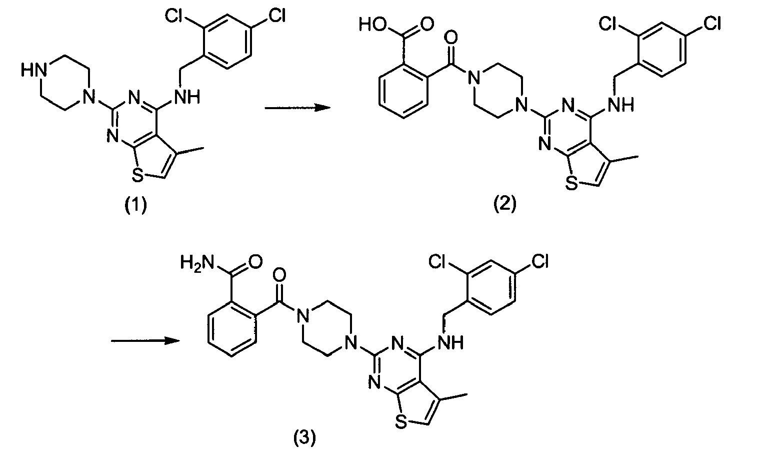

Phthalic anhydride(72.5 mg) and triethylamine(70 µl) were added to a solution of Compound(1)(100 mg) in methylenechloride(5 mL) and DMF(0.5 mL) and the mixture was stirred at room temperature overnight. The reaction solution was concentrated under reduced pressure, and chloroform was added to the residue, the mixture was washed with a saturated aqueous sodium bicarbonate solution, dried and the solvent was distilled away. The residue was purified with a silica gel column chromatography(chloroform/methanol=98/2 to 90/10) to give Compound(2).

Compound(2) was dissolved in DMF, and 28% ammonia water(1.61 ml), benzenetriazol-1-iloxy-tris(dimethylamino)phosphonium hexafluorophosphonate(426 mg) and triethylamine(135 µl) were added successively and the mixture was stirred overnight. Ethyl acetate was added to the reaction solution, the mixture was washed with a saturated aqueous sodium bicarbonate solution, dried and the solvent was distilled away. The residue was purified with a silica gel column chromatography(chloroform/methanol=100/0 to 90/10), solidified from hexane/ethyl acetate and dried to give Compound(3)(40 mg) as a colorless powder.
APCI-MS(m/e):555/557[M+H]⁺ .

### Example 308-325

The following compounds were obtained by the reaction and treatment in the same manner as the aforementioned examples.

**[Table 37]**

| | | | | | | | |
|---|---|---|---|---|---|---|---|
| 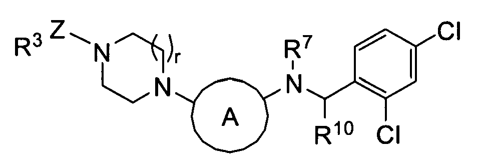 | | | | | | | |

| Example | R³ | Z | r | Ring A | R⁷ | R¹⁰ | MS([M+H]⁺) |
|---|---|---|---|---|---|---|---|
| 308 (2HCl) | 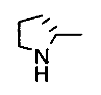 | -CO- | 2 | 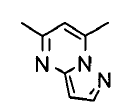 | H | H | 488/490, APCI |
| 309 | 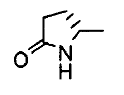 | -CO- | 1 | 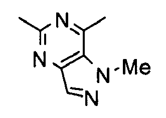 | H | H | 503/505, APCI |
| 310 | 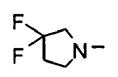 | -CH₂CO- | 1 | 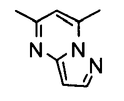 | H | H | 524/526, APCI |
| 311 | 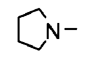 | -CO- | 1 | 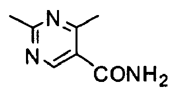 | H | H | 478/480, APCI |
| 312 | 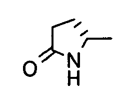 | -CO- | 1 | 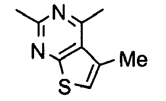 | H | Me (R isomer) | 533/535, APCI |
| 313 | 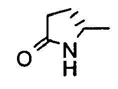 | -CO- | 1 | 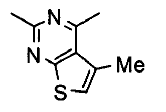 | Me | H | 533/535, APCI |

**[Table 38]**

| | | | | |
|---|---|---|---|---|
| 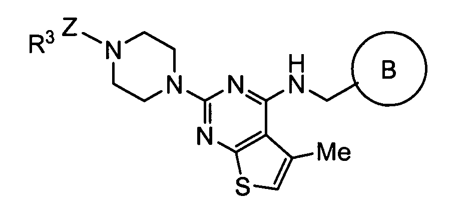 | | | | |

| Example | R³ | Z | Ring B | MS([M+H]⁺) |
|---|---|---|---|---|
| 314 | 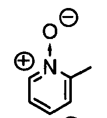 | -CO⁻ | 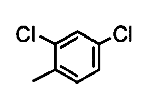 | 529/531, APCI |
| 315 | 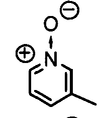 | -CO⁻ | 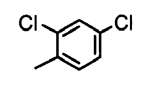 | 529/532, APCI |
| 316 | 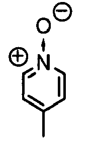 | -CO⁻ | 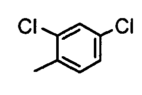 | 529/533, APCI |
| 317 | 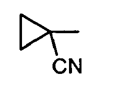 | -CO⁻ | 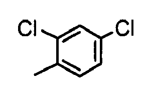 | 501/503, APCI |

**[Table 39]**

| | | |
|---|---|---|
| 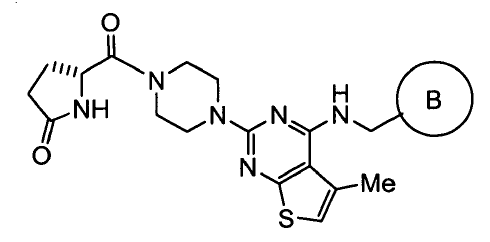 | | |

| Example | Ring B | MS([M+H]⁺) |
|---|---|---|
| 318 | 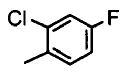 | 503/505, APCI |
| 319 | 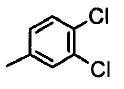 | 519/521, APCI |
| 320 | 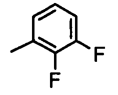 | 487, APCI |
| 321 | 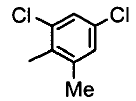 | 533/535, APCI |
| 322 | 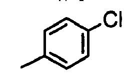 | 485/487, APCI |
| 323 | 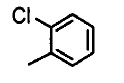 | 485/487, APCI |
| 324 | 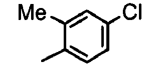 | 499/501, APCI |
| 325 | 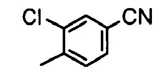 | 510/512, APCI |

### Example 326

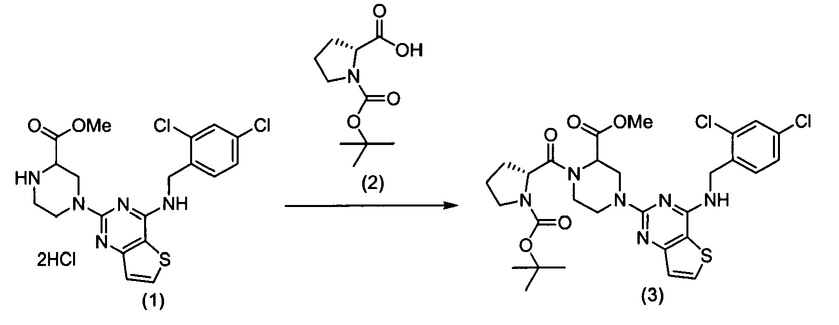

(1) Diethyl phosphorocyanidate(276 mg) and triethylamine(471 µL) were added successively to a solution of Compound(1)(355 mg) and N-(tert-butoxycarbonyl)-(R)-proline(2)(364 mg) in DMF(3.4 mL) under ice-cooling and the mixture was stirred at room temperature overnight. Ethyl acetate was added to the reaction solution, the mixture was washed with a saturated aqueous sodium bicarbonate solution and water, dried and the solvent was distilled away. The residue was purified with a silica gel column chromatography(chloroform) and crystallized from diethyl ether/hexane to give Compound(3)(267 mg) as a colorless powder.
   APCI-MS(m/e):649/645[M+H]⁺. 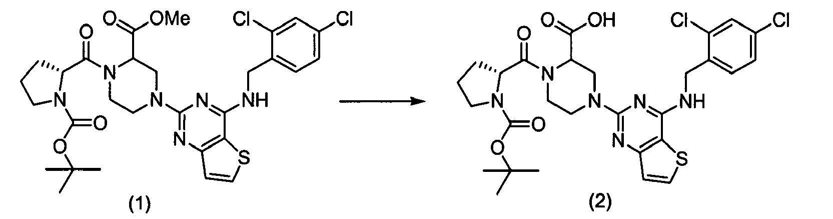
(2) Compound(2) was obtained by reacting and treating Compound(1) in the same manner as Example 41 (2) except the reaction condition is at 60°C.
   APCI-MS(m/e):635/637[M+H]⁺. 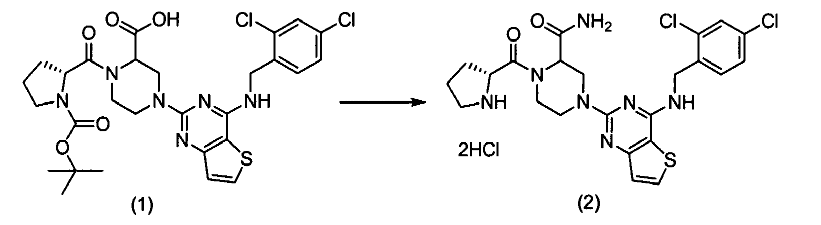
(3) Compound(2) was obtained by reacting and treating Compound(1) in the same manner as Reference Example 32(5) and then by reacting and treating it in the same manner as Reference Example 32(6)
   APCI-MS(m/e):534/536[M+H]⁺.

The following compounds were obtained by the reaction and treatment in the same manner as the aforementioned examples.

**[Table 40]**

| | | |
|---|---|---|
| 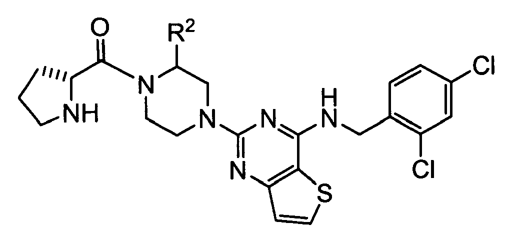 | | |

| Example | R² | MS([M+H]⁺) |
|---|---|---|
| 327 (2HCl) | -CONH-Me | 548/550, APCI |
| 328 (2HCl) | -CONMe₂ | 562/564, APCI |

**[Table 41]**

| | | |
|---|---|---|
| 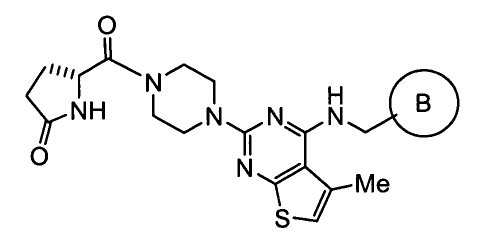 | | |

| Example | Ring B | MS([M+H]⁺) |
|---|---|---|
| 329 | 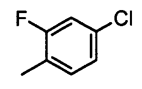 | 503/505, APCI |
| 330 | 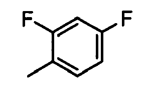 | 487, APCI |
| 331 | 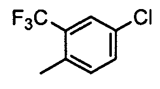 | 553/555, APCI |
| 332 | 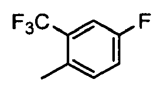 | 537, APCI |
| 333 | 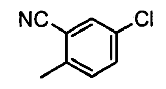 | 510/512, APCI |
| 334 | 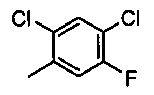 | 537/539, APCI |
| 335 | 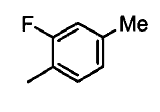 | 483, APCI |

**[Table 42]**

| | | |
|---|---|---|
| 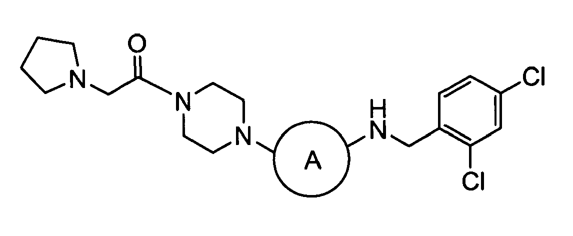 | | |

| Example | Ring A | MS([M+H]⁺) |
|---|---|---|
| 336 | 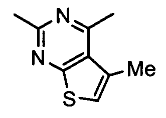 | 519/521, APCI |
| 337 | 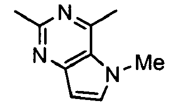 | 502/504, APCI |
| 338 | 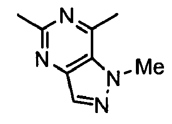 | 503/505, APCI |
| 339 | 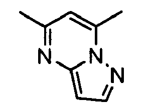 | 488/490, APCI |
| 340 | 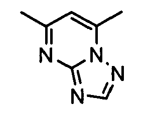 | 489/491, APCI |

**[Table 43]**

| | | | | |
|---|---|---|---|---|
| 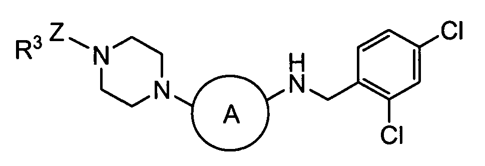 | | | | |

| Example | R³ | Z | Ring A | MS([M+H]⁺) |
|---|---|---|---|---|
| 341 (2HCI) | 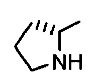 | -CO- | 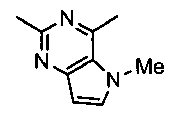 | 488/490, APCI |

**[Table 44]**

| | | | | | |
|---|---|---|---|---|---|
| 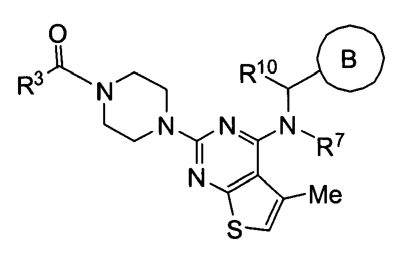 | | | | | |

| Example | R³ | R⁷ | R¹⁰ | Ring B | MS([M+H]⁺) |
|---|---|---|---|---|---|
| 342 | 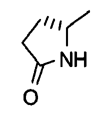 | H | H | 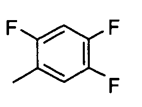 | 505, APCI |
| 343 | 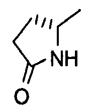 | H | H | 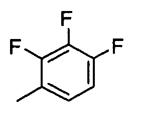 | 505, APCI |
| 344 | 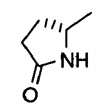 | H | Me (R isomer) | 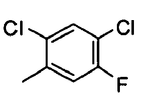 | 551/553, APCI |
| 345 | 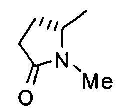 | Me | H | 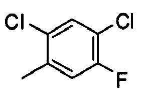 | 565/567, APCI |
| 346 | 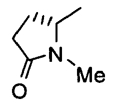 | H | H | 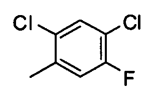 | 551/553, APCI |

### Example 347

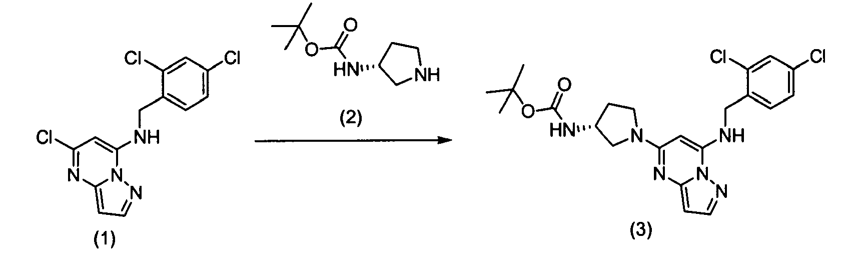

(1) Compound(3) was obtained by reacting and treating Compound(1) and Compound(2) in the same manner as Example 1(4).
   APCI-MS(m/e):477/479[M+H]⁺. 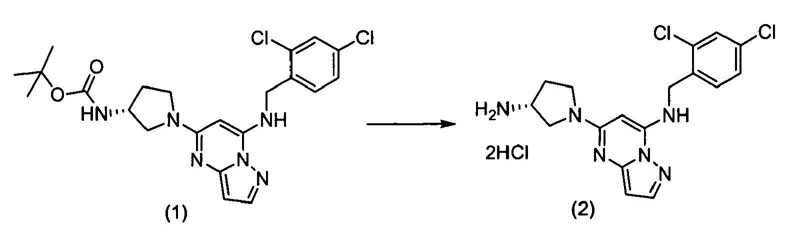
(2) Compound(2) was obtained by reacting and treating Compound(1) in the same manner as Example 1(5).
   APCI-MS(m/e):377/379[M+H]⁺. 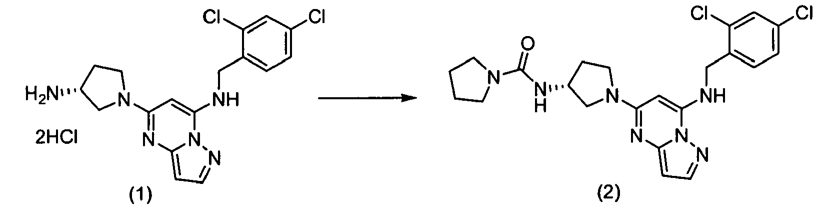
(3) 1-Pyrrolidinecarbonyl chloride(25 µL) and triethylamine(106 µL)were added to a solution of Compound(1)(68 mg) in methylene chloride(1.5 mL) at room temperature and the mixture was stirred at room temperature for 2 hours. THF(1.0 mL) was added to the reaction solution and the mixture was stirred at room temperature overnight, then it was stirred at 50°C for 2 hours. After standing to cool, chloroform was added thereto, and it was washed with a saturated aqueous sodium bicarbonate solution, dried and the solvent was distilled away. The residue was purified with a silica gel column chromatography(chloroform/methanol=100/0 to 95/5) and crystallized from ethyl acetate/diethyl ether to give Compound(2)(52 mg) as a pale orange powder.
   APCI-MS(m/e):474/476[M+H]⁺.

The following compounds were obtained by the reaction and treatment in the same manner as the aforementioned examples.

**[Table 45]**

| | | | | |
|---|---|---|---|---|
| 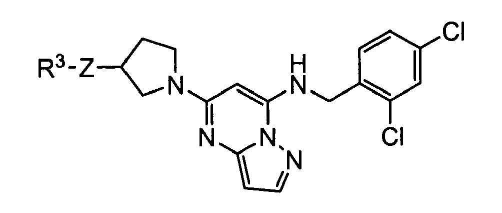 | | | | |

| Example | R³ | Z | Absolute configuration | MS([M+H]⁺) |
|---|---|---|---|---|
| 348 (2HCl) | 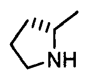 | -CONH- | R | 474/476, APCI |
| 349 | 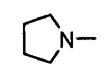 | -CO- | - | 459/461, APCI |
| 350 (2HCl) | 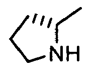 | -CONH- | S | 474/476, APCI |
| 351 | Me₂N- | -CONH- | R | 448/450, APCI |
| 352 | 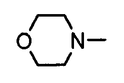 | -CONH- | R | 490/492, APCI |
| 353 | 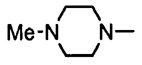 | -CONH- | R | 503/505, APCI |
| 354 | 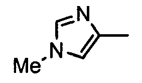 | -SO₂NH⁻ | R | 521/523, APCI |
| 355 | 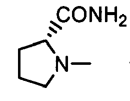 | -CONH- | R | 517/519, APCI |
| 356 | 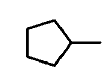 | -CONH- | R | 473/475, APCI |

### Reference Example 1

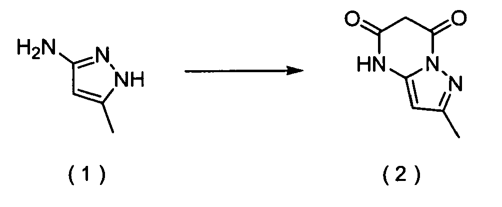

2-Methyl-4H-pyrazolo[1,5-a]-pyrimidin-5,7-dione was obtained by reacting and treating 5-methyl-1H-pyrazol-3-ylamine in the same manner as Example 1(1).
APCI-MS(m/e):166[M+H]⁺.

### Reference Example 2-1

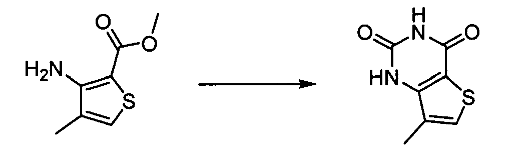

Methyl 3-amino-4-methyl-2-carboxylate(25.0 g) and urea(43.9 g) were stirred at 190°C for 4 hours. After standing to cool, an aqueous sodium hydroxide solution was added and the insoluble materials were filtered. The filtrate was neutralized by adding hydrochloric acid, allowed to stand under ice-cooling. The precipitated crystals were filtered, washed with water and methanol and dried to give 7-methyl-1H-thieno[3,2-d]pyrimidin-2,4-dione(15.9 g) as a pale pink powder.
APCI-MS(m/e): 183[M+H]⁺

### Reference Example 2-2

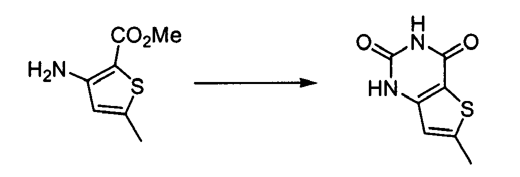

6-Methyl-1H-thieno[3,2-d]pyrimidin-2,4-dione was obtained by reacting and treating 3-amino-5-methyl-thiophen-2-carboxylic acid methyl ester in the same manner as Reference Example 2-1.
APCI-MS(m/e):183[M+H]⁺.

### Reference Example 3

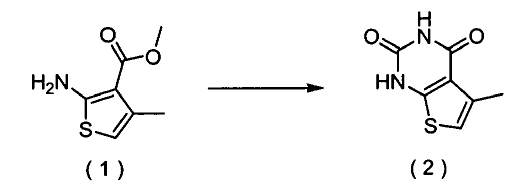

Compound(2) was obtained by reacting and treating Compound(1) in the same manner as Reference Example 2-1.
APCI-MS(m/e):183[M+H]⁺.

### Reference Example 4

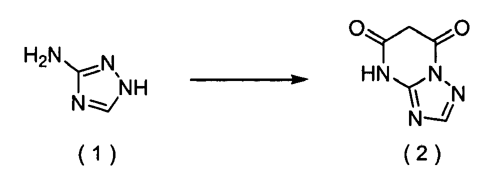

Compound(2) was obtained by reacting and treating Compound(1) in the same manner as Example 1(1).
APCI-MS(m/e):151[M-H]⁻.

### Reference Example 5-1

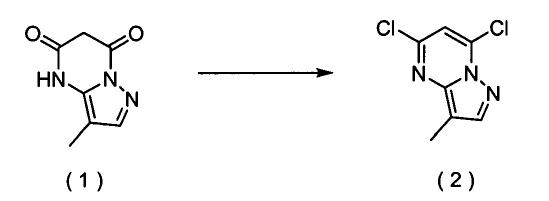

Compound(2) was obtained by reacting and treating Compound(1) in the same manner as Example 1(2).
APCI-MS(m/e):202/204[M+H]⁺.

### Reference Example 5-2

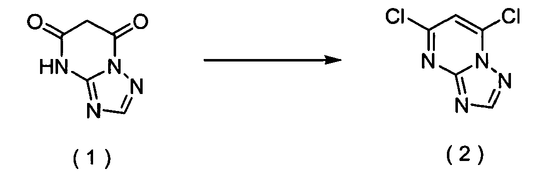

Compound(2) was obtained by reacting and treating Compound(1) in the same manner as Example 1(2).
APCI-MS(m/e):189/191[M+H]⁺.

### Reference Example 5-3

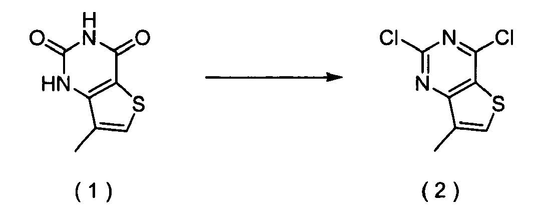

Compound(2) was obtained by reacting and treating Compound(1) in the same manner as Example 1(2).
APCI-MS(m/e):219/221 [M+H]⁺.

### Reference Example 6-1

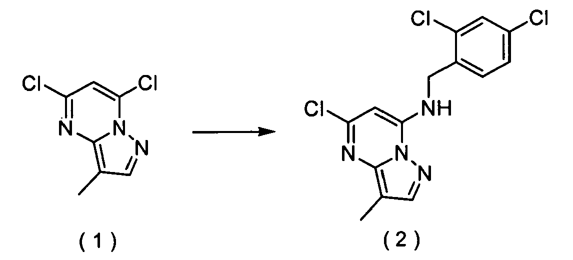

Compound(2) was obtained by reacting and treating Compound(1) in the same manner as Example 1(3).
APCI-MS(m/e):341/343[M+H]⁺.

### Reference Example 6-2

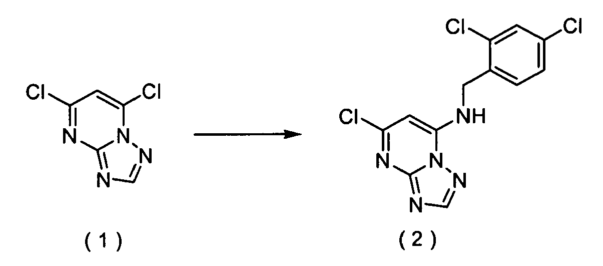

Compound(2) was obtained by reacting and treating Compound(1) in the same manner as Example 1(3).
APCI-MS(m/e):328/330[M+H]⁺.

### Reference Example 6-3

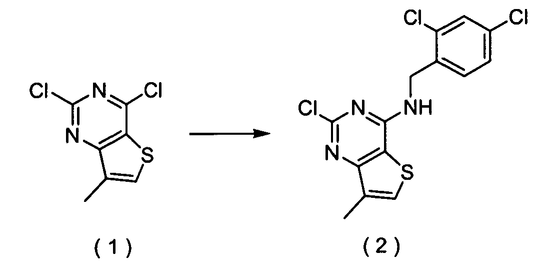

Compound(2) was obtained by reacting and treating Compound(1) in the same manner as Example 1(3).
APCI-MS(m/e):358/360[M+H]⁺.

### Reference Example 6-4

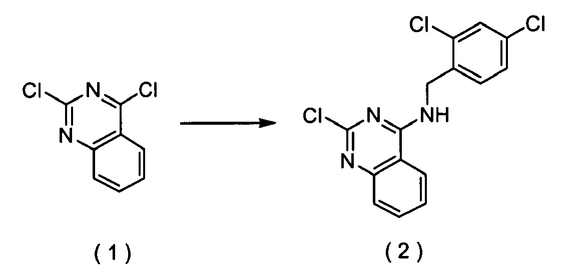

Compound(2) was obtained by reacting and treating Compound(1) in the same manner as Example 1(3).
APCI-MS(m/e):338/340[M+H]⁺.

### Reference Example 6-5

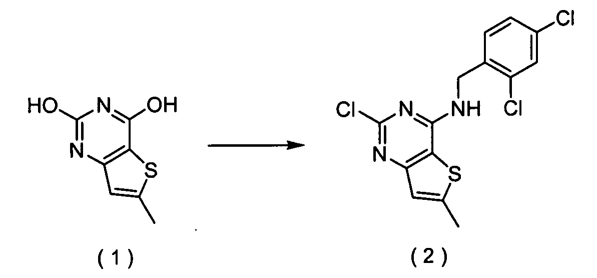

Compound(2) was obtained by reacting and treating Compound(1) in the same manner as Example 1(2) and 1(3).
APCI-MS(m/e):358/360[M+H]⁺.

### Reference Example 6-6

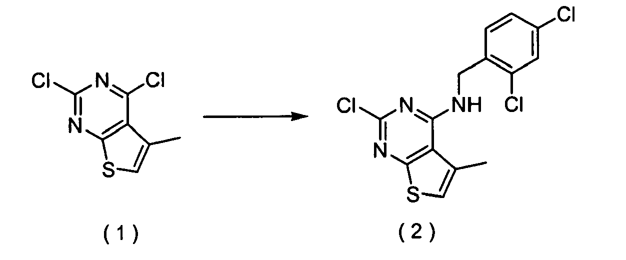

Compound(2) was obtained by reacting and treating Compound(1) in the same manner as Example 1(3).
APCI-MS(m/e):358/360[M+H]⁺.

### Reference Example 6-7

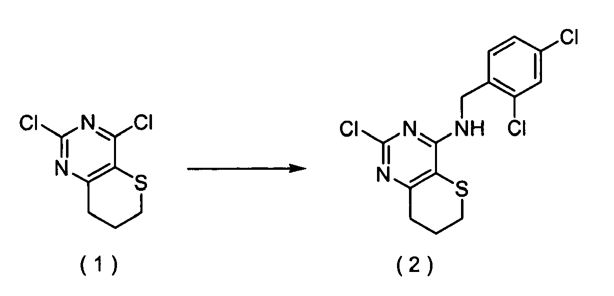

Compound(2) was obtained by reacting and treating Compound(1) in the same manner as Example 1(3).
APCI-MS(m/e):360/362[M+H]⁺.

### Reference Example 7

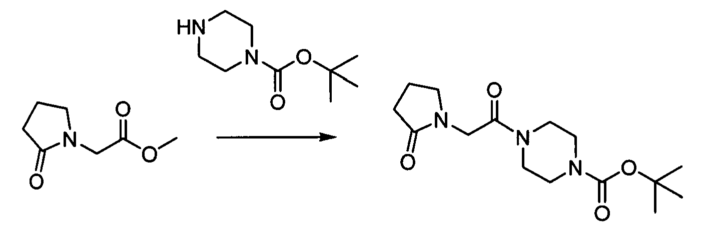

(1) A mixture of (2-oxo-1-pyrrolidin-1-yl)-acetic acid methyl ester(1.42 g) and piperazine-1-carboxylic acid tert-butyl ester(1.68 g) was irradiated at 150°C for 2 hours by using a microwave reaction apparatus. The resulting mixture was purified with a silica gel column chromatography(chloroform/methanol=100/0 to 90/10), and with a NH silica gel column chromatography(hexane/ethyl acetate=50/50 to 0/100) again to give 4-[2-(2-oxo-pyrrolidin-1-yl)acetyl]-piperazine-1-carboxylic acid tert-butyl ester(1.15 g) as an oily substance.
   APCI-MS(m/e):312[M+H]⁺. 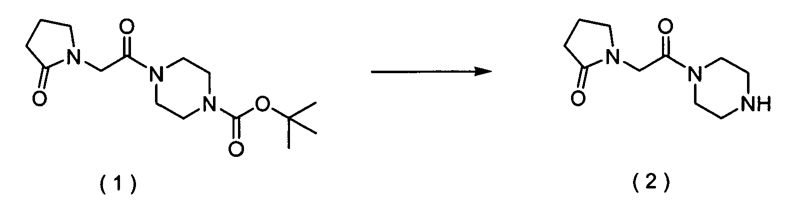
(2) Compound(2) was obtained by reacting and treating Compound(1) in the same manner as Example 1(5).
   APCI-MS(m/e):212[M+H]⁺.

### Reference Example 8

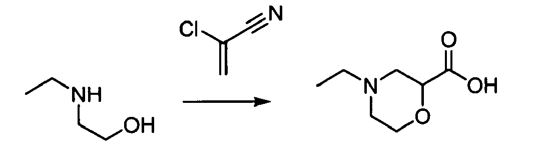

(1) 2-Chloroacrylonitrile(22.4 mL) was slowly added to a solution of N-ethyl-ethanolamine(24.96 g) in toluene(85 mL) at room temperature and the mixture was stirred overnight. To the reaction solution was added slowly a solution of potassium tert-butoxide(31.44 g) in toluene(200 mL)/THF(160 mL) and the mixture was further stirred for an hour. The reaction solution was distilled away under reduced pressure, diethyl ether was added thereto and the precipitated solid was filtered. The filtrate was concentrated, toluene(235 mL) and 6N hydrochloric acid(380 mL) were added successively and the mixture was refluxed under heating with stirring for 2 hours and then stirred at room temperature overnight. The solvent was distilled away and the residue was dried to give a crude product of 4-ethyl-morpholine-2-carboxylic acid, which was used in the next step without further purification.
   APCI-MS(m/e):160[M+H]⁺. 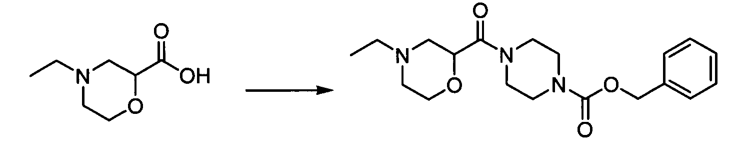
(2) Thionyl chloride was added to the crude product of 4-ethyl-morpholine-2-carboxylic acid and the mixture was refluxed under heating with stirring for 4 hours. The solvent was distilled away and the residue was azeotropically distilled with toluene twice. The residue was dissolved in methylene chloride(100 mL), benzyloxycarbonyl piperazine(59.2 g) and triethylamine(195 mL) were added under ice-cooling and the mixture was stirred at room temperature overnight. After the solvent was distilled away, di-tert-butyl carbonate(30.7 g) and ethyl acetate(120 mL) were added and the mixture was stirred at room temperature for 4 hours. An aqueous citric acid solution was added, and the mixture was extracted with ethyl acetate, dried and the solvent was distilled away. The residue was purified with a silica gel column chromatography(hexane/ethyl acetate=60/40 to 40/60) to give 4-(4-ethylmorpholine-2-carbonyl)-piperazine-1-carboxylic acid benzyl ester(3.69 g) as an orange oily substance.
   APCI-MS(m/e):362[M+H]⁺. 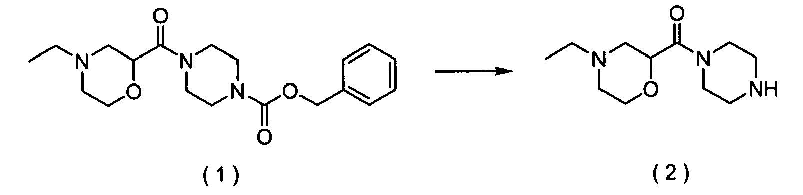
(3) Compound(2) was obtained by reacting and treating Compound(1) in the same manner as Reference Example 9(2).
   APCI-MS(m/e):228[M+H]⁺.

### Reference Example 9

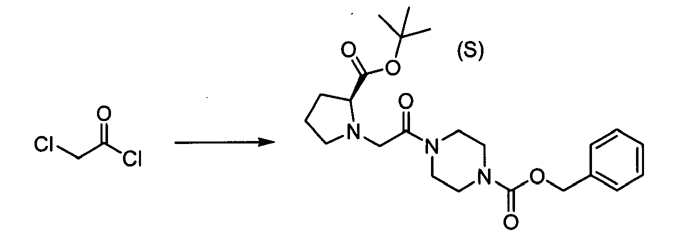

(1) A solution of benzyloxycarbonyl piperazine(2.49 g) and triethylamine(1.58 mL) in THF(20 mL) was added dropwise to a solution of chloroacetyl chloride(1.28 g) in THF(40 mL) at 0°C and the mixture was stirred for 15 minutes. A solution of pyrrolidin-2(R)-carboxylic acid tert-butyl ester(4.84 g) and triethylamine(1.58 mL) in THF(5 mL) was added to the reaction solution, and the mixture was stirred at room temperature for 3 days and at 50-65°C for a day. Ethyl acetate was added to the reaction solution and the solution was washed with water and brine, dried over magnesium sulfate and the solvent was distilled away. The residue was purified with a NH silica gel column chromatography(hexane/ethyl acetate=4/1 to 2/1) to give 4-[2-(2S-tert-butoxycarbonyl-pyrrolidin-1-yl)acetyl]-piperazine-1-carboxylic acid benzyl ester(4.32 g) as a pale yellow oily substance.
   APCI-MS(m/e):432[M+H]⁺. 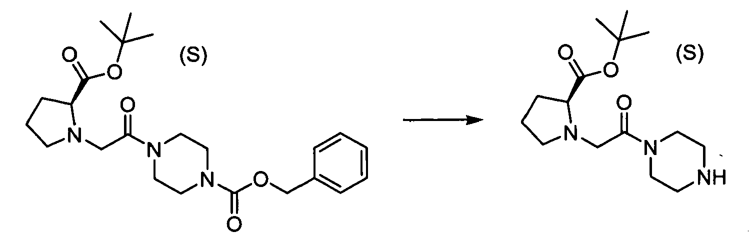
(2) 10% Palladium/carbon(1.0 g) was added to a solution of 4-[2-(2(S)-tert-butoxycarbonyl-pyrrolidin-1-yl)acetyl]-piperazine-1-carboxylic acid benzyl ester(4.32 g) in methanol, and the mixture was stirred at room temperature under hydrogen atmosphere overnight. The reaction solution was filtered through Celite and the filtrate was concentrated to give 1-(2-oxo-2-piperazin-1-yl-ethyl)-pyrrolidine-2(S)-carboxylic acid tert-butyl ester(3.09 g) as an oily substance.
   APCI-MS(m/e):298[M+H]⁺.

### Reference Example 10-1

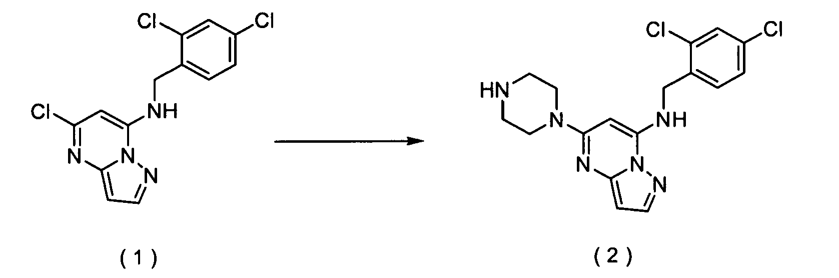

Compound(2) was obtained by reacting and treating Compound(1) in the same manner as Example 1(4).
APCI-MS(m/e):377/379[M+H]⁺.

### Reference Example 10-2

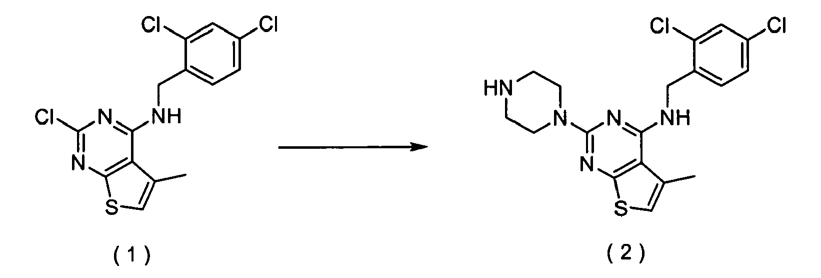

Compound(2) was obtained by reacting and treating Compound(1) in the same manner as Example 1(4).
APCI-MS(m/e):408/410[M+H]⁺.

### Reference Example 10-3

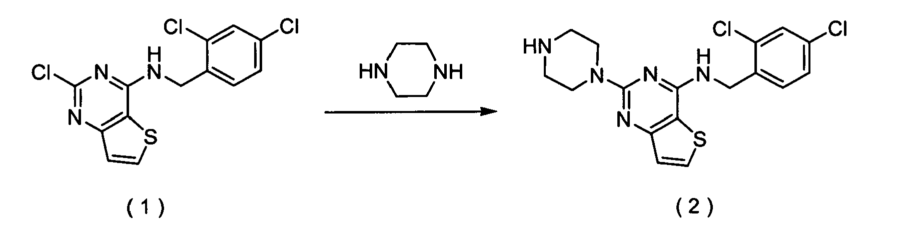

Compound(2) was obtained by reacting and treating Compound(1) in the same manner as Example 1(4).
APCI-MS(m/e):394/396[M+H]⁺.

### Reference Example 11

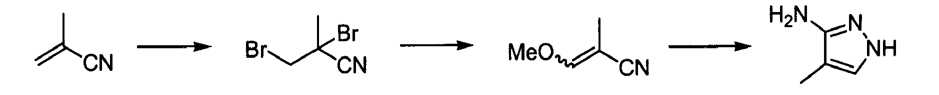

(1) Bromine was slowly added dropwise to methyl acrylonitrile(40 g) at 45°C for 2 hours and the mixture was stirred for 2 hours. The reaction solution was added slowly for 30 minutes to a solution of potassium hydroxide(67.4 g) in methanol(500 mL) at 0°C. Two hours later, the temperature of the reaction solution was raised to room temperature and the reaction solution was stirred for a day. The reaction solution was concentrated under reduced pressure, the residue was dissolved in water and extracted with diethyl ether twice. The combined organic layer was washed with brine, dried over magnesium sulfate, filtered and the solvent was distilled away. Ethanol(50 mL) and hydrazine hydrate(35.8 g) were added to the residue and the mixture was stirred at 80°C for a day. Hydrazine(10 g) was further added to the reaction solution and it was stirred at 80°C for a day. After the reaction solution was concentrated under reduced pressure, the residue was dissolved in chloroform/methanol(9/1), a silica gel was added thereto and allowed to stand for a while. The mixture was filtered and the filtrate was concentrated under reduced pressure to give 4-methyl-1H-pyrazol-3-ylamine(46.7 g) as a dark purple oily substance
   APCI-MS(m/e):98[M+H]⁺.

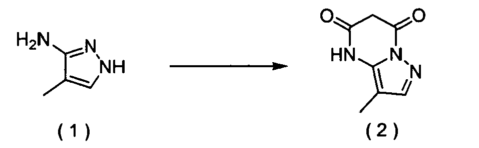

Compound(2) was obtained by reacting and treating Compound(1) in the same manner as Example 1(1).
APCI-MS(m/e):166[M+H]⁺.

### Reference Example 12

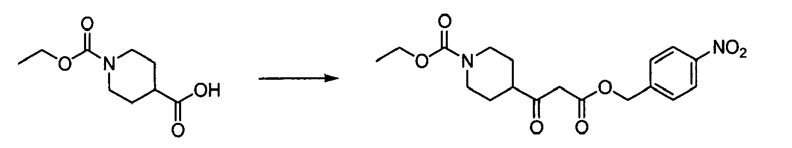

(1) N,N'-Carbonyldiimidazole(9.9 g) was added to a solution of N-ethoxycarbonyl isonipecotic acid(4.43 g) in THF(50 mL) under ice-cooling, and the mixture was stirred at room temperature for an hour. Magnesium (4-nitrobenzyl) malonate(12.0 g) and THF(30 mL) were added thereto and the mixture was stirred at 50°C for 10 hours. The reaction solution was concentrated, diethyl ether was added to the residue and the mixture was washed with hydrochloric acid, water, a saturated aqueous sodium bicarbonate solution and brine, dried over sodium sulfate and the solvent was distilled away. The residue was purified with a silica gel column chromatography(hexane/ethyl acetate=100/0 to 65/35) and recrystallized from ethyl acetate/hexane to give 4-[2-(4-nitro-benzyloxycarbonyl)-acetyl]-piperidine-1-carboxylic acic ethyl ester(7.82 g) as a colorless powder.
   APCI-MS(m/e):379[M+H]⁺. 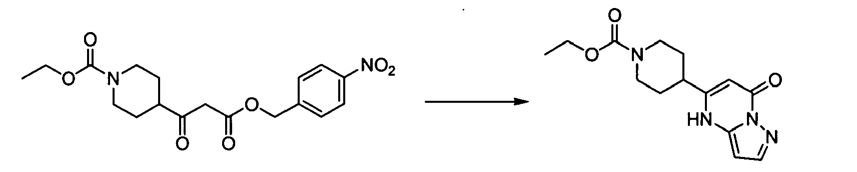
(2) 4-[2-(4-Nitro-benzyloxycarbonyl)-acetyl]-piperidin-1-carboxylic acid ethyl ester(10.3 g) and 3-amino-pyrazole(2.2 g) were dissolved in acetic acid(15 mL) and the mixture was stirred at 95°C overnight. The reaction solution was concentrated under reduced pressure, ethyl acetate was added to the residue and precipitated crystals were filtered, washed with ethyl acetate and dried to give 4-(7-oxo-4,7-dihydro-pyrazolo[1,5-a]pyrimidin-5-yl)-piperidine-1-carboxylic acic ethyl ester(6.9 g) as pale brown crystals.
   APCI-MS(m/e):291[M+H]⁺. 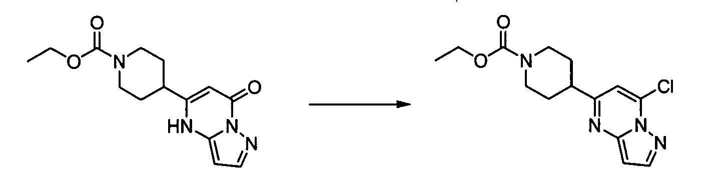
(3) Diethyl aniline(1.2 mL) and phosphorous oxychloride(3.0 g) were successively added to 4-(7-oxo-4,7-dihydro-pyrazolo[1,5-a]pyrimidin-5-yl)-piperidine-1-carboxylic acid ethyl ester(500 mg) and the mixture was stirred at 50°C for 10 minutes. The reaction solution was dissolved in chloroform and the mixture was washed with a saturated aqueous sodium bicarbonate solution and brine, dried and the solvent was distilled away. The residue was purified with a silica gel column chromatography(hexane/ethyl acetate=4/1 to 2/1) to give 4-(7-chloro-pyrazolo[1,5-a]pyrimidin-5-yl)-piperidine-1-carboxylic acid ethyl ester(501 mg) as a yellow oily substance.
   APCI-MS(m/e):309/311 [M+H]⁺. 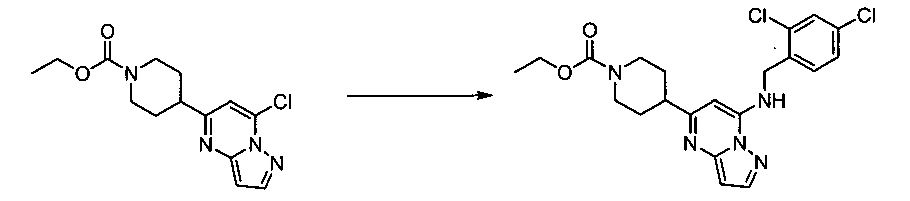
(4) Diisopropylethylamine(0.28 mL) was added to a solution of 4-(7-chloro-pyrazolo[1,5-a]pyrimidin-5-yl)-piperidine-1-carboxylic acid ethyl ester(490 mg) and 2,4-dichlorobenzylamine(419 mg) in 1,4-dioxane(5 mL) and the mixture was stirred at 90°C for 9 hours. After standing to cool, chloroform was added to the reaction solution and the mixture was washed with a saturated aqueous sodium bicarbonate solution and brine, dried and the solvent was distilled away. The residue was purified with a NH silica gel column chromatography(hexane/ethyl acetate/chloroform=1/5/5 to 3/10/10) to give 4-[7-(2,4-dichlorobenzylamino)-pyrazolo[1,5-a]pyrimidin-5-yl]-piperidine-1-carboxylic acid ethyl ester(0.75 g) as a pale brown oily substance.
   APCI-MS(m/e):448/450[M+H]⁺. 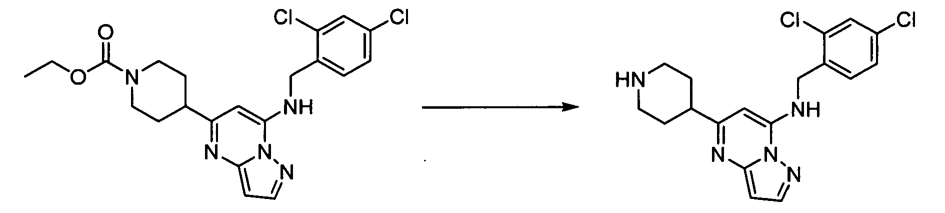
(5) Potassium hydroxide(13.0 g) was added to a solution of 4-[7-(2,4-dichlorobenzylamino)-pyrazolo[1,5-a]pyrimidin-5-yl]-piperidine-1-carboxylic acid ethyl ester(10.4 g) in 2-propanol(150 mL) and the mixture was refluxed under heating with stirring overnight. The reaction solution was cooled, an aqueous ammonium chloride solution was added and the mixture was extracted with chloroform. The organic layer was washed with brine, dried over sodium sulfate and the solvent was distilled away. The residue was crystallized from ethyl acetate to give (2,4-dichlorobenzyl)-(5-piperidin-4-yl-pyrazolo[1,5-a]pyrimidin-7-yl)-amine(7.08 g) as colorless crystals.
   APCI-MS(m/e):376/378[M+H]⁺.

### Reference Example 13

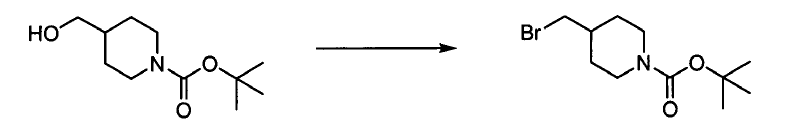

(1) Triphenylphosphine(7.42 g) and carbon tetrabromide(11.69 g) were added to a solution of 4-hydroxymethyl-piperidine-1-carboxylic acid tert-butyl ester(5.05 g) in methylene chloride(50 ml), which is prepared from 4-hydroxymethyl-piperidine-1-carboxylic acid in a conventional method, and the mixture was stirred at room temperature for 30 minutes. A saturated aqueous sodium bicarbonate solution was added to the reaction solution, the mixture was extracted with chloroform three times. The organic layer was dried over magnesium sulfate and the solvent was distilled away. The residue was purified with a silica gel column chromatography(hexane/ethyl acetate=100/0 to 90/10) to give 4-bromomethyl-piperidine-1-carboxylic acid tert-butyl ester(7.25 g) as a colorless oily substance.
   APCI-MS(m/e):178/180[M+H]⁺. 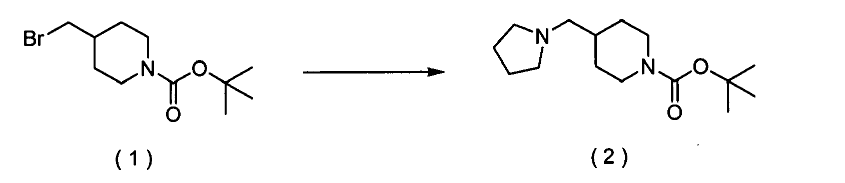
(2) Compound(2) was obtained by reacting and treating Compound(1) in the same manner as Example 1(4).
   APCI-MS(m/e):269[M+H]⁺
   Reference Example 13(4):
   4-Pyrrolidin-1-ylmethyl-piperidine dihydrochloride 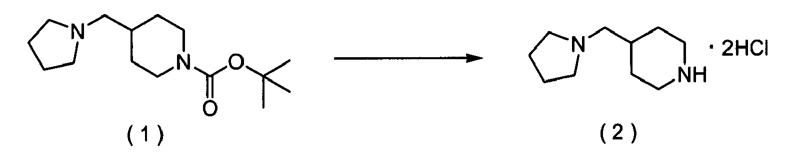
(3) Compound(2) was obtained by reacting and treating Compound(1) in the same manner as Example 1(5).
   APCI-MS(m/e):169[M+H]⁺.

### Reference Example 14

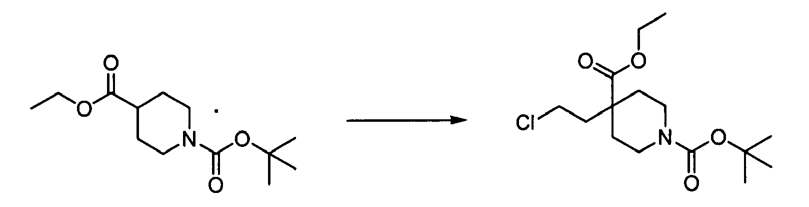

(1) n-Butyl lithium(1.7M hexane solution) was added to a solution of diisopropylamine(4.1 mL) in THF(4.1 mL) at -78°C under nitrogen atmosphere and the mixture was stirred for 30 minutes. A solution of N-tert-butyloxycarbonyl-isonipecotinic acid ethyl ester(5.01 g) in THF(15 mL) was added dropwise to the reaction solution and the mixture was stirred for 30 minutes. Further chlorobromoethane(4.8 mL) was added and the mixture was stirred at -30 to -20°C for 5 hours. A saturated aqueous sodium bicarbonate solution was added to the reaction solution under ice-cooling and the mixture was extracted with ethyl acetate three times. The combined organic solution was dried over magnesium sulfate and the solvent was distilled away. The residue was purified with a silica gel column chromatography(hexane/ethyl acetate= 90/10 to 80/20) to give 4-(2-chloroethyl)-piperidine-1,4-dicarboxylic acid 1-tert-butyl ester-4-ethyl ester(2.19 g) as a pale yellow oily substance.
   APCI-MS(m/e):220/222(M+2H-Boc)⁺. 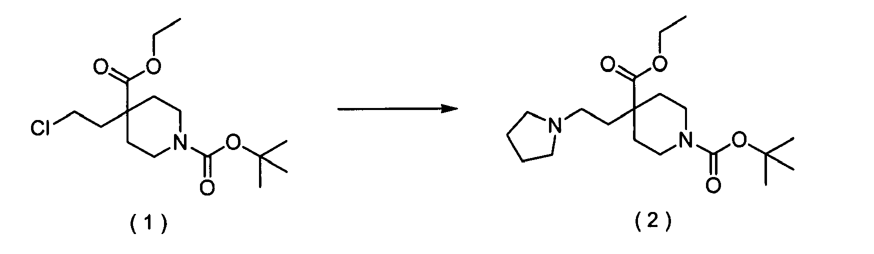
(2) Compound(2) was obtained by reacting and treating Compound(1) in the same manner as Example 1(4).
   APCI-MS(m/e):355[M+H]⁺. 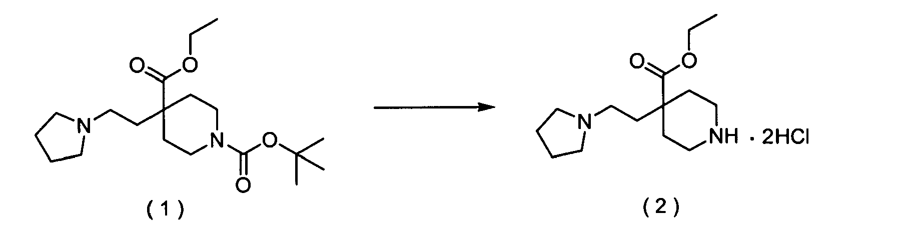
(3) Compound(2) was obtained by reacting and treating Compound(1) in the same manner as Example 1(5).
   APCI-MS(m/e):255[M+H]⁺.

### Reference Example 15

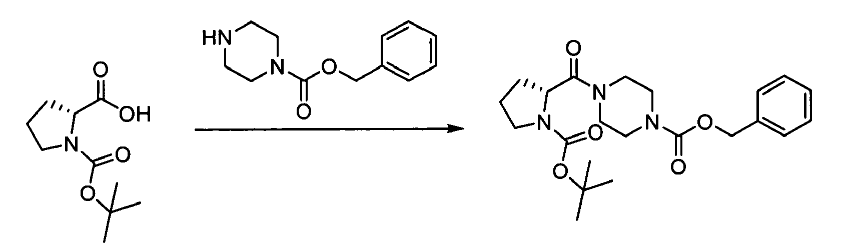

(1) 1-(3-dimethylaminopropyl)-3-ethylcarbodiimide hydrochloride(10.7 g) and 1-hydroxybenzotriazole hydrate(8.5 g) were added to a solution of N-(1-tert-butoxycarbonyl)-(R)-proline(10 g) in DMF(200 mL) and the mixture was stirred at room temperature for an hour. Piperazine-1-carboxylic acid benzyl ester(12.3 g) was added to the reaction solution and the mixture was stirred at room temperature overnight. Water and ethyl acetate were added to the reaction solution and the organic layer was separated, washed with water, dried and the solvent was distilled away. The residue was purified with a silica gel column chromatography(hexane/ethyl acetate/methanol= 2/1/0 to 0/1/0 to 0/100/1) and diethyl ether was added to the residue. The precipitate was filtered, washed with hexane and dried to give the objective compound(4.02 g, yield 21%) as a powder.
   APCI-MS(m/e):418[M+H]⁺. 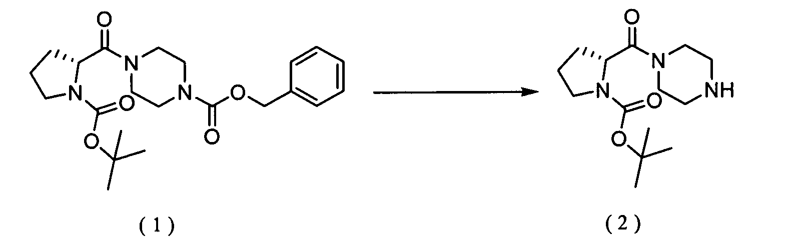
(2) Compound(2) was obtained by reacting and treating Compound(1) in the same manner as Reference Example 9(2).
   APCI-MS(m/e):284[M+H]⁺.

### Reference Example 16

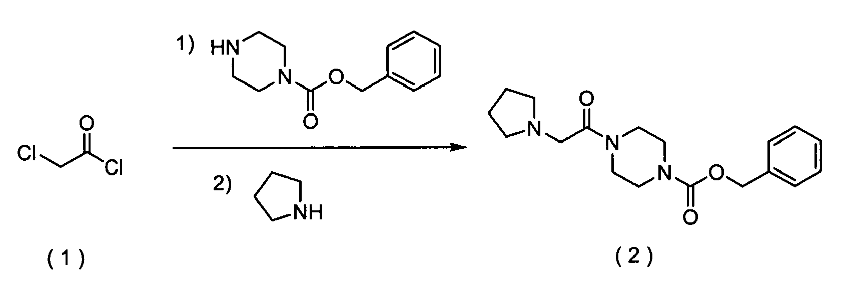

(1) Compound(2) was obtained by reacting and treating Compound(1) in the same manner as Reference Example 9(1).
   APCI-MS(m/e):332[M+H]⁺. 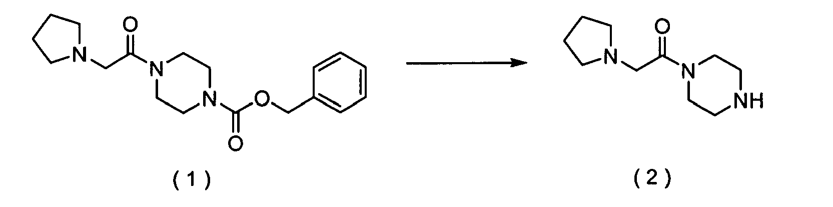
(2) Compound(2) was obtained by reacting and treating Compound(1) in the same manner as Reference Example 9(2).
   APCI-MS(m/e):198[M+H]⁺.

### Reference Example 17

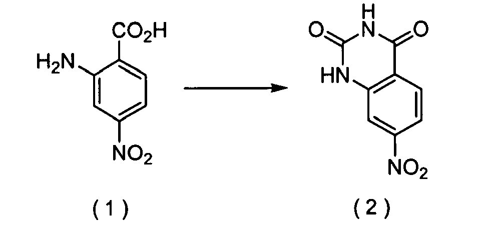

(1) Compound(2) was obtained by reacting and treating Compound(1) in the same manner as Reference Example 2(1).
   APCI-MS(m/e):206[M-H]⁻. 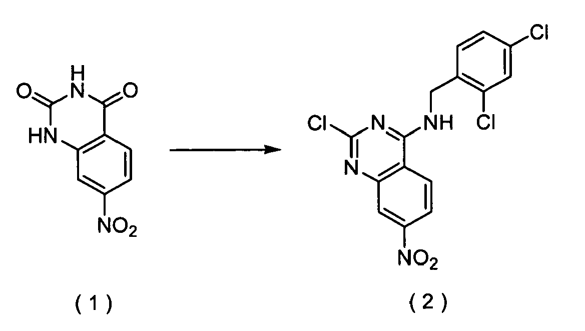
(2) Compound(2) was obtained by reacting and treating Compound(1) in the same manner as Examples 1(2) and (3).
   APCI-MS(m/e):383/385[M+H]⁺.

### Reference Example 18

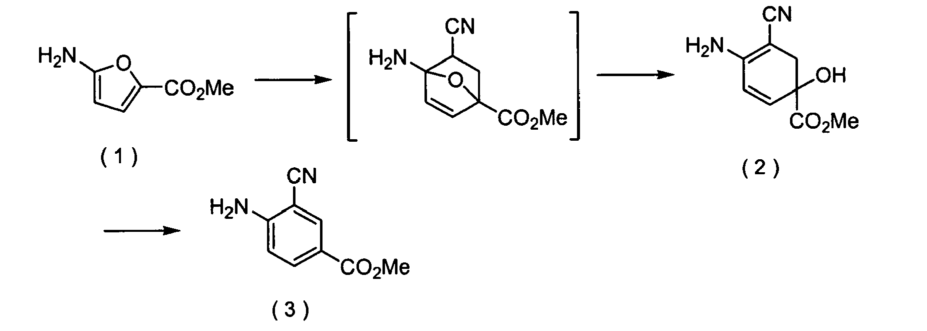

(1) Acrylonitrile(11.4 mL) was added to a solution of 5-aminofuran-2-carboxylic acid methyl ester(12.26 g) in toluene(100 mL) and the mixture was stirred under reflux overnight. The reaction solution was concentrated and diethyl ether was added to the residue. The precipitate was filtered and dried to give Compound(2)(14.87 g) as a powder. Toluene(100 mL) was added to the obtained powder(14.87 g) and boron trifluoride dimethyl ether complex(15.87 g) was added, and the mixture was stirred under reflux for 2 hours. After the reaction solution was cooled to room temperature, ethyl acetate(200 mL) was added and then water(100 mL) was added dropwise. The organic layer was separated, dried and the solvent was distilled way, and the residue was purified with a silica gel column chromatography(hexane/ethyl acetate=90/10 to 70/30) to give Compound(3)(2.48 g) as a colorless powder.
   APCI-MS(m/e):177[M+H]⁺. 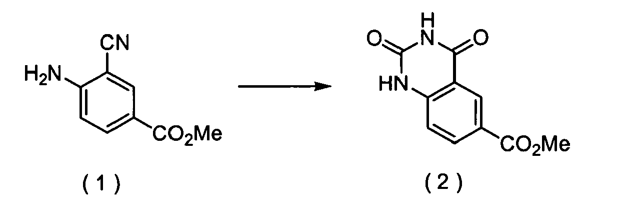
(2) Compound(2) was obtained by reacting and treating Compound(1) in the same manner as Example 42(1).
   APCI-MS(m/e):221[M+H]⁺. 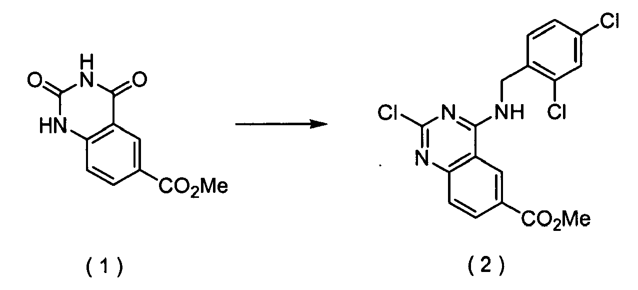
(3) Compound(2) was obtained by reacting and treating Compound(1) in the same manner as Examples 1(2) and 1(3).
   APCI-MS(m/e):396/398[M+H]⁺.

### Reference Example 19

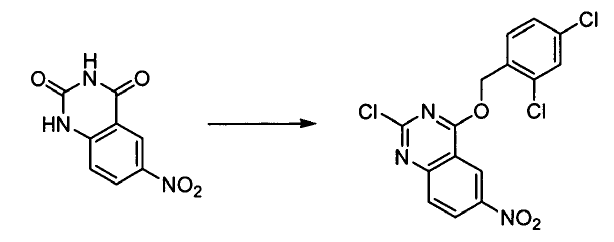

Phosphorous oxychloride(18.4 g) was added to a suspension of 6-nitro-1H-quinazolin-2,4-dione(2.07 g) and 2,4,6-trimethylpyridine(3.64 g) in acetonitrile(150mL) and the mixture was stirred under reflux overnight. After the reaction solution was concentrated, ethyl acetate and water were added to the residue. The organic layer was separated, washed with water, dried and the solvent was distilled away. Acetonitrile(100 mL) was added to the residue and the mixture was cooled to 0°C. To the solution, was added dropwise at 0°C a suspension of 2,4-dichlorobenzylalcohol(3.54 g) and sodium hydride(800 mg) in DMF(50 mL), which had been stirred at 50°C for an hour. After the mixture was stirred at 0°C for an hour, ethyl acetate and water were added to the reaction solution. The organic layer was separated, washed with water, dried and the solvent was distilled away. Chloroform and diethyl ether were added to the residue, the insoluble materials were filtered, and the filtrate was concentrated. The residue was purified with a silica gel column chromatography(hexane/ethyl acetate=100/0 to 85/15). Diethyl ether and hexane were added to the residue, the precipitate was filtered and dried to give the objective compound(378 mg) as a yellow powder.
APCI-MS(m/e):384/386[M+H]⁺.

### Reference Example 20

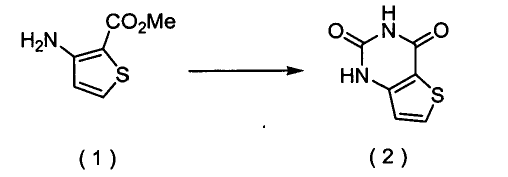

(1) Compound(2) was obtained by reacting and treating Compound(1) in the same manner as Reference Example 2(1).
   APCI-MS(m/e):169[M+H]⁺. 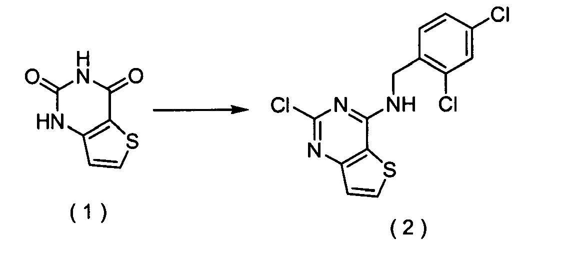
(2) Compound(2) was obtained by reacting and treating Compound(1) in the same manner as Examples 1(2) and 1(3).
APCI-MS(m/e):344/346[M+H]⁺.

### Reference Example 21

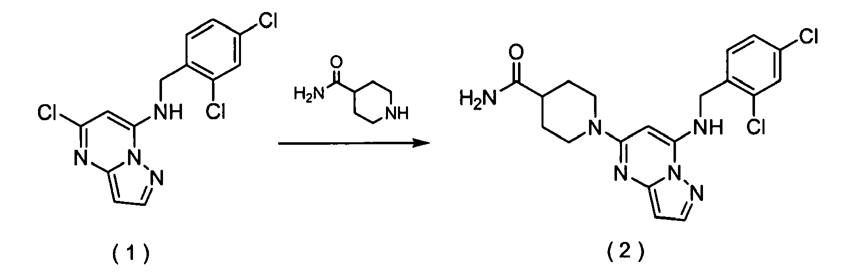

Compound(2) was obtained by reacting and treating Compound(1) in the same manner as Example 1(4).
APCI-MS(m/e):419/421[M+H]⁺.

### Reference Example 22

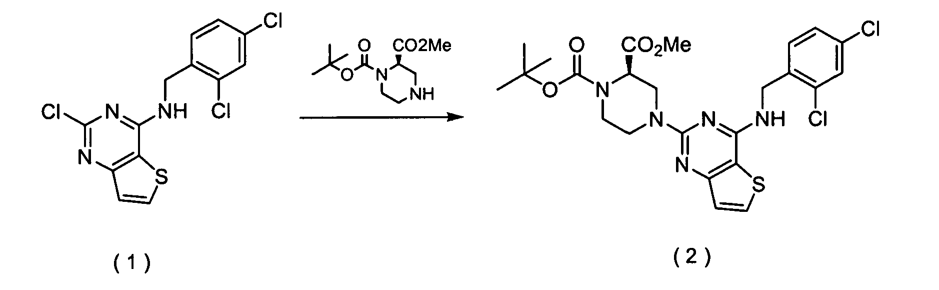

(1) Compound(2) was obtained by reacting and treating Compound(1) in the same manner as Example 1(4).
   APCI-MS(m/e):552/554[M+H]⁺. 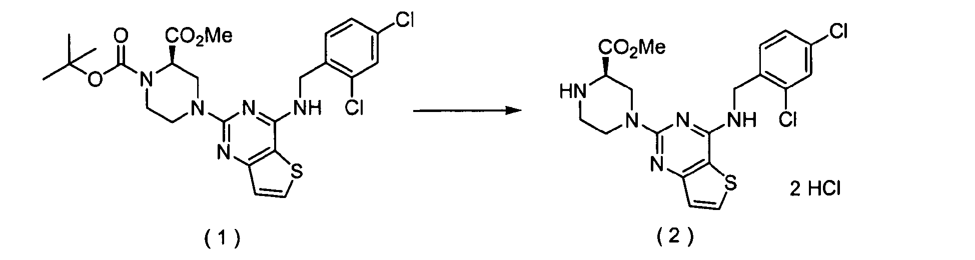
(2) Compound(2) was obtained by reacting and treating Compound(1) in the same manner as Example 1(5).
   APCI-MS(m/e):452/454[M+H]⁺.

### Reference Example 23

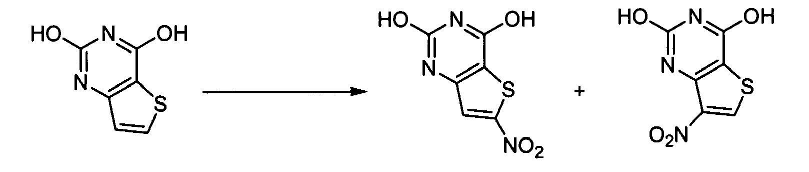

(1) Concentrated sulfuric acid(68 mL) was cooled to -5 to -2°C and concentrated nitric acid(68 mL) was added dropwise. 1H-Thieno[3,2-d]pyrimidin-2,4-dione(24.38 g) was added thereto at 0°C and the mixture was stirred for 2.5 hours. The reaction solution was poured into ice water and the precipitated crystals were collected. It was washed with water and dried to give a mixture of 6-nitrothieno[3,2-d]-pyrimidin-2,4-diol and 7-nitrothieno[3,2-d]-pyrimidin-2,4-diol(16.31 g).
   APCI-MS(m/e):212[M-H]⁻. 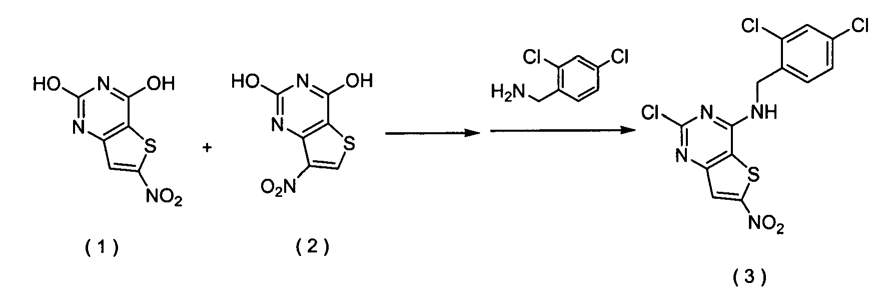
(2) Compound(3) was obtained by reacting and treating the mixture of compound(1) and Compound(2) in the same manner as Examples 1(2) and (3).
   APCI-MS(m/e):389/391[M+H]⁺.

### Reference Example 24

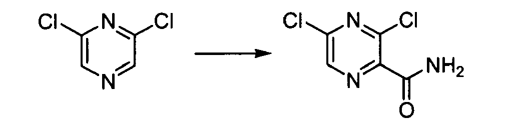

(1) 1.6M n-Butyl lithium(48.25 mL) was added dropwise to a solution of diisopropylamine(7.81 g) in THF(400 mL) cooled to -70°C, and the mixture was stirred at -70°C for 30 minutes. A solution of 2,6-dichloropyrazine(5.0 g) in THF(50 mL) was added dropwise thereto at -70°C for 30 minutes and the mixture was stirred at -70°C for 1.5 hours. Dry ice(150 g) was added to the reaction solution and the mixture was stirred for additional 30 minutes. 6N Hydrochloric acid(200 mL) was added to the reaction solution and then ethyl acetate was added. The separated organic layer was concentrated, and ethyl acetate and a saturated aqueous sodium bicarbonate solution were added to the residue. The aqueous layer was separated, made acidic by adding conc. hydrochloric acid and ethyl acetate was added thereto. The organic layer was washed with water and brine, dried and the solvent was distilled away. Thionyl chloride(100 mL) was added to the residue, and then DMF(0.5 mL) was added, and the mixture was stirred under reflux for 45 minutes. After the reaction solution was concentrated, toluene was added to the residue and the mixture was concentrated again. Methylene chloride(100 mL) was added to the residue and the mixture was cooled to 0°C, 28% ammonia water(25 mL) was added dropwise thereto. The mixture was stirred at 0°C for 15 minutes. The organic layer was separated, dried and the solvent was distilled away. The residue was purified with a silica gel column chromatography(hexane/ethyl acetate=75/25 to 45/55) and diisopropyl ether was added to the residue. The precipitate was filtered and dried to give 3,5-dichloropyrazine-2-carboxylic amide(1.66 g) as a colorless powder.
   APCI-MS(m/e):192/194[M+H]⁺. 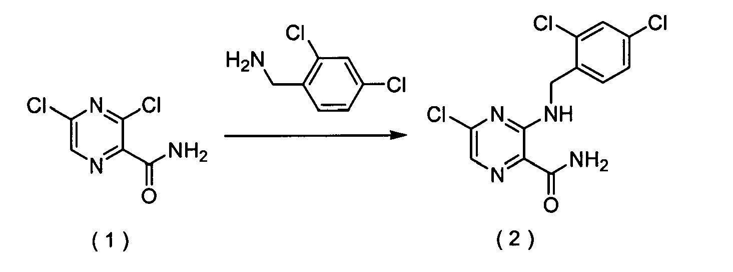
(2) Compound(2) was obtained by reacting and treating 3,5-dichloropyrazine-2-carboxylic amide in the same manner as Example 1(3).
   APCI-MS(m/e):331/333[M+H]⁺.

### Reference Example 25

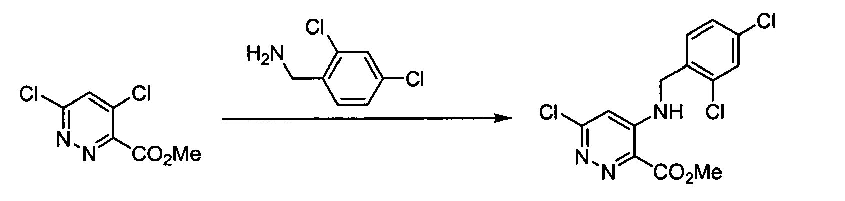

A solution of 2,4-dichlorobenzylamine(2.81 g) and triethylamine(2.20 g) in toluene(20 mL) was added dropwise to a suspension of 4,6-dichloropyridazine-3-carboxylic acid methyl ester(3.08 g) in toluene(100 mL), which was prepared according to a method described in WO01/83460, and the mixture was stirred overnight. Ethyl acetate and water were added to the reaction solution and stirred. The organic layer was separated, dried and the solvent was distilled away. The residue was purified with a silica gel column chromatography(hexane/ethyl acetate=80/20 to 0/100) to give 6-chloro-4-(2,4-dichlorobenzylamino)-pyridazine-3-carboxylic acid methyl ester(4.46 g) as a colorless powder.
APCI-MS(m/e):346/348[M+H]⁺.

### Reference Example 26-1

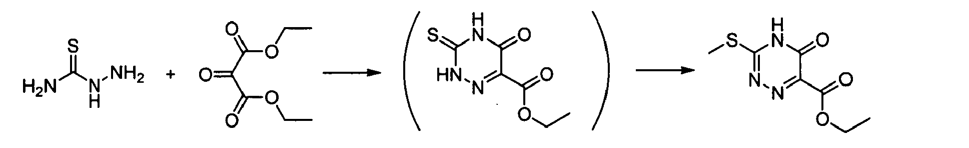

A solution of thiosemicarbazide(49.8 g) and diethyl oxomalonate(98.3 g) in ethanol(900 mL) was refluxed under heating with stirring for 5 hours. After standing to cool, sodium ethoxide(37.4 g) was added to the reaction solution at room temperature and the mixture was refluxed under heating with stirring for 2 hours. The reaction solution was cooled again, a solution of methyl iodide(113 g) and potassium carbonate(41.5 g) in water(300 mL) was added slowly thereto and the mixture was stirred at 0°C for 4 hours. An aqueous citric acid solution was slowly added dropwise to the reaction solution at 0°C and ethanol was distilled away under reduced pressure. Sodium chloride was added to the residue and the mixture was extracted with ethyl acetate. The combined organic layer was dried over magnesium sulfate and the solvent was distilled away. The residue was purified with a silica gel column chromatography(chloroform/methanol=98/2 to 93/7) to give a yellow solid. It was recrystallized from ethyl acetate to give 3-methylsulfanyl-5-oxo-4,5-dihydro-[1,2,4]triazine-6-carboxylic acid ethyl ester(85.2 g) as pale yellow crystals.
APCI-MS(m/e):216[M+H]⁺.

### Reference Example 26-2

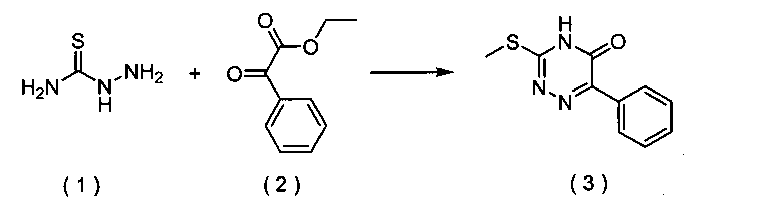

Compound(3) was obtained by reacting and treating Compound(1) and Compound(2) in the same manner as Reference Example 26-1. APCI-MS(m/e):220[M+H]⁺.

### Reference Example 26-3

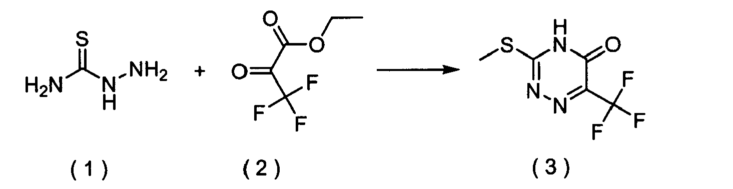

Compound(3) was obtained by reacting and treating Compound(1) and Compound(2) in the same manner as Reference Example 26-1. APCI-MS(m/e):212[M+H]⁺.

### Reference Example 27-1

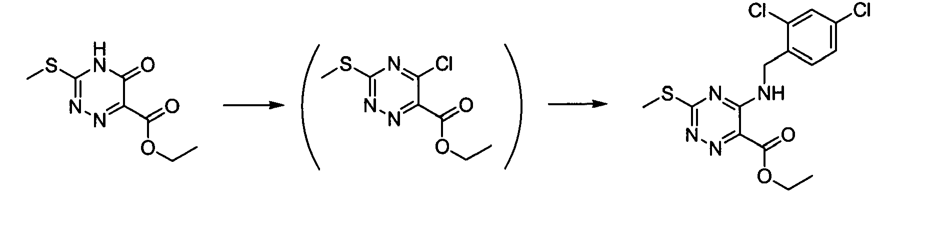

Thionyl chloride(650 mL) was added to 3-methylsulfanyl-5-oxo-4,5-dihydro-[1,2,4]triazine-6-carboxylic acid ethyl ester(60.0 g) and the mixture was refluxed under heating with stirring for a day. After standing to cool, the reaction solution was concentrated and the residue was azeotropically distilled with toluene three times. The residue was dissolved in methylene chloride(500 mL) and a solution of 2,4-dichlorobenzylamine(51.1 g) and triethylamine(40.5 g) in methylene chloride(300 mL) was slowly added dropwise at 0°C, and the mixture was stirred at room temperature for an hour. The reaction soluiton was washed with an aqueous citric acid solution, dried over magnesium sulfate and the solvent was distilled away. The residue was purified with a silica gel column chromatography(hexane/ethyl acetate=10/1 to 4/1) to give a yellow solid, which was recrystallized from ethyl acetate/diisopropyl ether to give 5-(2,4-dichlorobenzylamino)-3-methylsulfanyl-[1,2,4]triazine-6-carboxylic acid ethyl ester(48.2 g) as pale yellow crystals.
APCI-MS(m/e):373/375[M+H]⁺.

The following compounds were obtained by reaction and treatment in the same manner as Reference Example 27-1.

### Reference Example 27-2

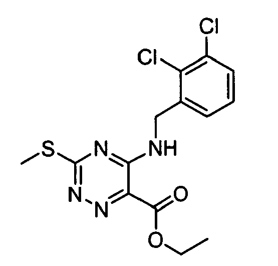

APCI-MS(m/e):373/375[M+H]⁺.

### Reference Example 27-3

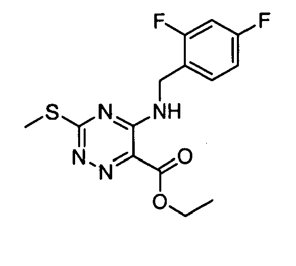

APCI-MS(m/e):341[M+H]⁺.

### Reference Example 27-4

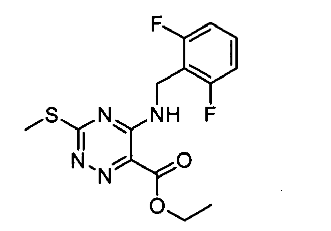

APCI-MS(m/e):341 [M+H]⁺.

### Reference Example 27-5

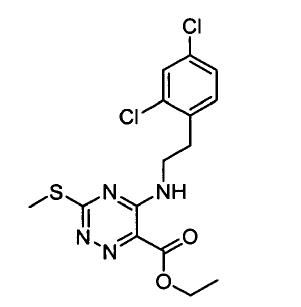

APCI-MS(m/e):387/389[M+H]⁺.

### Reference Example 27-6

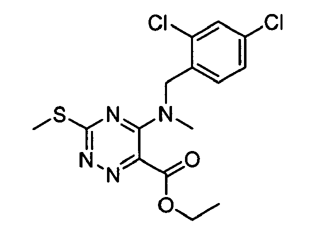

APCI-MS(m/e):387/389[M+H]⁺.

### Reference Example 27-7

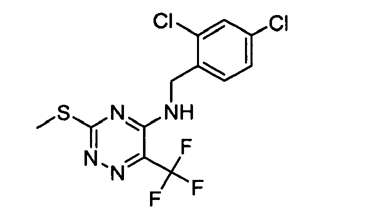

APCI-MS(m/e):369/371[M+H]⁺.

### Reference Example 27-8

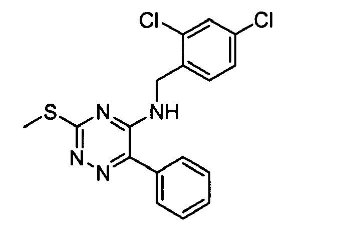

APCI-MS(m/e):377/379[M+H]⁺.

### Reference Example 27-9

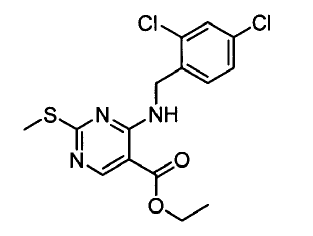

APCI-MS(m/e):372/374[M+H]⁺ .

### Reference Example 27-10

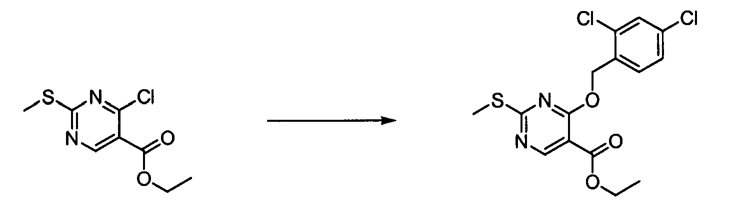

APCI-MS(m/e):373/375[M+H]⁺.

### Reference Example 27-11

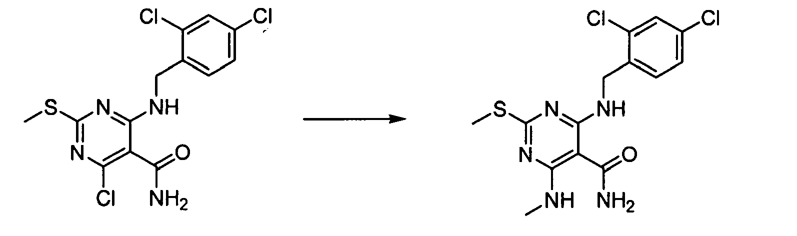

1,4-Dioxane(5 mL) was added to 4-chloro-6-(2,4-dichlorobenzylamino)-2-methylsulfanyl-pyrimidine-5-carboxylic amide(201 mg) and 2M methylamine(THF solution, 0.8 mL) and the mixture was stirred at 40-50°C. The reaction solution was concentrated and the residue was purified with a silica gel column chromatography(hexane/ethyl acetate=1/1 to 1/0) to give 4-(2,4-dichlorobenzylamino)-6-methylamino-2-methylsulfanyl-pyrimidine-5-carboxylic amide(197 mg) as colorless crystals.
APCI-MS(m/e):372/374[M+H]⁺.

The following compounds were obtained by reaction and treatment in the same manner as Reference Example 27-11.

### Reference Example 27-12

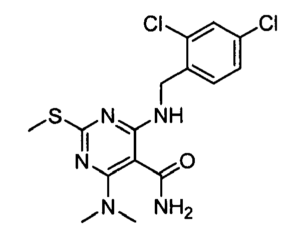

APCI-MS(m/e):386/388 [M+H]⁺.

### Reference Example 27-13

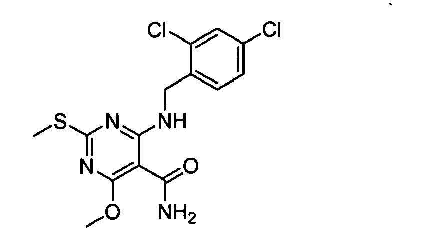

APCI-MS(m/e):373/375[M+H]⁺.

### Reference Example 27-14

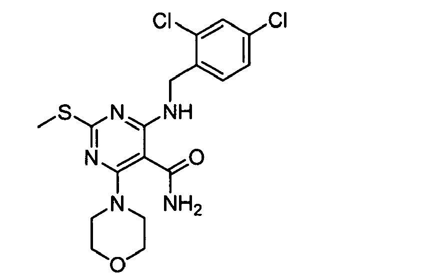

APCI-MS(m/e):428/430[M+H]⁺.

### Reference Example 28-1

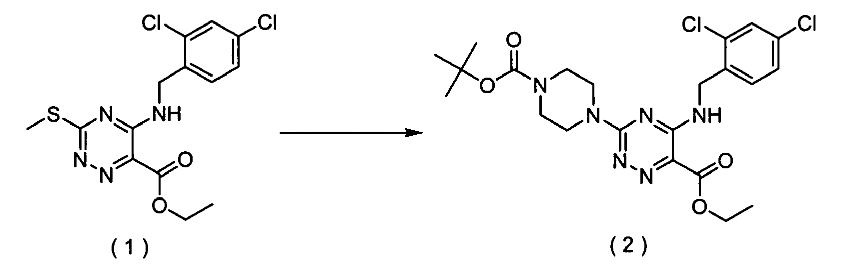

Compound(2) was obtained by reacting and treating Compound(1) in the same manner as Example 16(2).
APCI-MS(m/e):511/513[M+H]⁺.

The following compounds were obtained by reaction and treatment in the same manner as Reference Example 28-1, Example 22, Example 203 and Example 1(5).

### Reference Example 28-2

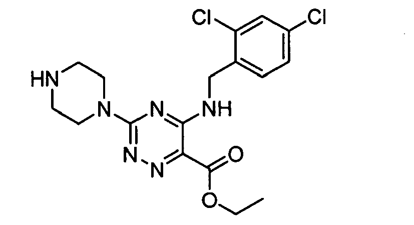

APCI-MS(m/e):411/413[M+H]⁺.

### Reference Example 28-3

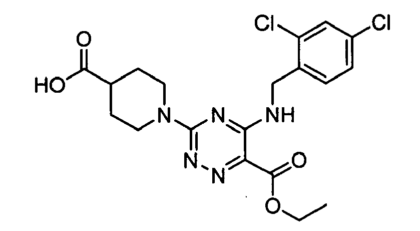

APCI-MS(m/e):454/456[M+H]⁺.

### Reference Example 28-4

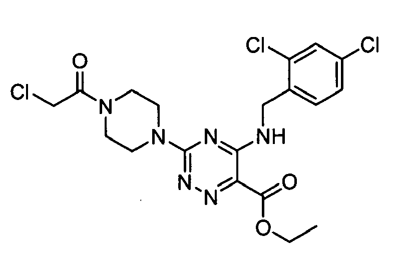

APCI-MS(m/e):487/489[M+H]⁺.

### Reference Example 28-5

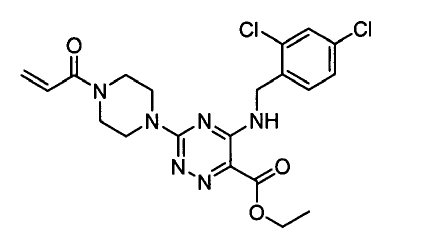

APCI-MS(m/e):465/467[M+H]⁺.

### Reference Example 28-6

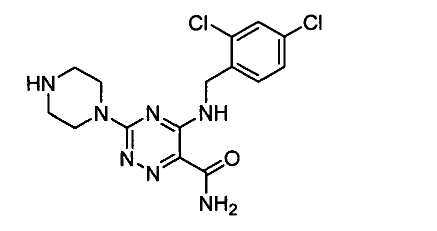

APCI-MS(m/e):382/384[M+H]⁺.

### Reference Example 28-7

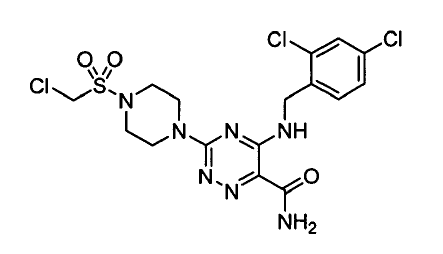

APCI-MS(m/e):494/496[M+H]⁺.

### Reference Example 28-8

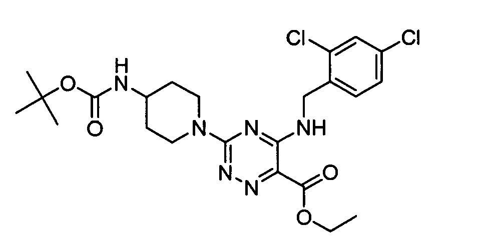

APCI-MS(m/e):525/527[M+H]⁺.

### Reference Example 28-9

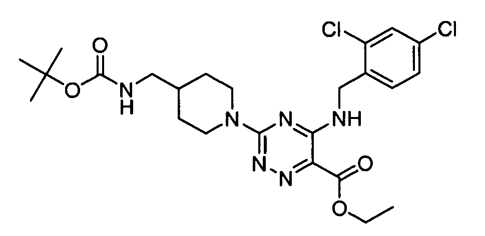

APCI-MS(m/e):539/541 [M+H]⁺.

### Reference Example 28-10

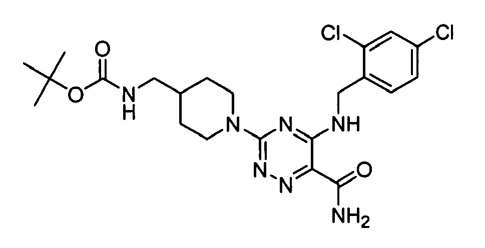

APCI-MS(m/e):510/512[M+H]⁺.

### Reference Example 28-11

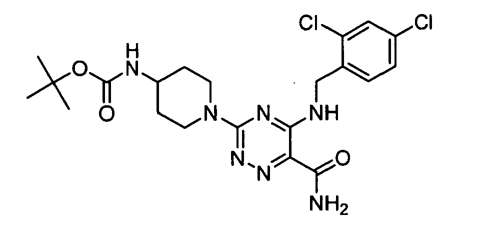

APCI-MS(m/e):496/498[M+H]⁺.

### Reference Example 28-12

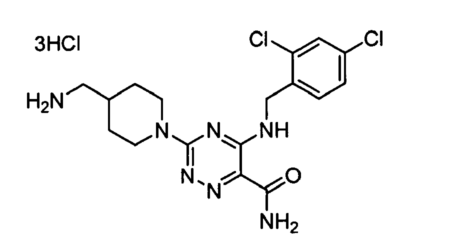

APCI-MS(m/e):410/412[M+H]⁺.

### Reference Example 28-13

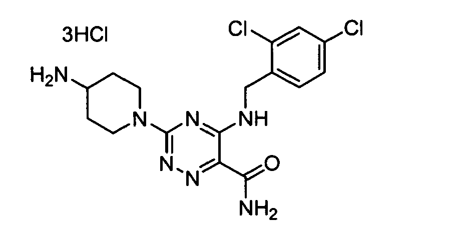

APCI-MS(m/e):396/398[M+H]⁺.

### Reference Example 28-14

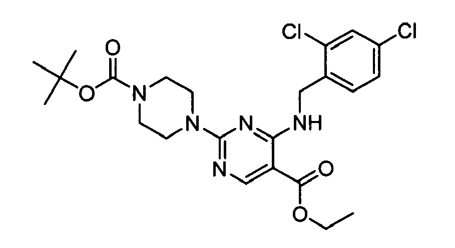

APCI-MS(m/e):510/512[M+H]⁺.

### Reference Example 28-15

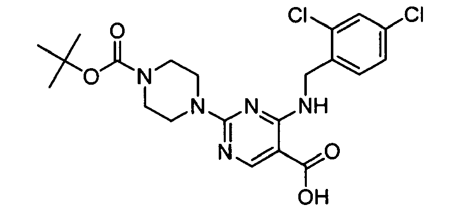

APCI-MS(m/e):482/484[M+H]⁺.

### Reference Example 28-16

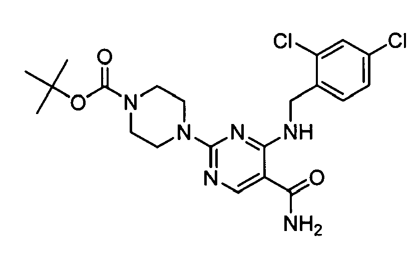

APCI-MS(m/e):481/483[M+H]⁺.

### Reference Example 29

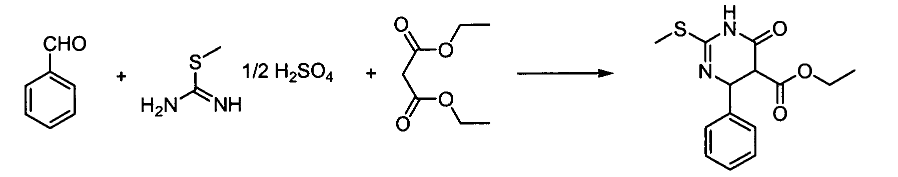

(1) Potassium carbonate(13.8 g) was added to a solution of benzaldehyde(10.6 g), S-methyl thiourea sulfate(13.9 g) and diethyl malonate(16.0 g) in ethanol(200 mL) and the mixture was stirred at 50-60°C overnight. The reaction solution was concentrated, ethyl acetate was added to the residue and the mixture was washed with hydrochloric acid, water, a saturated aqueous sodium bicarbonate solution and brine, dried over magnesium sulfate and the solvent was distilled away. The residue was purified with a silica gel column chromatography(hexane/ethyl acetate=100/0 to 70/30) and recrystallized from ethyl acetate/hexane to give the objective compound(6.21 g) as colorless crystals.
   APCI-MS(m/e):293[M+H]⁺. 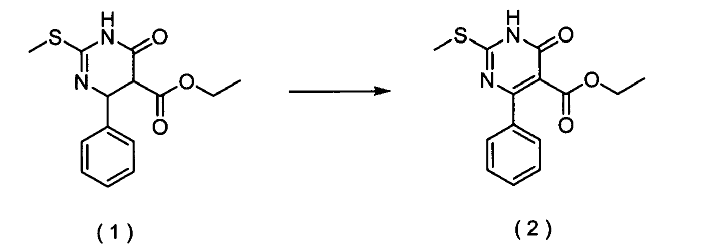
(2) 2,3-Dichloro-5,6-dicyano-1,4-benzoquinone(4.52 g) was slowly added to a solution of Compound(1) (5.85 g) in 2-propanol(120 mL) at 0°C, and the mixture was stirred at 0°C for an hour and at room temperature for a day. 2,3-Dichloro-5,6-dicyano-1,4-benzoquinone(1.16 g) was added and the mixture was further stirred at room temperature for 4 hours. The reaction solution was concentrated under reduced pressure, ethyl acetate was added to the residue, the mixture was washed with water three times, dried over magnesium sulfate and the solvent was distilled away. The residue was purified with a silica gel column chromatography(chloroform/methanol= 1 00/0 to 93/7) and recrystallized from ethyl acetate/hexane to give the objective compound(4.42 g) as colorless crystals.
   APCI-MS(m/e):291[M+H]⁺. 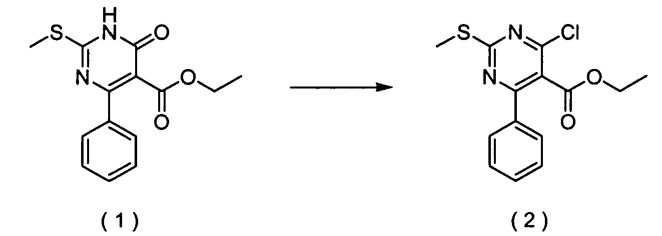
(3) Compound(2) was obtained by reacting and treating Compound(1) in the same manner as Example 1(2).
   APCI-MS(m/e):309/311[M+H]⁺. 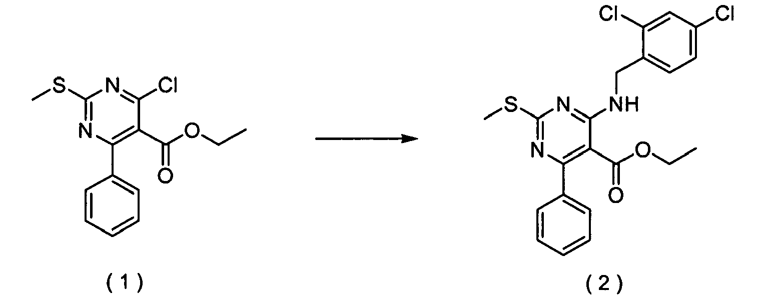
(4) Compound(2) was obtained by reacting and treating Compound(1) in the same manner as Example 1(3).
   APCI-MS(m/e):448/450[M+H]⁺.

### Reference Example 30

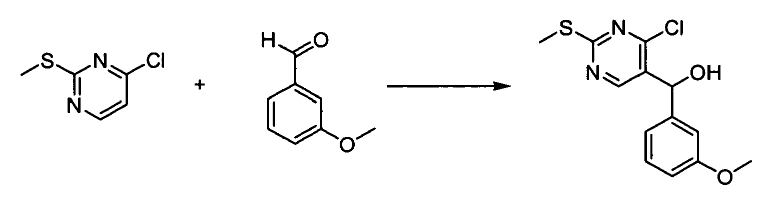

(1) n-Butyl lithium(1.7M hexane solution, 5.5 mL) was slowly added to a solution of diisopropylamine(1.32 g) in THF(10 mL) at -78°C under nitrogen atmosphere and the mixture was stirred for 30 minutes. Then, a solution of 4-chloro-2-methylsulfanyl-pyrimidine(1.01 g) in THF(12 mL) was slowly added thereto and the mixture was stirred for an hour. Further, a solution of 3-methoxybanzaldehyde(950 mg) in THF(12 mL) was slowly added thereto and the mixture was stirred at -78°C for 2 hours and at room temperature for 2 hours. A saturated aqueous sodium bicarbonate solution was added to the reaction solution and the mixture was extracted with ethyl acetate three times. The combined organic layer was dried over magnesium sulfate and the solvent was distilled away. The residue was purified with a silica gel column chromatography(hexane/ethyl acetate=90/10 to 65/35) and further purified with a silica gel column chromatography(hexane/ethyl acetate=100/0 to 50/50) to give (4-chloro-2-methylsulfanyl-pyrimidin-5-yl)-(3-methoxyphenyl)methanol(878 mg) as an orange oily substance.
   APCI-MS(m/e):296[M+H]⁺. 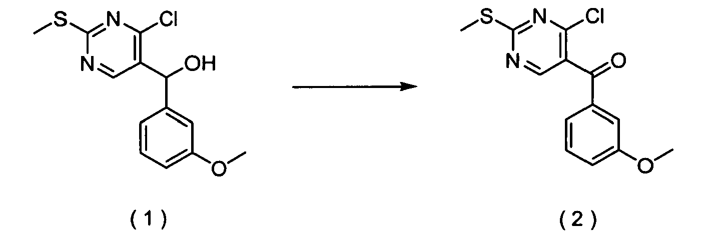
(2) Manganese dioxide(powder, 4.83 g) was added to a solution of (4-chloro-2-methylsulfanyl-pyrimidin-5-yl)-(3-methoxyphenyl)methanol(824 mg) in chloroform(30 mL) at 0°C, and the mixture was stirred at room temperature for 6 hours. Further, manganese dioxide(powder, 4.83 g) was added and the mixture was stirred overnight. The insoluble materials were filtered through Celite and the filtrate was concentrated. The residue was purified with a silica gel column chromatography(hexane/ethyl acetate=100/0 to 80/20) to give (4-chloro-2-methylsulfanyl-pyrimidin-5-yl)-(3-methoxyphenyl)methanone (500 mg) as a colorless oily substance.
   APCI-MS(m/e):295/297[M+H]⁺. 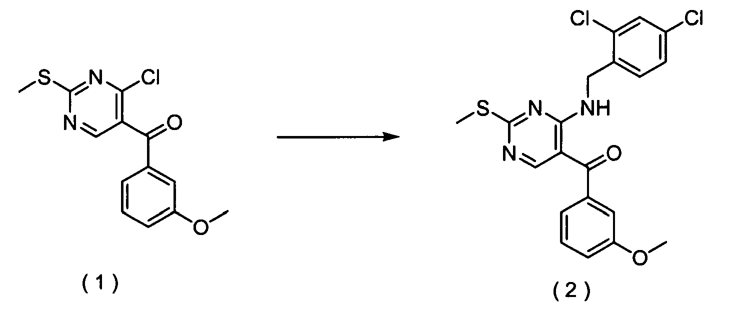
(3) Compound(2) was obtained by reacting and treating 4-chloro-2-methylsulfanyl-pyrimidin-5-yl)-(3-methoxyphenyl)methanone in the same manner as Example 1(3).
   APCI-MS(m/e):434/436[M+H]⁺.

### Reference Example 31

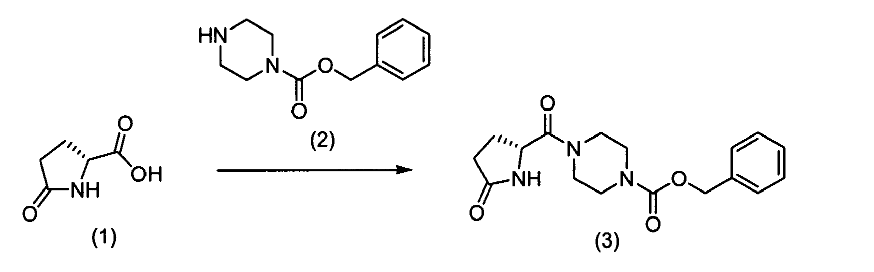

(1) Diethyl phosphorocyanidate(22.64 g) and triethylamine(19.36 mL) were slowly added successively to a solution of Compound(1)(8.96 g) and Compound(2)(19.88 g) in DMF(120 mL) under ice-cooling and the mixture was stirred at room temperature overnight. The reaction solution was concentrated under reduced pressure, ethyl acetate was added to the residue, the mixture was washed with a saturated aqueous sodium bicarbonate solution, dried and the solvent was distilled away. The residue was purified with a silica gel column chromatography(chloroform/methanol=98/2 to 90/10) to give Compound(3)(6.80 g) as a pale yellow oily substance.
   APCI-MS(m/e):302[M+H]⁺. 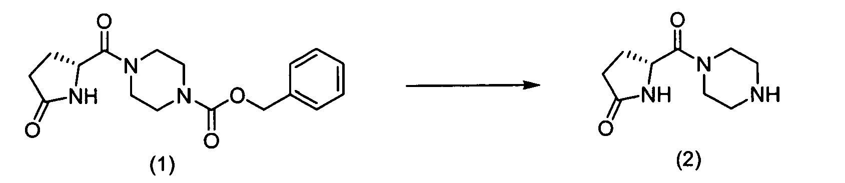
(2) 10% Palladium carbon(1.0 g) was added to a solution of Compound(1)(7.73 g) in methanol(100 mL) and the mixture was stirred under hydrogen pressure(50 psi) at room temperature overnight. The insoluble materials were filtered through a membrane filter and the filtrate was concentrated under reduced pressure to give Compound(2)(4.76 g) as a pale yellow oily substance.
   APCI-MS(m/e):198[M+H]⁺.

### Reference Example 32

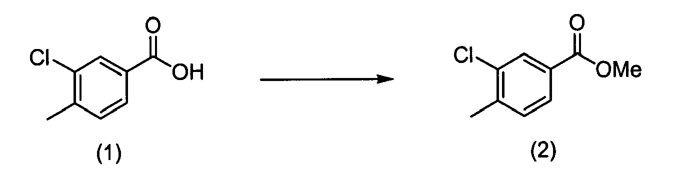

(1) Thionyl chloride(30 mL) was slowly added to methanol(450 mL), Compound(1)(44.9 g) was added thereto and the mixture was stirred at room temperature overnight. The reaction solution was concentrated under reduced pressure, ethyl acetate(500 mL) was added to the residue, and the mixture was washed with a saturated aqueous sodium bicarbonate solution, water and brine and dried. The solvent was distilled away to give Compound(2)(57.8 g) as a pale yellow oily substance.
   GC-MS(m/e):184/186[M+H]⁺. 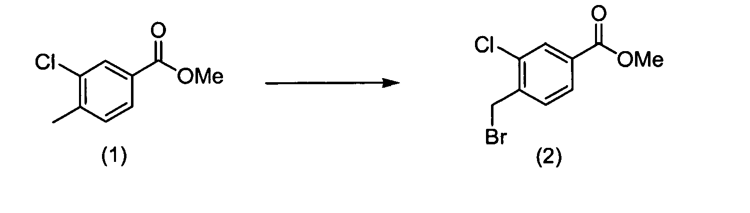
(2) N-Bromosuccinimide(54.3 g) and 2,2'-azobisisobutyronitorile(4.2 g) were added to a solution of Compound(1)(51.0 g) in carbon tetrachloride(478 mL) and the mixture was refluxed under heating with stirring for 2 hours. After standing to cool, the reaction solution was concentrated under reduced pressure and the residue was purified with a silica gel column chromatography(hexane/ethyl acetate=100/0 to 95/5) to give Compound(2)(69.3 g) as a pale yellow oily substance.
   ¹H-NMR(400MHZ/CDCl₃) δ(ppm): 3.93(s,3H), 4.60(s,2H), 7.52(d, 1H, J=7.8 Hz), 7.91(dd, 1H, J=1.5Hz, 7.8Hz), 8.06(d, 1H, J=1.5 Hz). 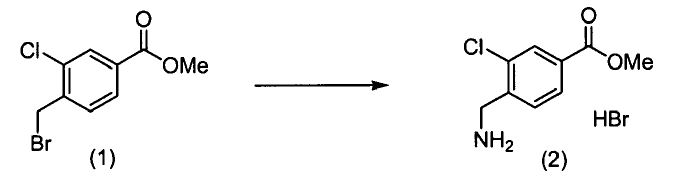
(3) Methanol(1.5 L) was ice-cooled and ammonia gas was dissolved with stirring. Compound(1) was dissolved thereto, the mixture was stirred at room temperature for 2 hours, and ammonia/methanol(1 L) was further added and the mixture was stirrred at room temperature for an hour. The reaction solution was concentrated under reduced pressure, and the residue was triturated with diethyl ether, filtered and dried to give Compound(2)(34.4 g) as a colorless solid.
   APCI-MS(m/e):200/202[M+H]⁺. 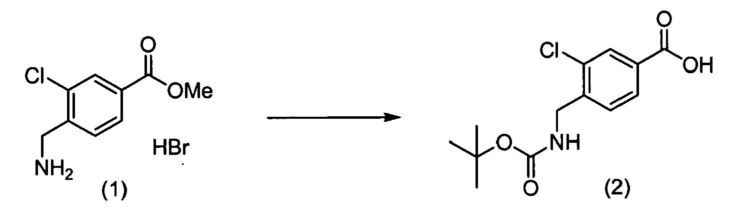
(4) Compound(1)(34.4 g) was dissolved in water(1.2 L), a solution of sodium hydroxide(10.8 g) and di-tert-butyl dicarbonate(29.4 g) in 1,4-dioxane(0.6 L) was added thereto and the mixture was stirred at room temperature for 4 hours. Further, a solution of sodium hydroxide(5.4 g) in water(50 mL) was added and the mixture was stirred overnight. Further, a solution of sodium hydroxide(10.8 g) in water(100 mL) was added, the mixture was stirred at room temperature for an hour and then the reaction solution was concentrated under reduced pressure. The residue was adjusted to pH 3-4 by adding an aqueous citric acid solution (70 g) slowly and the mixture was extracted with 2-propanol/chloroform(9/1). The organic layer was washed with water and brine, dried and the solvent was distilled away to give Compound(2)(33.2 g) as a colorless solid.
   ESI-MS(m/e):284[M-H]⁻. 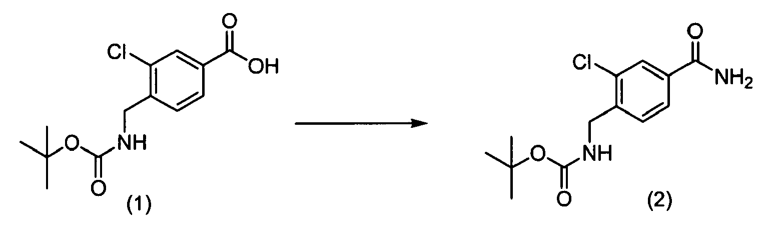
(5) 28% Ammonia water(2 mL), 1-ethyl-3-(3-dimethylaminopropyl)-carbodiimide(1.50 g) and 1-hydroxybenzotriazole(1.20 g) were added to a solution of Compound(1)(1.12 g) in DMF(20 mL) and the mixture was stirred at room temperature for 4 days. Ethyl acetate was added to the reaction soluiton, the mixture was washed with a saturated aqueous sodium bicarbonate solution, water and brine, dried and the solvent was distilled away. The residue was triturated with isopropyl ether/hexane and dried to give Compound(2)(1.04 g) as a colorless powder.
   APCI-MS(m/e):185/187[M+H]⁺. 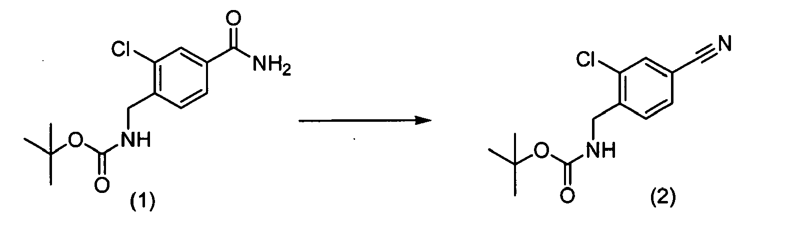
(6) Triphenylphosphine(3.32 g) and carbon tetrachloride(972 mg) were added to a solution of Compound(1)(600 mg) in acetonitrile(50 mL) and the mixture was stirred at room tmperature for 2 days. Chloroform was added to the reaction solution, the mixture was washed with a saturated aqueous sodium bicarbonate solution, dried and the solvent was distilled away. The residue was purified with a silica gel column chromatography(chloroform/methanol=100/0 to 95/5) to give Compound(2)(554 mg) as a colorless oily substance.
   APCI-MS(m/e):267/269[M+H]⁺. 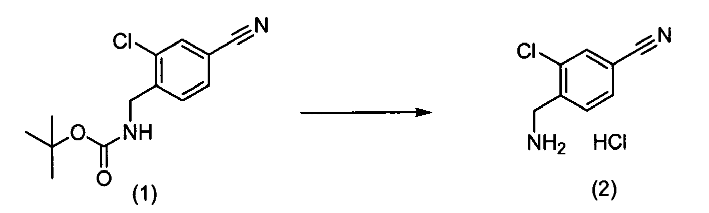
(7) 4N HCl/dioxane(15 mL) was added to a solution of Compound(1)(597 mg) in 1,4-dioxane(10 mL) at room temperature and the mixture was stirred at room temperature for 1.5 hours. The reaction solution was concentrated under reduced pressure and the residue was triturated with diethyl ether, filtered and dried to give Compound(2)(298 mg) as a colorless powder.
   APCI-MS(m/e):167/169[M+H]⁺.

### Reference Example 33

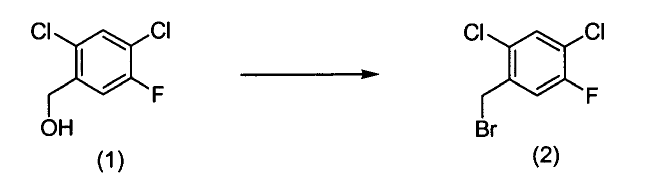

(1) Carbon tetrabromide(2.02 g) and triphenylphosphine(2.0 g) were successively added to a solution of Compound(1)(990 mg) in methylene chloride(30 mL) under ice-cooling and the mixture was stirred at room temperature overnight. Chloroform was added to the reaction solution, and the mixture was washed with water, dried and the solvent was distilled away. The residue was purified with a silica gel column chromatography(hexane/ethyl acetate=100/0 to 95/5) to give Compound(2)(1.39 g) as a colorless transparent oily substance.
   ¹H-NMR(400MZ/CDCl₃) δ(ppm):4.50(s, 2H), 7.26(d, 1H, J=9.0 HZ), 7.46(d, 1H, J=6.7 HZ). 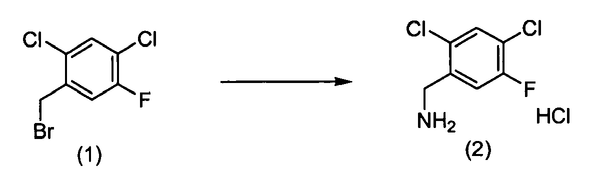
(2) 60% Sodium hydride(145 mg) was added to a solution of Compound(1)(624 mg) in THF(30 mL) and the mixture was stirred for 30 minutes. Then, iminodicarboxylic acid di-tert-butyl ester(788 mg) was added thereto and the mixture was stirred at 55°C for an hour. Iminodicarboxylic acid di-tert-butyl ester(788 mg) was added and the mixture was stirerd at 55°C overnight. After standing to cool, ethyl acetate was added to the reaction solution, the mixture was washed with water, dried and the solvent was distilled away. The residue was purified with a silica gel column chromatography(hexane/ethyl acetate=100/0 to 95/5) to give an oily substance. To a solution of the oily sybstance in ethyl acetate was added 4N HCl/ethyl acetate(20 mL) and the mixture was stirred at room temperature for 1.5 hours. Then, it was concentrated under reduced pressure, the residue was triturated with diethyl ether/hexane and dried to give Compound(2)(490 mg) as a colorless solid.
   APCI-MS(m/e):194/196[M+H]⁺.

### Reference Example 34

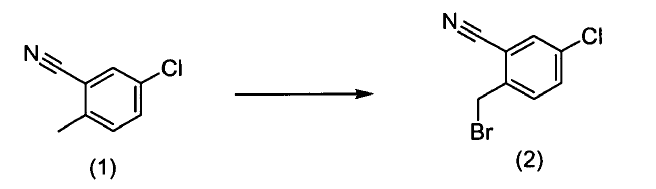

(1) N-Bromosuccinimide(9.26 g) and benzoyl peroxide(515 mg) were added to a solution of Compound(1)(7.8 g) in carbon tetrachloride(50 mL) and the mixture was refluxed under heating with stirring overnight. After standing to cool, it was purified with a silica gel column chromatography(hexane/ethyl acetate=30/1) to give Compound(2)(8.87 g) as a yellow transparent oily substance.
   ¹H-NMR(400MHZ/CDCl₃) δ(ppm):4.60(s, 2H), 7.50(d, 1H, J=8.5 HZ), 7.57(dd, 1H, J=2.3, 8.5 HZ), 7.65(d, 1H, J=2.3 HZ). 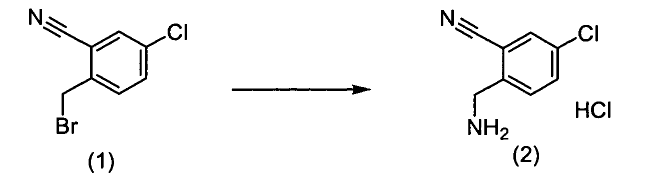
(2) Compound(2) was obtained by reacting and treating Compound(1) in the same manner as Reference Example 33(2).
   APCI-MS(m/e):167/169[M+H]⁺.

### INDUSTRIAL APPLICABILITY

The compound of the present invention or a pharmaceutically acceptable salt thereof has an activity of modulating CCR4, TARC and/or MDC functions and useful for preventing or treating an allergic diseases, inflammatory diseases and autoimmune diseases, such as bronchial asthma or atopic dermatitis.

## Claims

1. A compound of the formula (1) 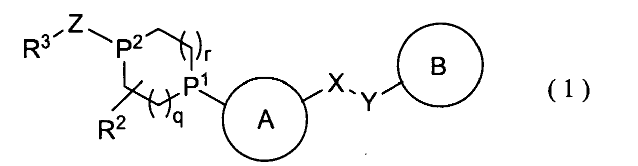 wherein the ring A is a group selected from the group consisting of the following formulas: 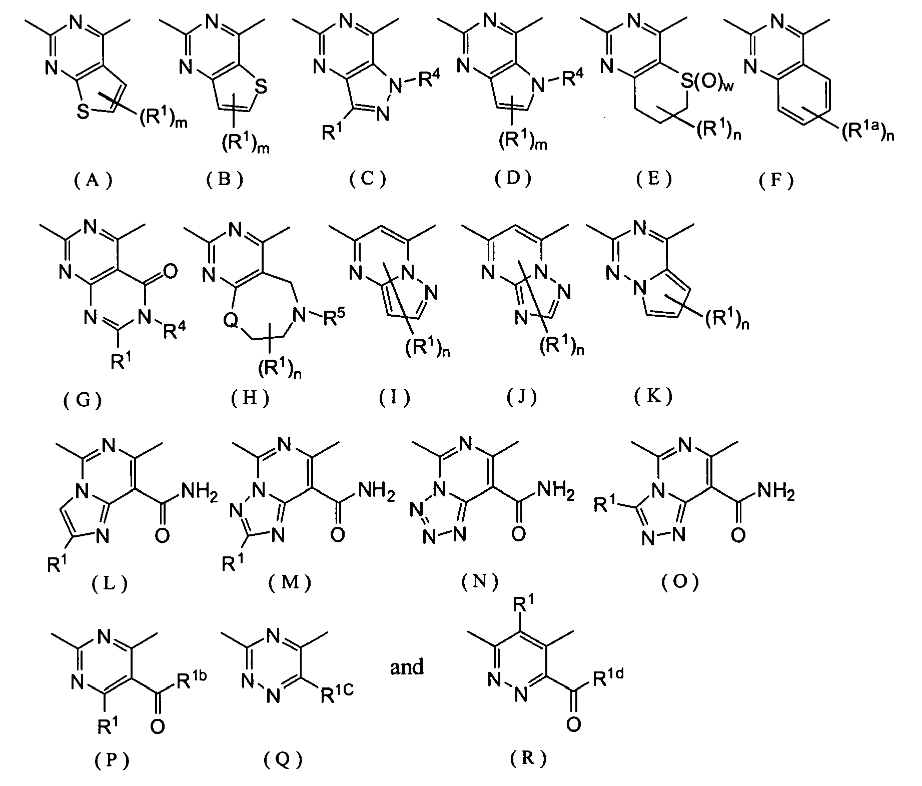 ,
the ring B is an optionally substituted aromatic carbocyclic ring or an optionally substituted heterocyclic ring;
P¹ and P² are the same or different and each is CH or N, provided that neither P¹ nor P² is CH at the same time;
q and r are 0, 1 or 2 respectively;
m is 1 or 2, and n is an integer of 1 to 3;
w is 0, 1 or 2;
Q is an oxygen atom, a sulfur atom or -N(R⁶)-
X is -N(R⁷)-, -O- or -C(R⁸)(R⁹)-;
Y is -C(R¹⁰)(R¹¹)-, -CO- or -SO₂-;
Z is alkylene optionally substituted with oxo, -CON(R¹²)-, - SO₂ N(R¹²)-, -N(R¹²)- or -SO₂ -, provided that P² is CH when Z is -CON(R¹²)-, - SO₂ N(R¹²)- or -N(R¹²)-;
R¹ is hydrogen, alkyl, alkoxy, halogen, carboxy, alkoxycarbonyl, optionally substituted carbamoyl, optionally substituted amino, nitro or optionally substituted ureido;
R^{1a} is carboxy, alkoxycarbonyl, optionally substituted carbamoyl, optionally substituted amino, nitro, phenyl or optionally substituted ureido;
R^{1b} is amino optionally mono- or di-substituted with alkyl(s), or optionally substituted phenyl;
R^{1c} is carboxy, alkoxycarbonyl, cyano, carbamoyl optionally mono-or di-substituted with alkyl(s), or optionally substituted benzoyl;
R^{1d} is hydroxy, alkoxy, amino optionally mono- or di-substituted with alkyl(s), or optionally substituted phenyl;
R² is hydrogen, alkyl, alkoxycarbonyl, carboxy or oxo;
R³ is an optionally substituted carbocyclic group, an optionally substituted heterocyclic group, hydroxy, alkoxy or optionally substituted amino;
R⁴ is hydrogen or alkyl;
R⁵ is hydrogen, alkyl or optionally substituted alkanoyl;
R⁶ is hydrogen, alkyl or optionally substituted alkanoyl;
R⁷ is hydrogen or alkyl;
R⁸ and R⁹, and R¹⁰ and R¹¹ are the same or different, and each is hydrogen or alkyl;
R¹² is hydrogen or alkyl;
or a pharmaceutically acceptable salt thereof,

2. The compound or a pharmaceutically acceptable salt thereof according to claim 1, wherein the ring A is a group selected from the group consisting of the following formulas: 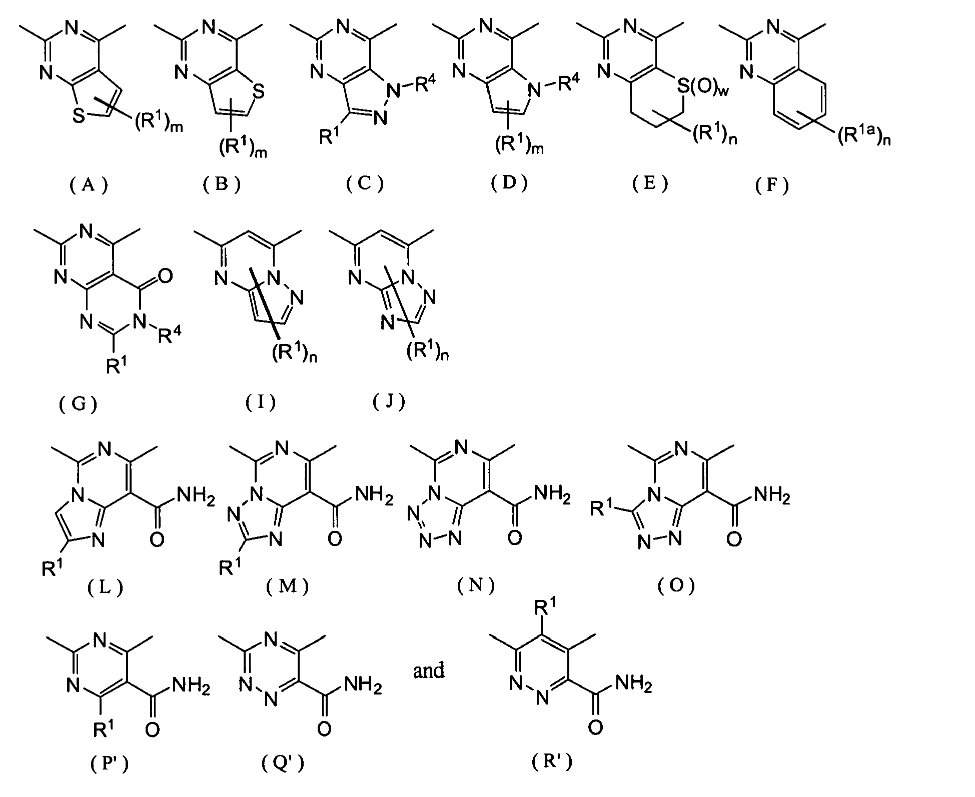 wherein each symbol is the same meanings as defined in claim 1.

3. The compound or a pharmaceutically acceptable salt thereof according to claim 1 or 2 wherein Z is alkylene substituted with oxo.

4. The compound or a pharmaceutically acceptable salt thereof according to any one of claims 1 to 3, wherein R³ is (1) pyrrolidinyl optionally substituted with oxo or cyano, (2) piperidinyl optionally substituted with a group selected from alkyl, alkanoyl and oxo, (3) piperazinyl optionally substituted with alkyl, (4) morpholinyl optionally substituted with alkyl, (5) imidazolyl optionally substituted with alkyl, (6) pyridyl, (7) thiomorpholinyl optionally substituted with one or two oxo(s), (8) tetrahydropyranyl and (9) tetrahydropyrimidinyl optionally substituted with one or two oxo(s).

5. The compound or a pharmaceutically acceptable salt thereof according to any one of claims 1 to 4, wherein X is -NH-, Y is -CH₂-, -CH(CH₃)- or - C(CH₃)₂- and the ring B is benzene substituted with one or two group(s) selected from halogen, alkyl and haloalkyl.
